# EUROPEAN PATENT APPLICATION

(11) **EP 2 460 800 A1**
(43) Date of publication of application: **06.06.2012**
(21) Application number: 11187750.2
(22) Date of filing: 08.03.2007
(51) Int. Cl.: C07D 277/42, C07D 277/46, C07D 277/48, C07D 277/50, C07D 277/56, C07D 417/04, C07D 417/14, C07F 9/09, A61K 31/426, A61K 31/4436, A61P 31/14

(54) **Substituted aminothiazole derivatives with anti-HCV activity**

(30) Priority: 08.03.2006 US 780609 P
(62) Divisional of application: 07752705.9
(71) Applicant: Achillion Pharmaceuticals, Inc., New Haven, CT 06511 (US)
(72) Inventor: Zhang, Suoming, Palo Alto, Canada 94306 (CA); Phadke, Avinash, Branford, CT Connecticut 06405 (US); Wang, Xiangzhu, Branford, CT Connecticut 06405 (US); Liu, Cuixian, Madison, CT Connecticut 06443 (US)
(74) Representative: Smaggasgale, Gillian Helen

(57) **Abstract**

The invention provides amino-substituted aminothiazole compounds of Formula I (Formula I) in which A is a group of the formula: and the variables R and R₁ to R₇ are described herein. These compounds are useful as inhibitors of viral replication. Compositions containing such compounds, and methods of treating viral infections with these compounds, as well as to processes and intermediates useful for preparing such compounds are also provided by the invention.

## Description

### CROSS REFERENCE TO RELATED APPLICATIONS

This application claims priority from U.S. Provisional Patent Application No. 60/780,609 filed March 8, 2006, which is hereby incorporated by reference in its entirety.

### FIELD OF THE INVENTION

The present invention provides substituted aminothiazole derivatives. The invention also includes methods for preparing such compounds. The present invention further includes pharmaceutical compositions containing substituted aminothiazole derivatives and methods for using such compounds, including methods for using the compounds to treat hepatitis C infection.

### BACKGROUND

The invention relates generally to compounds with HCV inhibitory activity.

An estimated 170 million people worldwide are reported to be infected with hepatitis C virus (HCV), the causative agent of non-A, non-B viral hepatitis. Seventy to eighty percent of HCV infections lead to chronic infection, which in turn may result in severe liver disease, including liver fibrosis, cirrhosis, and hepatocellular carcinoma (Lauer, G. M.; Walker, B. D., N. Engl. J. Med. (2001), 345, 41-52).

Presently, one HCV therapy employs a combination of alpha-interferon and ribavirin, leading to sustained efficacy in 40% of patients. (Poynard, T. et al., Lancet (1998), 352, 1426-1432). Clinical results have demonstrated that pegylated alpha-interferon is superior to unmodified alpha-interferon as monotherapy (Zeuzem, S. et al., N. Engl. J. Med. (2000), 343, 1666-1672). However, even with experimental therapeutic regimens involving combinations of pegylated alpha-interferon and ribavirin, a substantial fraction of patients do not have a sustained reduction in viral load. Thus, there is a clear and long-felt need to develop effective therapeutics for treatment of HCV infection.

HCV constitutes the *Hepacivirus* genus of the family *Flaviviridae,* and contains a positive-stranded 9.6 kb RNA genome. Based on a comparison of the deduced amino acid sequence and the extensive similarity in the 5' untranslated region, HCV has been classified as a separate genus in the *Flaviviridae* family. All members of the *Flaviviridae* family have enveloped virions that contain a positive stranded RNA genome encoding all known virus-specific proteins via translation of a single, uninterrupted, open reading frame.

Considerable heterogeneity is found within the nucleotide and encoded amino acid sequence throughout the HCV genome. At least six major genotypes have been characterized, and more than 50 subtypes have been described. The major genotypes of HCV differ in their distribution worldwide, and the clinical significance of the genetic heterogeneity of HCV remains elusive despite numerous studies of the possible effect of genotypes on pathogenesis and therapy.

The single strand HCV RNA genome is approximately 9500 nucleotides in length and has a single open reading frame (ORF) encoding a single large polyprotein of about 3000 amino acids. In infected cells, this polyprotein is cleaved at multiple sites by cellular and viral proteases to produce the structural and non- structural (NS) proteins. In the case of HCV, the generation of mature non-structural proteins (NS2, NS3, NS4A, NS4B, NS5A, and NS5B) is effected by two viral proteases. The first one is believed to cleave at the NS2-NS3 junction; the second one is a serine protease contained within the N-terminal region of NS3 and mediates all the subsequent cleavages downstream of NS3, both in cis, at the NS3-NS4A cleavage site, and in trans, for the remaining NS4A- NS4B, NS4B-NS5A, NS5A-NS5B sites. The NS4A protein appears to serve multiple functions, acting as a cofactor for the NS3 protease and possibly assisting in the membrane localization of NS3 and other viral replicase components. The complex formation of the NS3 protein with NS4A seems necessary to the processing events, enhancing the proteolytic efficiency at all of the sites. The NS3 protein also exhibits nucleoside triphosphatase and RNA helicase activities. NS5B is a RNA-dependent RNA polymerase that is involved in the replication of HCV.

Among the compounds that have demonstrated efficacy in inhibiting HCV replication, as selective HCV serine protease inhibitors, are the peptide compounds disclosed in U.S. Pat. No. 6,323,180.

Nevertheless, there remains a need for a more effective means of treating patients afflicted with HCV. The present invention fulfills this need and provides additional advantages, which are described herein.

### SUMMARY OF THE INVENTION

The present invention generally relates to methods for inhibiting HCV, and compounds useful in such methods.

In one aspect, the present invention includes compounds of Formula I and pharmaceutically acceptable salts thereof.

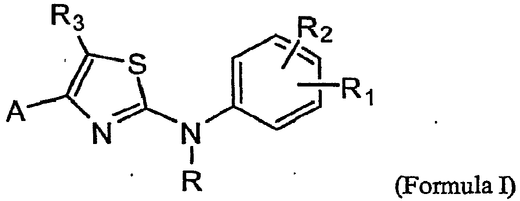

Within Formula I the variables A, R, and R₁ to R₃ carry the definitions set forth below.

R is -C(O)Rₐ, -C(O)CH₂Rₐ, ―N=CHR_{b}, mono- or di-(C₁-C₄alkylamino)C₀-C₄alkyl, or optionally substituted C₁-C₄ alkyl.

R₁ is C₁-C₂haloalkyl, C₁-C₂haloalkoxy, or R₁ is C₄-C₁₀alkoxy, C₄-C₁₀alkyl, C₄-C₁₀alkylamino, di-(C₁-C₆)(C₄-C₁₀)alkylamino, (C₃-C₇cycloalkyl)C₀-C₄alkyl, (C₃-C₇cycloalkyl)C₀-C₄alkoxy, (5- or 6-membered heterocycloalkyl)C₀-C₂alkyl, (5- or 6-membered heterocycloalkyl)C₀-C₂alkoxy, (aryl)C₀-C₂alkyl, or (aryl)C₀-C₂alkoxy, each of which is optionally substituted.

R₂ is 0, 1, or 2 substituents independently chosen from halogen, hydroxyl, amino, cyano, C₁-C₂alkyl, C₁-C₂alkoxy, C₁-C₂haloalkyl, and C₁-C₂haloalkoxy.

R₃ is hydrogen, halogen, hydroxyl, amino, cyano, C₁-C₂alkyl, or (mono- or di-C₁-C₂alkylamino)C₀-C₂alkyl.

A is an optionally substituted C₁ to C₄ alkyl, an optionally substituted C₆ to C₁₀ aryl group, or an optionally substituted 5 to 10 membered heterocycle group; each of which is directed attached to the central thiazole group, or bound via a linker Q, where Q is Q is ―NHC(O)-, -C(O)-, O, S, NH, or CH₂.

Rₐ is hydrogen, R_{c}, -C(O)OH, -C(O)NH₂, or Rₐ is C₁-C₆alkoxy, (C₁-C₆alkylester)C₀-C₄alkyl, (C₁-C₆alkylester)C₁-C₄alkoxy, (mono- or di-C₁-C₆alkylcarbamate)C₀-C₄alkyl, (C₃-C₇cycloalkyl)C₀-C₄alkyl, (5-10-membered heterocycle)C₀-C₄alkyl, C₆-C₁₂polyethylene oxide, or C₁-C₁₀ alkyl, each of which is optionally substituted.

R_{b} is hydrogen or optionally substituted C₁-C₆alkyl.

R_{c} is a hydroxy or optionally substituted amino group.

R₄ is
(a) halogen, hydroxyl, amino, cyano, -C(O)OH, -C(O)NH₂, -PO₄, C₁-C₂haloalkyl, or C₁-C₂haloalkoxy, or
(b) mono- or di- C₁-C₄alkylamino, mono- or di-(C₁-C₄)alkylcarboxamide, C₂-C₄alkanoyl, C₁-C₄aminoalkyl, C₁-C₄aminoalkoxy, C₁-C₄hydroxyalkyl, or C₁-C₄alkylester, each of which is substituted with 0 or 1 or more substituents independently chosen from halogen, hydroxyl, amino, cyano, oxo, C₁-C₂alkyl, C₁-C₂alkoxy, mono- and di- C₁-C₂alkylamino.

R₅ is 0 or 1 or more substituents independently chosen from
(i) hydroxyl, halogen, amino, cyano, nitro, -COOH, -CONH₂, -PO₄, C₁-C₂haloalkyl,
   and C₁-C₂haloalkoxy; and
(ii) C₁-C₄alkyl, C₁-C₄alkoxy, mono- and di- C₁-C₄alkylamino, C₂-C₄alkanoyl, mono-and di-C₁-C₄alkylphosphate, C₁-C₄alkylester, (C₃-C₇cycloalkyl)C₀-C₂alkyl, or (5- or 6-membered heterocycloalkyl)C₀-C₂alkyl, each of which is substituted with 0 or 1 or more substituents independently chosen from halogen, hydroxyl, amino, cyano, oxo, C₁-C₂alkoxy, mono- and di- C₁-C₂alkylamino.

Or, any two R₄ and R₅ bound to adjacent carbon atoms may be joined to form a 5- or 6-membered ring having 0,1, or 2 ring heteroatoms chosen from N, O, and S; which 5- or 6-membered ring is optionally substituted with 1 or 2 substituents independently chosen from halogen, hydroxyl, oxo, C₁-C₂alkyl, and C₁-C₂alkoxy.

R₆ is 0 or 1 or more substituents independently chosen from
(iii) hydroxyl, halogen, amino, cyano, -COOH, -CONH₂, -PO₄, C₁-C₂haloalkyl, and C₁-C₂haloalkoxy;
(iv) C₁-C₄alkyl, C₁-C₄alkoxy, mono- and di- C₁-C₄alkylamino, C₂-C₄alkanoyl, C₁-C₄alkylphosphate, and C₁-C₄alkylester, each of which is substituted with 0 or 1 or more substituents independently chosen from halogen, hydroxyl, amino, cyano, C₁-C₂alkoxy, C₁-C₂mono- and di-alkylamino.

Or, R₆ is joined with R₇ to form a 6-membered aryl or heteroaryl ring, which is substituted with 0 or 1 or more substituents independently chosen from halogen, hydroxyl, amino, cyano, C₁-C₂alkyl, C₁-C₂alkoxy, mono- and di- C₁-C₂alkylamino, C₁-C₂haloalkyl, and C₁-C₂haloalkoxy.

The invention includes pharmaceutical compositions comprising a compound of the invention or a salt thereof, containing at least one pharmaceutically acceptable carrier. The invention also includes pharmaceutical compositions comprising a compound of the invention and containing at least one additional active agent. The invention includes packaged pharmaceutical compositions comprising a compound of the invention in a container and further comprising instructions for using the composition to treat and/ or prevent HCV infection in a patient.

In another aspect the invention provides a method for treating or preventing hepatitis C infection comprising providing an effective amount of a compound or salt of the invention to a patient in need of such treatment or prevention.

A method of inhibiting HCV replication in vivo comprising administering to a patient infected with HCV a concentration of a compound or salt of the invention sufficient to inhibit HCV replicon replication in vitro is also included in the invention. Methods of inhibiting HCV activity that comprise treating a sample containing HCV with an HCV inhibitory amount of at least one compound of Formula I are included. The sample may be a cell or tissue sample, and may be present *in vitro* or in a patient.

These and other aspects of the invention will be more clearly understood with reference to the following detailed description, examples and claims.

### DETAILED DESCRIPTION OF THE INVENTION

Reference will now be made in detail to certain embodiments of the invention, examples of which are illustrated in the accompanying structures and formulas. While the invention will be described in conjunction with the enumerated embodiments, it will be understood that they are not intended to limit the invention to those embodiments. On the contrary, the invention is intended to cover all alternatives, modifications, and equivalents, which may be included within the scope of the present invention as defined by the embodiments.

### I. TERMINOLOGY

Prior to setting forth the invention in detail, it may be helpful to provide definitions of certain terms to be used herein. Compounds of the present invention are described using standard nomenclature. Unless defined otherwise, all technical and scientific terms used herein have the same meaning as is commonly understood by one of skill in the art to which this invention belongs.

Formula I includes all subformulae thereof For example Formula I includes compounds of Formula IA, IB, and IC and the pharmaceutically acceptable salts, prodrugs and other derivatives, hydrates, and polymorphs, thereof.

When tradenames are used herein, applicants intend to independently include the tradename product and the active pharmaceutical ingredient(s) of the tradename product.

The terms "a" and "an" do not denote a limitation of quantity, but rather denote the presence of at least one of the referenced items. The term "or" means "and/or". The terms "comprising", "having", "including", and "containing" are to be construed as open-ended terms (i.e., meaning "including, but not limited to"). Recitation of ranges of values are merely intended to serve as a shorthand method of referring individually to each separate value falling within the range, unless otherwise indicated herein, and each separate value is incorporated into the specification as if it were individually recited herein. The endpoints of all ranges are included within the range and independently combinable. All methods described herein can be performed in a suitable order unless otherwise indicated herein or otherwise clearly contradicted by context. The use of any and all examples, or exemplary language (e.g., "such as"), is intended merely to better illustrate the invention and does not pose a limitation on the scope of the invention unless otherwise claimed. No language in the specification should be construed as indicating any non-claimed element as essential to the practice of the invention as used herein. Unless defined otherwise, technical and scientific terms used herein have the same meaning as is commonly understood by one of skill in the art to which this invention belongs.

An "active agent" means a compound (including a compound of the invention), element, or mixture that when administered to a patient, alone or in combination with another compound, element, or mixture, confers, directly or indirectly, a physiological effect on the patient. The indirect physiological effect may occur via a metabolite or other indirect mechanism. When the active agent is a compound, then salts, solvates (including hydrates) of the free compound, crystalline forms, non-crystalline forms, and any polymorphs of the compound are included. Compounds may contain one or more asymmetric elements such as stereogenic centers, stereogenic axes and the like, e.g., asymmetric carbon atoms, so that the compounds can exist in different stereoisomeric forms. These compounds can be, for example, racemates or optically active forms. For compounds with two or more asymmetric elements, these compounds can additionally be mixtures of diastereomers. For compounds having asymmetric centers, all optical isomers in pure form and mixtures thereof are encompassed. In addition, compounds with carbon-carbon double bonds may occur in Z- and E-forms, with all isomeric forms of the compounds. In these situations, the single enantiomers, i.e., optically active forms can be obtained by asymmetric synthesis, synthesis from optically pure precursors, or by resolution of the racemates. Resolution of the racemates can also be accomplished, for example, by conventional methods such as crystallization in the presence of a resolving agent, or chromatography, using, for example a chiral HPLC column. All forms are contemplated herein regardless of the methods used to obtain them.

All forms (for example solvates, optical isomers, enantiomeric forms, polymorphs, free compound and salts) of an active agent may be employed either alone or in combination.

In certain situations, the compounds of the invention may contain one or more asymmetric elements such as stereogenic centers, including chiral centers, stereogenic axes and the like, e.g. asymmetric carbon atoms, so that the compounds can exist in different stereoisomeric forms. These compounds can be, for example, racemates or optically active forms. For compounds with two or more asymmetric elements, these compounds can additionally be mixtures of diastereomers. For compounds having asymmetric centers, it should be understood that all of the optical isomers and mixtures thereof are encompassed. In addition, compounds with carbon-carbon double bonds may occur in Z- and E-forms, with all isomeric forms of the compounds being included in the present invention. Formula I include all chiral forms, stereoisomers, diastereomers, and enantiomers of compounds of Formula I.

The term "chiral" refers to molecules, which have the property of non-superimposability of the mirror image partner.

"Stereoisomers" are compounds, which have identical chemical constitution, but differ with regard to the arrangement of the atoms or groups in space.

A "Diastereomer" is a stereoisomer with two or more centers of chirality and whose molecules are not mirror images of one another. Diastereomers have different physical properties, e.g., melting points, boiling points, spectral properties, and reactivities. Mixtures of diastereomers may separate under high resolution analytical procedures such as electrophoresis, crystallization in the presence of a resolving agent, or chromatography, using, for example a chiral HPLC column.

"Enantiomers" refer to two stereoisomers of a compound, which are non-superimposable mirror images of one another. A 50:50 mixture of enantiomers is referred to as a racemic mixture or a racemate, which may occur where there has been no stereoselection or stereospecificity in a chemical reaction or process.

Stereochemical definitions and conventions used herein generally follow S. P. Parker, Ed., McGraw-Hill Dictionary of Chemical Terms (1984) McGraw-Hill Book Company, New York; and Eliel, E. and Wilen, S., Stereochemistry of Organic Compounds (1994) John Wiley & Sons, Inc., New York. Many organic compounds exist in optically active forms, *i.e.*, they have the ability to rotate the plane of plane-polarized light. In describing an optically active compound, the prefixes D and L or R and S are used to denote the absolute configuration of the molecule about its chiral center(s). The prefixes d and 1 or (+) and (-) are employed to designate the sign of rotation of plane-polarized light by the compound, with (-) or 1 meaning that the compound is levorotatory. A compound prefixed with (+) or d is dextrorotatory.

A "racemic mixture" or "racemate" is an equimolar (or 50:50) mixture of two enantiomeric species, devoid of optical activity. A racemic mixture may occur where there has been no stereoselection or stereospecificity in a chemical reaction or process.

Where a compound exists in various tautomeric forms, the invention is not limited to any one of the specific tautomers, but rather includes all tautomeric forms.

The invention includes compounds of the invention having all possible isotopes of atoms occurring in the compounds. Isotopes include those atoms having the same atomic number but different mass numbers. By way of general example, and without limitation, isotopes of hydrogen include tritium and deuterium and isotopes of carbon include ¹¹C, ¹³C, and ¹⁴C.

Certain compounds are described herein using a general formula that includes variables, e.g. A, R, and R₁ to R₃. Unless otherwise specified, each variable within the invention is defined independently of other variables. Thus, if a group is said to be substituted, e.g. with 0-2 R*, then said group may be substituted with up to two R* groups and R* at each occurrence is selected independently from the definition of R*. Also, combinations of substituents and/or variables are permissible only if such combinations result in stable compounds.

The term "substituted", as used herein, means that any one or more hydrogens on the designated atom or group is replaced with a selection from the indicated group, provided that the designated atom's normal valence is not exceeded. When the substituent is oxo (i.e., =O), then 2 hydrogens on the atom are replaced. When aromatic moieties are substituted by an oxo group, the aromatic ring is replaced by the corresponding partially unsaturated ring. For example a pyridyl group substituted by oxo is a pyridone. Combinations of substituents and/or variables are permissible only if such combinations result in stable compounds or useful synthetic intermediates. A stable compound or stable structure is meant to imply a compound that is sufficiently robust to survive isolation from a reaction mixture, and subsequent formulation into an effective therapeutic agent.

A dash ("-") that is not between two letters or symbols is used to indicate a point of attachment for a substituent. For example, -(CH₂)C₃-C₇cycloalkyl is attached through carbon of the methylene (CH₂) group.

"Alkyl" includes both branched and straight chain saturated aliphatic hydrocarbon groups, having the specified number of carbon atoms, generally from 1 to about 18 carbon atoms, or more preferably from 1 to about 12 carbon atoms. The term C₁-C₆alkyl as used herein indicates an alkyl group having from 1 to about 6 carbon atoms. When C₀-Cₙ alkyl is used herein in conjunction with another group, for example, (phenyl)C₀-C₄ alkyl, the indicated group, in this case phenyl, is either directly bound by a single covalent bond (C₀), or attached by an alkyl chain having the specified number of carbon atoms, in this case from 1 to about 4 carbon atoms. Examples of alkyl include, but are not limited to, methyl, ethyl, n-propyl, isopropyl, n-butyl, 3-methylbutyl, t-butyl, n-pentyl, and sec-pentyl.

"Alkanoyl" is an alkyl group as defined above, attached through a keto (-(C=O)-) bridge. Alkanoyl groups have the indicated number of carbon atoms, with the carbon of the keto group being included in the numbered carbon atoms. For example a C₂alkanoyl group is an acetyl group having the formula CH₃(C=O)-.

"Alkoxy" means an alkyl group, as defined above, with the indicated number of carbon atoms attached via an oxygen bridge. Examples of alkoxy include, but are not limited to, methoxy, ethoxy, 3-hexoxy, and 3- methylpentoxy. When C₀-Cₙ alkoxy is used herein in conjunction with another group, for example, (heterocycloalkyl)C₀-C₂alkoxy, the indicated group, in this case heterocycloalkyl, is either bound through and oxygen linker, i.e. heterocycloalkyl-O-, (C₀alkoxy), or bound via the oxygen group of an alkoxy chain having the specified number of carbon atoms, in this case from 1 to about 2 carbon atoms.

"Mono- and/ or di-alkylamino" indicates secondary or tertiary alkyl amino groups, wherein the alkyl groups are as defined above and have the indicated number of carbon atoms. The point of attachment of the alkylamino group is on the nitrogen. The alkyl groups are independently chosen. Examples of mono- and di-alkylamino groups include ethylamino, dimethylamino, and methyl-propyl-amino. "Mono- and/or dialkylaminoalkyl" groups are mono- and/ or di-alkylamino groups attached through an alkyl linker having the specified number of carbon atoms, for example a di-methylaminoethyl group. Tertiary amino substituents may by designated by nomenclature of the form N-R-N-R', indicating that the groups R and R' are both attached to a single nitrogen atom.

"Aminoalkyl" indicates an alkyl, group as described herein substituted with at least one amino substituent. Likewise "hydroxyalkyl" indicates an alkyl group as described herein substituted with at least one ―OH substituent.

"Alkylester" indicates an alkyl group as defined above attached through an ester linkage. The ester linkage may be in either orientation, e.g. a group of the formula― O(C=O)alkyl or a group of the formula ―(C=O)Oalkyl.

"Alkylphosphate" indicates a phosphoester linkage which is mono- or disubstituted with independently chosen alkyl groups. A mono-alkyl phosphate substituent has the formula alkyl-HPO4- and a di-alkyl phosphate has the formula alkyl₁alkyl₂PO₄-and the 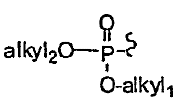 structure. The alkyl groups are as defined above.

"Aryl" means aromatic groups containing only carbon in the aromatic ring or rings. Typical aryl groups contain 1 to 3 separate, fused, or pendant rings and from 6 to about 18 ring atoms, without heteroatoms as ring members. When indicated, such aryl groups may be further substituted with carbon or non-carbon atoms or groups. Such substitution may include fusion to a 5 to 7-membered saturated cyclic group that optionally contains 1 or 2 heteroatoms independently chosen from N, O, and S, to form, for example, a 3,4-methylenedioxy-phenyl group. Aryl groups include, for example, phenyl, naphthyl, including 1- naphthyl and 2-naphthyl, and bi-phenyl.

"Mono- and/or di-alkylcarbamate" indicates groups of the formula (alkyl₁)-O (C=O)NH- and (alkyl₁)-O (C=O)N(alkyl₂)- in which the alkyl₁ and alkyl₂ groups are independently chosen alkyl groups as defined above having the indicated number of carbon atoms.

"Mono- and/ or di-alkylcarboxamide" indicates groups of formula (alkyl₁)-NH-(C=O)- and (alkyl₁)(alkyl₂)-N-(C=O)- in which the alkyl₁ and alkyl₂ groups are independently chosen alkyl groups as defined above having the indicated number of carbon atoms. Mono and/ or di-alkylcarboxamide also refers to groups of the formula ― NH(C=O)(alkyl₁) and ―N(alkyl₂)(C=O)(alkyl₁), carboxamide groups in which the point of attachment is the nitrogen atom, in which the alkyl₁ and alkyl₂ groups are independently chosen alkyl groups as defined above having the indicated number of carbon atoms.

"Cycloalkyl" indicates saturated hydrocarbon ring groups, having the specified number of carbon atoms, usually from 3 to about 8 ring carbon atoms, or from 3 to about 7 carbon atoms. Examples of cycloalkyl groups include cyclopropyl, cyclobutyl, cyclopentyl, or cyclohexyl as well as bridged or caged saturated ring groups such as norborane or adamantane. In the term "(cycloalkyl)alkyl" the terms "cycloalkyl" and "alkyl" carry the definitions set forth herein and the point of attachment of the (cycloalkyl)alkyl group is in the alkyl linker. Likewise (cycloalkyl)alkoxy indicates a cycloalkyl group covalently bound to the group it substitutes via the oxygen of the linker alkoxy group.

"5- or 6-membered heteroaryl" indicates a stable monocyclic aromatic ring having 5 or 6 ring members and from 1 to 4, or preferably from 1 to 3, ring heteroatoms chosen from N, O, and S, with remaining ring atoms being carbon. When the total number of S and O atoms in the heteroaryl group exceeds 1, these heteroatoms are not adjacent to one another. It is preferred that the total number of S and O atoms in the heteroaryl group is not more than 2. It is particularly preferred that the total number of S and O atoms in the heteroaryl group is not more than 1. A nitrogen atom in a heteroaryl group may optionally be quaternized. When indicated, such heteroaryl groups may be further substituted with carbon or non-carbon atoms or groups. Such substitution may include fusion to a 5 to 7-membered saturated cyclic group that optionally contains 1 or 2 heteroatoms independently chosen from N, O, and S, to form, for example, a [1,3]dioxolo[4,5-c]pyridyl group. Examples of heteroaryl groups include, but are not limited to, pyridyl, indolyl, pyrimidinyl, pyridizinyl, pyrazinyl, imidazolyl, oxazolyl, furanyl, thiophenyl, thiazolyl, triazolyl, and tetrazolyl. "H

"Heterocycloalkyl" means a saturated cyclic group containing from 1 to about 3 heteroatoms chosen from N, O, and S, with remaining ring atoms being carbon. Heterocycloalkyl groups have from 3 to about 8 ring atoms, and more typically have from 5 to 7 ring atoms. Examples of heterocycloalkyl groups include morpholinyl, piperazinyl, piperidinyl, and pyrrolidinyl groups. A nitrogen in a heterocycloalkyl group may optionally be quaternized.

"Heterocycle" indicates saturated, unsaturated, and aromatic ring groups having at least one ring containing a heteroatom chosen from N, O, and S. Heterocycle as used herein includes by way of example and not limitation the heterocycles described in Paquette, Leo A.; Principles of Modern Heterocyclic Chemistry (W.A. Benjamin, New York, 1968), particularly Chapters 1, 3, 4, 6, 7, and 9; The Chemistry of Heterocyclic Compounds, A Series of Monographs" (John Wiley & Sons, New York, 1950 to present), in particular Volumes 13, 14, 16, 19, and 28; and J. Am. Chem. Soc. (1960) 82:5566.

Examples of heterocycles include by way of example and not limitation pyridyl, dihydroypyridyl, tetrahydropyridyl (piperidyl), thiazolyl, tetrahydrothiophenyl, sulfur oxidized tetrahydrothiophenyl, pyrimidinyl, furanyl, thienyl, pyrrolyl, pyrazolyl, imidazolyl, tetrazolyl, benzofuranyl, thianaphthalenyl, indolyl, indolenyl, quinolinyl, isoquinolinyl, benzimidazolyl, piperidinyl, 4-piperidonyl, pyrrolidinyl, 2-pyrrolidonyl, pyrrolinyl, tetrahydrofuranyl, tetrahydroquinolinyl, tetrahydroisoquinolinyl, decahydroquinolinyl, octahydroisoquinolinyl, azocinyl, triazinyl, 6H-1,2,5-thiadiazinyl, 2H,6H-1,5,2-dithiazinyl, thienyl, thianthrenyl, pyranyl, isobenzofuranyl, chromenyl, xanthenyl, phenoxathinyl, 2H-pyrrolyl, isothiazolyl, isoxazolyl, pyrazinyl, pyridazinyl, indolizinyl, isoindolyl, 3H-indolyl, 1H-indazoly, purinyl, 4H-quinolizinyl, phthalazinyl, naphthyridinyl, quinoxalinyl, quinazolinyl, cinnolinyl, pteridinyl, 4aH-carbazolyl, carbazolyl, β-carbolinyl, phenanthridinyl, acridinyl, pyrimidinyl, phenanthrolinyl, phenazinyl, phenothiazinyl, furazanyl, phenoxazinyl, isochromanyl, chromanyl, imidazolidinyl, imidazolinyl, pyrazolidinyl, pyrazolinyl, piperazinyl, indolinyl, isoindolinyl, quinuclidinyl, morpholinyl, oxazolidinyl, benzotriazolyl, benzisoxazolyl, oxindolyl, benzoxazolinyl, isatinoyl, and bis-tetrahydrofuranyl.

By way of example and not limitation, carbon bonded heterocycles are bonded at position 2, 3, 4, 5, or 6 of a pyridine, position 3, 4, 5, or 6 of a pyridazine, position 2, 4, 5, or 6 of a pyrimidine, position 2, 3, 5, or 6 of a pyrazine, position 2, 3, 4, or 5 of a furan, tetrahydrofuran, thiofuran, thiophene, pyrrole or tetrahydropyrrole, position 2, 4, or 5 of an oxazole, imidazole or thiazole, position 3, 4, or 5 of an isoxazole, pyrazole, or isothiazole, position 2 or 3 of an aziridine, position 2, 3, or 4 of an azetidine, position 2, 3, 4, 5, 6, 7, or 8 of a quinoline or position 1, 3, 4, 5, 6, 7, or 8 of an isoquinoline. Still more typically, carbon bonded heterocycles include 2-pyridyl, 3-pyridyl, 4-pyridyl, 5-pyridyl, 6-pyridyl, 3-pyridazinyl, 4-pyridazinyl, 5-pyridazinyl, 6-pyridazinyl, 2-pyrimidinyl, 4-pyrimidinyl, 5-pyrimidinyl, 6-pyrimidinyl, 2-pyrazinyl, 3-pyrazinyl, 5-pyrazinyl, 6-pyrazinyl, 2-thiazolyl, 4-thiazolyl, or 5-thiazolyl.

By way of example and not limitation, nitrogen bonded heterocycles are bonded at position 1 of an aziridine, azetidine, pyrrole, pyrrolidine, 2-pyrroline, 3-pyrroline, imidazole, imidazolidine, 2-imidazoline, 3-imidazoline, pyrazole, pyrazoline, 2-pyrazoline, 3-pyrazoline, piperidine, piperazine, indole, indoline, 1H-indazole, position 2 of a isoindole, or isoindoline, position 4 of a morpholine, and position 9 of a carbazole, or β-carboline. Still more typically, nitrogen bonded heterocycles include 1-aziridyl, 1-azetedyl, 1-pyrrolyl, 1-imidazolyl, 1-pyrazolyl, and 1-piperidinyl.

"Haloalkyl" indicates both branched and straight-chain alkyl groups having the specified number of carbon atoms, substituted with 1 or more halogen atoms, generally up to the maximum allowable number of halogen atoms. Examples of haloalkyl include, but are not limited to, trifluoromethyl, difluoromethyl, 2-fluoroethyl, and penta-fluoroethyl.

"Haloalkoxy" indicates a haloalkyl group as defined above attached through an oxygen bridge (oxygen of an alcohol radical).

"Halo" or "halogen" as used herein refers to fluoro, chloro, bromo, or iodo.

"Pharmaceutical compositions" are compositions comprising at least one active agent, such as a compound or salt of the invention, and at least one other substance, such as a carrier, excipient, or diluent. Pharmaceutical compositions meet the U.S. FDA's GMP (good manufacturing practice) standards for human or non-human drugs.

"Pharmaceutically acceptable salts" includes derivatives of the disclosed compounds in which the parent compound is modified by making inorganic and organic, non-toxic, acid or base addition salts thereof. Any reference to any of the compounds of the invention also includes a reference to a physiologically acceptable salt thereof, unless clearly contraindicated by the context. The salts of the present compounds can be synthesized from a parent compound that contains a basic or acidic moiety by conventional chemical methods. Generally, such salts can be prepared by reacting free acid forms of these compounds with a stoichiometric amount of the appropriate base (such as Na, Ca, Mg, or K hydroxide, carbonate, bicarbonate, or the like), or by reacting free base forms of these compounds with a stoichiometric amount of the appropriate acid. Such reactions are typically carried out in water or in an organic solvent, or in a mixture of the two. Generally, non-aqueous media like ether, ethyl acetate, ethanol, isopropanol, or acetonitrile are preferred, where practicable. Salts of the present compounds further include solvates of the compounds and of the compound salts.

Examples of pharmaceutically acceptable salts include, but are not limited to, mineral or organic acid salts of basic residues such as amines; alkali or organic salts of acidic residues such as carboxylic acids; and the like. The pharmaceutically acceptable salts include the conventional non-toxic salts and the quaternary ammonium salts of the parent compound formed, for example, from non-toxic inorganic or organic acids. For example, conventional non-toxic acid salts include those derived from inorganic acids such as hydrochloric, hydrobromic, sulfuric, sulfamic, phosphoric, nitric and the like; and the salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, mesylic, esylic, besylic, sulfanilic, 2-acetoxybenzoic, fumaric, toluenesulfonic, methanesulfonic, ethane disulfonic, oxalic, isethionic, HOOC-(CH₂)ₙ-COOH where n is 0-4, and the like. Lists of additional suitable salts may be found, e.g., in Remington's Pharmaceutical Sciences, 17th ed., Mack Publishing Company, Easton, Pa., p. 1418 (1985).

The term "carrier" applied to pharmaceutical compositions of the invention refers to a diluent, excipient, or vehicle with which an active compound is administered.

A "pharmaceutically acceptable excipient" means an excipient that is useful in preparing a pharmaceutical composition that is generally safe, non-toxic and neither biologically nor otherwise undesirable, and includes an excipient that is acceptable for veterinary use as well as human pharmaceutical use. A "pharmaceutically acceptable excipient" as used in the present application includes both one and more than one such excipient.

A "patient" is a human or non-human animal in need of medical treatment. Medical treatment can include treatment of an existing condition, such as a disease or disorder, prophylactic or preventative treatment, or diagnostic treatment. In some embodiments the patient is a human patient.

"Prodrug" means any compound that becomes compound of the invention when administered to a mammalian subject, e.g., upon metabolic processing of the prodrug. Examples of prodrugs include, but are not limited to, acetate, formate and benzoate and like derivatives of functional groups (such as alcohol or amine groups) in the compounds of the invention.

"Providing" means giving, administering, selling, distributing, transferring (for profit or not), manufacturing, compounding, or dispensing.

"Providing a compound of the invention with at least one additional active agent" means the compound of the invention and the additional active agent(s) are provided simultaneously in a single dosage form, provided concomitantly in separate dosage forms, or provided in separate dosage forms for administration separated by some amount of time that is within the time in which both the compound of the invention and the at least one additional active agent are within the blood stream of a patient. The compound of the invention and the additional active agent need not be prescribed for a patient by the same medical care worker. The additional active agent or agents need not require a prescription. Administration of the compound of the invention or the at least one additional active agent can occur via any appropriate route, for example, oral tablets, oral capsules, oral liquids, inhalation, injection, suppositories or topical contact.

"Treatment," as used herein includes providing a compound of the invention and at least one additional active agent sufficient to: (a) prevent a disease or a symptom of a disease from occurring in a patient who may be predisposed to the disease but has not yet been diagnosed as having it (e.g. including diseases that may be associated with or caused by a primary disease (as in liver fibrosis that can result in the context of chronic HCV infection); (b) inhibiting the disease, i.e. arresting its development; and (c) relieving the disease, i.e., causing regression of the disease. "Treating" and "treatment" also means providing a therapeutically effective amount of a compound of the invention and at least one additional active agent to a patient having or susceptible to a hepatitis C infection.

A "therapeutically effective amount" of a pharmaceutical combination of this invention means an amount effective, when administered to a patient, to provide a therapeutic benefit such as an amelioration of symptoms, e.g., an amount effective to decrease the symptoms of a hepatitis C infection. For example a patient infected with a hepatitis C virus may present elevated levels of certain liver enzymes, including AST and ALT. Normal levels of AST are from 5 to 40 units per liter of serum (the liquid part of the blood) and normal levels of ALT are from 7 to 56 units per liter of serum. A therapeutically effect amount is thus an amount sufficient to provide a significant reduction in elevated AST and ALT levels or an amount sufficient to provide a return of AST and ALT levels to the normal range. A therapeutically effective amount is also an amount sufficient to prevent a significant increase or significantly reduce the detectable level of virus or viral antibodies in the patient's blood, serum, or tissues. One method of determining treatment efficacy includes measuring HCV RNA levels by a convention method for determining viral RNA levels such as the Roche TaqMan assay. In certain preferred embodiments treatment reduces HCV RNA levels below the limit of quantitation (30 IU/mL, as measured by the Roche TaqMan(R) assay) or more preferably below the limit of detection (10 IU/mL, Roche TaqMan).

A significant increase or reduction in the detectable level of virus or viral antibodies is any detectable change that is statistically significant in a standard parametric test of statistical significance such as Student's T-test, where p < 0.05.

### II. SPECIFIC EMBODIMENTS OF COMPOUNDS OF THE INVENTION

In addition to compounds of Formula I, described in the summary of invention section, the invention also includes compounds of Formula I, in which the variables A, R, and R₁ to R₃ carry any of the following definitions.

The invention includes compounds of Formula I 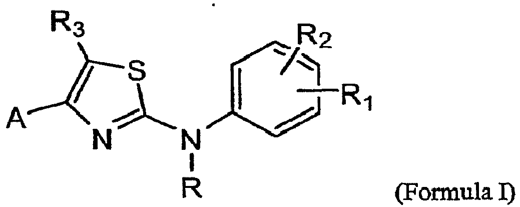 in which A is:

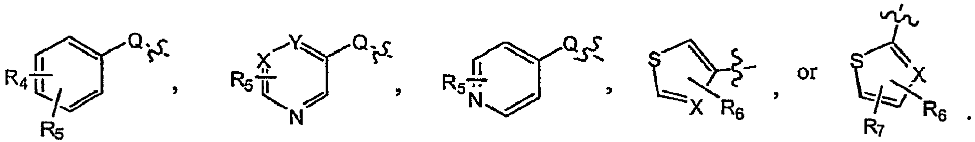

Q is ―NHC(O)-, -C(O)-, O, S, NH, CH2, or absent.

X and Y are independently N or CH.

R is -C(O)Rₐ, -C(O)CH₂Rₐ, ―N=CHR_{b}, mono- or di-(C₁-C₄alkylamino)C₀-C₄alkyl, or substituted C₁-C₄ alkyl.

R₁ is C₁-C₂haloalkyl, C₁-C₂haloalkoxy, or R₁ is C₄-C₁₀alkoxy, C₄-C₁₀alkyl, C₄-C₁₀alkylamino, di-(C₁-C₆)(C₄-C₁₀)alkylamino, (C₃-C₇cycloalkyl)C₀-C₄alkyl, (C₃-C₇cycloalkyl)C₀-C₄alkoxy, (5- or 6-membered heterocycloalkyl)C₀-C₂alkyl, (5- or 6-membered heterocycloalkyl)C₀-C₂alkoxy, (5- or 6-membered heteroaryl)C₀-C₄alkyl, (5- or 6-membered heteroaryl)C₀-C₄alkoxy, (aryl)C₀-C₂alkyl, or (aryl)C₀-C₂alkoxy, each of which is optionally substituted.

R₂ is 0, 1, or 2 substituents independently chosen from halogen, hydroxyl, amino, cyano, C₁-C₂alkyl, C₁-C₂alkoxy, C₁-C₂haloalkyl, and C₁-C₂haloalkoxy;

Rₐ is hydrogen, R_{c}, -C(O)OH, -C(O)NH₂, or Rₐ is C₁-C₆alkoxy, (C₁-C₆alkylester)C₀-C₄alkyl, (C₁-C₆alkylester)C₁-C₄alkoxy, (mono- or di-C₁-C₆alkylcarbamate)C₀-C₄alkyl, (C₃-C₇cycloalkyl)C₀-C₄alkyl, (5-10-membered heterocycle)C₀-C₄alkyl, C₆-C₁₂polyethylene oxide, or C₁-C₁₀ alkyl, each of which is optionally substituted.

R_{b} is hydrogen or optionally substituted C₁-C₆alkyl.

R_{c} is optionally substituted amino.

R₃ is hydrogen, halogen, hydroxyl, amino, cyano, C₁-C₂alkyl, or (mono- or di-C₁-C₂alkylamino)C₀-C₂alkyl.

R₄ is
(a) halogen, hydroxyl, amino, cyano, -C(O)OH, -C(O)NH₂, -PO₄, C₁-C₂haloalkyl, or C₁-C₂haloalkoxy, or
(b) mono- or di- C₁-C₄alkylamino, mono- or di-(C₁-C₄)alkylcarboxamide, C₂-C₄alkanoyl, C₁-C₄aminoalkyl, C₁-C₄aminoalkoxy, C₁-C₄hydroxyalkyl, or C₁-C₄alkylester, each of which is substituted with 0 or 1 or more substituents independently chosen from halogen, hydroxyl, amino, cyano, oxo, C₁-C₂alkyl, C₁-C₂alkoxy, mono- and di- C₁-C₂alkylamino.

R₅ is 0 or 1 or more substituents independently chosen from
(i) hydroxyl, halogen, amino, cyano, nitro, -COOH, -CONH₂, -PO₄, C₁-C₂haloalkyl, and C₁-C₂haloalkoxy; and
(ii) C₁-C₄alkyl, C₁-C₄alkoxy, mono- and di- C₁-C₄alkylamino, C₂-C₄alkanoyl, mono- and di-C₁-C₄alkylphosphate, C₁-C₄alkylester, (C₃-C₇cycloalkyl)C₀-C₂alkyl, or (5- or 6-membered heterocycloalkyl)C₀-C₂alkyl, each of which is substituted with 0 or 1 or more substituents independently chosen from halogen, hydroxyl, amino, cyano, oxo, C₁-C₂alkoxy, mono- and di- C₁-C₂alkylamino.

Or, any two R₄ and R₅ bound to adjacent carbon atoms may be joined to form a 5- or 6-membered ring having 0, 1, or 2 ring heteroatoms chosen from N, O, and S; which 5- or 6-membered ring is optionally substituted with 1 or 2 substituents independently chosen from halogen, hydroxyl, oxo, C₁-C₂alkyl, and C₁-C₂alkoxy.

R₆ is 0 or 1 or more substituents independently chosen from
(iii) hydroxyl, halogen, amino, cyano, -COOH, -CONH₂, -PO₄, C₁-C₂haloalkyl, and C₁-C₂haloalkoxy;
(iv) C₁-C₄alkyl, C₁-C₄alkoxy, mono- and di- C₁-C₄alkylamino, C₂-C₄alkanoyl, C₁-C₄alkylphosphate, and C₁-C₄alkylester, each of which is substituted with 0 or 1 or more substituents independently chosen from halogen, hydroxyl, amino, cyano, C₁-C₂alkoxy, C₁-C₂mono- and di-alkylamino.

Or, R₆ is joined with R₇ to form a 6-membered aryl or heteroaryl ring, which is substituted with 0 or 1 or more substituents independently chosen from halogen, hydroxyl, amino, cyano, C₁-C₂alkyl, C₁-C₂alkoxy, mono- and di- C₁-C₂alkylamino, C₁-C₂haloalkyl, and C₁-C₂haloalkoxy.

### The R variable

The invention includes embodiments in which the variable "R" carries any one of the following definitions.

(1) R is -C(O)Rₐ, -C(O)CH₂Rₐ, ―N=CHR_{b}, or R is C₁-C₄alkyl substituted with 1 or 2 substituents independently chosen from hydroxyl, amino, -C(O)OH, ―C(O)NH₂, -C(O)ONa, or C₁-C₄alkylester; where
Rₐ is hydrogen, R_{c}, -C(O)OH, -C(O)NH₂, C₁-C₄alkyl, C₁-C₄alkoxy, (C₁-C₄alkylester)C₀-C₂alkyl, (mono- or di-C₁-C₆alkylcarbamate)C₀-C₂alkyl, C₆-C₁₂polyethylene oxide, C₃-C₇cycloalkyl, (phenyl)C₀-C₄alkyl, 5-or 6-membered heteroaryl containing 1 or 2 heteroatoms independently chosen from N, S, and O, each or which is substituted with 0, or 1 or more substituents independently chosen from halogen, hydroxyl, amino, phosphate, C₁-C₂alkyl, C₁-C₂alkoxy, mono- and di-(C₁-C₂alkyl)amino, trifluoromethyl, trifluoromethoxy, and phenyl;
R_{b} is C₁-C₄alkyl; and
R_{c} is amino, mono- or di(C₁-C₄alkyl amino, mono- or di(C₁-C₄alkyl amino substituted with ―C(O)OH, C₃-C₇cycloalkylamino, or phenylamino.

(2) R is -C(O)Rₐ, -C(O)CH₂Rₐ, or ―N=CHR_{b};
Rₐ is hydrogen, R_{c}, -C(O)OH, -C(O)NH₂, C₁-C₄alkyl, C₁-C₄alkoxy, (C₁-C₄alkylester)C₀-C₂alkyl, (mono- or di-C₁-C₆alkylcarbamate)C₀-C₂alkyl, C₆-C₁₂polyethylene oxide, or C₃-C₆cycloalkyl, or
Rₐ is phenyl, isoxazolyl, thienyl, imidazolyl, or pyridyl, each of which is substituted with 0, 1, or 2 substituents independently chosen from halogen, hydroxyl, C₁-C₂alkyl, and C₁-C₂alkoxy;
R_{b} is C₁-C₄alkyl; and
R_{c} is amino, mono- or di(C₁-C₄alkyl amino, mono- or di(C₁-C₄alkyl)amino substituted with ―C(O)OH, C₃-C₇cycloalkylamino, or phenylamino.

(3) R is -C(O)Rₐ or -C(O)CH₂Rₐ, where Rₐ is hydrogen, C₁-C₄alkyl, C₁-C₄alkoxy, (C₁-C₄alkylester)C₀-C₂alkyl, or C₃-C₆cycloalkyl, or R is ―C(O)NH₂.

(4) R is -C(O)Rₐ, -C(O)CH₂Rₐ, or ―N=CHR_{b},
Rₐ is hydrogen, R_{c}, -C(O)OH, -C(O)NH₂, C₁-C₄alkyl, C₁-C₄alkoxy, (C₁-C₄alkylester)C₀-C₂alkyl, (mono- or di-C₁-C₆alkylcarbamate)C₀-C₂alkyl, C₆-C₁₂polyethylene oxide, or C₃-C₆cycloalkyl, or Rₐ is phenyl, isoxazolyl, thienyl, imidazolyl, or pyridyl, each of which is substituted with 0, 1, or 2 substituents independently chosen from halogen, hydroxyl, C₁-C₂alkyl, and C₁-C₂alkoxy;
R_{b} is C₁-C₄alkyl; and
R_{c} is amino, mono- or di(C₁-C₄alkyl amino, mono- or di(C₁-C₄alkyl)amino substituted with ―C(O)OH, C₃-C₇cycloalkylamino, or phenylamino.

(5) R is ―C(O)Rₐ or -C(O)CH₂Rₐ,where Rₐ is hydrogen ―C(O)NH₂, C₁-C₂alkyl, C₁-C₂alkoxy, (C₁-C₂alkylester)C₀-C₂alkyl, cyclopropyl, or cyclohexyl.

(6) R is a group of the formula:

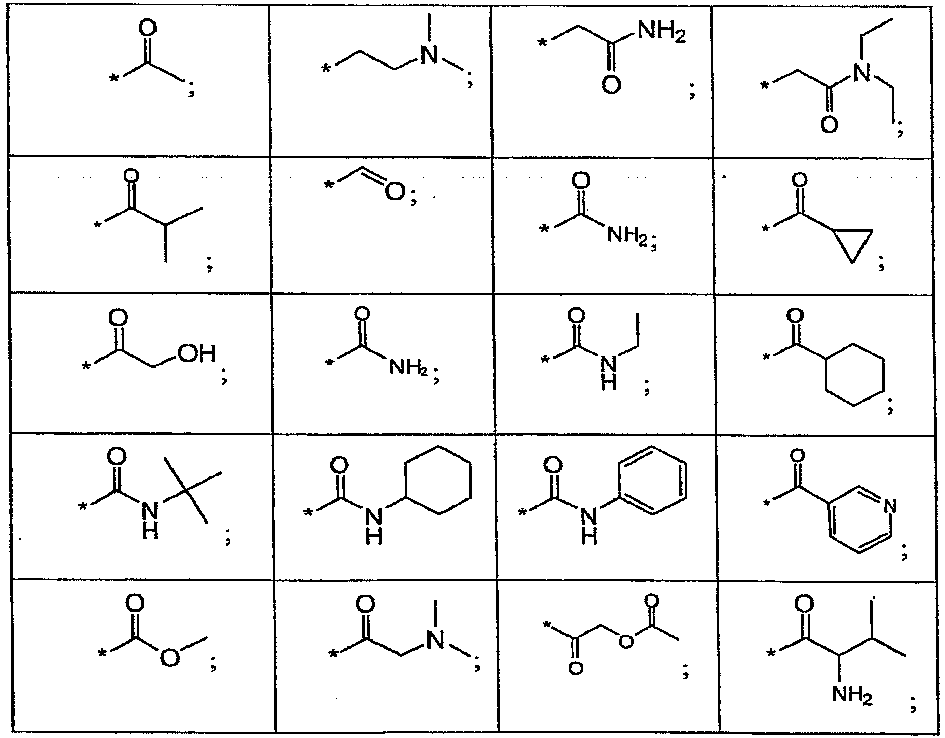

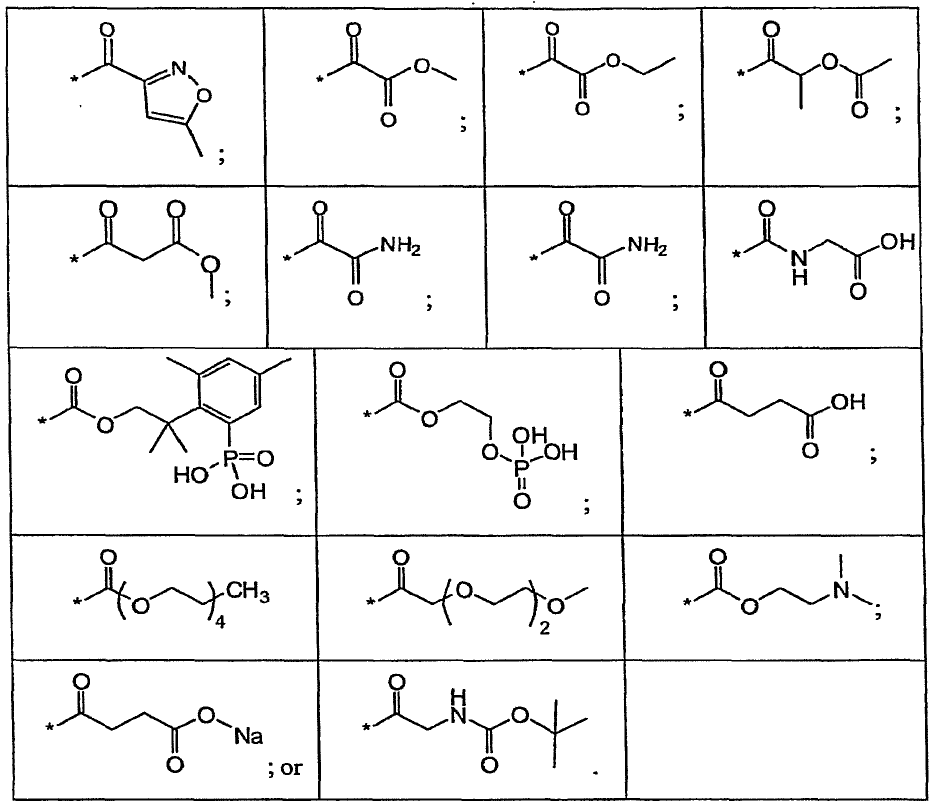

wherein the * indicates the bond of attachment.
The R₁ and R₂ variables

The invention includes embodiments in which the variables R₁ and R₂ any one of the following definitions or meet any of the following conditions.

(1) R₁ is in the para position and R₂ is absent or in which R₁ is in the para position and R₂ is a single substituent in the meta position.

(2) R₁ is in the para position and R₁ is (5- or 6-membered heteroaryl)C₀-C₄alkyl or (5- or 6-membered heteroaryl)C₀-C₄alkoxy, wherein the 5- or 6-membered heteroaryl is thienyl, thiazolyl, imidazolyl, oxazolyl, or pyridyl.

(3) R₁ is C₄-C₁₀alkoxy, C₄-C₁₀alkyl, C₄-C₁₀alkylamino, or di-(C₁-C₆)(C₄-C₁₀)alkylamino, (C₃-C₇cycloalkyl)C₀-C₄alkyl, and R₂ is 0 or 1 substituent chosen from halogen, hydroxyl, amino, cyano, C₁-C₂alkyl, C₁-C₂alkoxy, C₁-C₂haloalkyl, and C₁-C₂haloalkoxy.

(4) R₁ is a para position and is C₄-C₁₀alkoxy, C₄-C₁₀alkyl, C₄-C₁₀alkylamino, or di-(C₁-C₆)(C₄-C₁₀)alkylamino, and R₂ is absent or R₂ is a meta substituent and is halogen, trifluoromethyl, or trifluoromethoxy.

(5) R₁ is n-octyl, n-butoxy, isobutoxy, n-pentoxy, n-hexoxy, n-heptoxy, n-octyloxy, (cyclohexyl)methoxy, trifluoromethyl, methoxy, fluoro, or N-methyl-N-pentylamino.

### The A variable

The invention includes embodiment in which the variable "A" carries any one of the following definitions. The variables Q and R₄ to R₇ are all within the definition of A and are discussed in connection with the definitions of the variable "A."

(1) Q is ―NHC(O)- or absent.

(2) Q is absent.

(3) A is

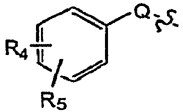

Within this embodiment R₄ and R₅ may carry any of the following definitions.

(a) R₄ is halogen, -C(O)NH₂, C₁-C₂haloalkyl, mono- or di- C₁-C₄alkylamino, mono- or di-(C₁-C₄)alkylcarboxamide, C₂-C₄alkanoyl, C₁-C₄aminoalkyl, C₁-C₄aminoalkoxy, C₁-C₄hydroxyalkyl, or C₁-C₄alkylester, and
R₅ is 0 or 1 or more substituents independently chosen from hydroxyl, halogen, amino, -CONH₂, trifluoromethyl, trifluoromethoxy, C₁-C₂alkyl, and C₁-C₂alkoxy.

(b) R₄ and R₅ bound to adjacent carbon atoms are joined to form a 5- or 6-membered ring having 0, 1, or 2 ring heteroatoms chosen from N, O, and S; which 5- or 6-membered ring is optionally substituted with 1 or 2 substituents independently chosen from halogen, hydroxyl, oxo, C₁-C₂alkyl, and C₁-C₂alkoxy.

(4) A is a group of the formula 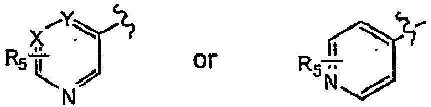 where X and Y are independently N or CH and R₅ carries the definition set forth for Formula I.

Within this embodiment R₅ may carry any of the following definitions.

(a) R₅ is 0 or 1 or more substituents independently chosen from (i) hydroxyl, halogen, amino, cyano, nitro, -COOH, -CONH₂, -PO₄, C₁-C₂haloalkyl, and C₁-C₂haloalkoxy; and (ii) C₁-C₄alkyl, C₁-C₄alkoxy, mono- and di- C₁-C₄alkylamino, C₂-C₄alkanoyl, C₁-C₄alkylester, each of which is substituted with 0 or 1 or more substituents independently chosen from halogen, hydroxyl, amino, cyano, oxo, C₁-C₂alkoxy, mono- and di- C₁-C₂alkylamino.

(b) R₅ is 0, 1, or 2 substituents independently chosen from hydroxyl, halogen, amino, cyano, -COOH, -CONH₂, C₁-C₂haloalkyl, C₁-C₂haloalkoxy, C₁-C₄alkyl, C₁-C₄alkoxy, mono- and di- C₁-C₄alkylamino, and C₂-C₄alkanoyl.

(c) R₅ is absent or R₅ is 0, 1, or 2 substituents independently chosen from hydroxyl, halogen, cyano, -CONH₂, C₁-C₂alkyl, or C₁-C₂alkoxy.

(5) A is 3-pyridyl and R₅ is absent or R₅ is 0, 1, or 2 substituents independently chosen from hydroxy, fluoro, chloro, cyano, -CONH₂, methyl, and methoxy.

(6) A is 3-pyridyl and R₅ is absent.

(7) A is a group of the formula 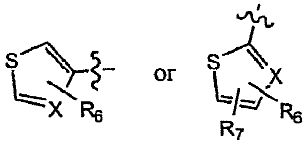 where X is N or CH and R₆ and R₇ carry the definitions set forth for Formula I.

Within this embodiment R₆ and R₇ may carry any of the following definitions.

(a) R₆ is 0 or 1 or more substituents independently chosen from hydroxyl, halogen, amino, cyano, -COOH, -CONH₂, C₁-C₂haloalkyl, C₁-C₂haloalkoxy; C₁-C₄alkyl, C₁-C₄alkoxy, mono- and di- C₁-C₄alkylamino, C₂-C₄alkanoyl, and C₁-C₄alkylester.

(b) R₆ is 0, 1, or 2 substituents independently chosen from halogen, C₁-C₂alkyl, and C₁-C₂alkoxy.
(c) R₆ is joined with R₇ to form a 6-membered aryl or heteroaryl ring, which is substituted with 0 or 1 or more substituents independently chosen from halogen, hydroxyl, C₁-C₂alkyl, and C₁-C₂alkoxy.

A is a group of the formula:

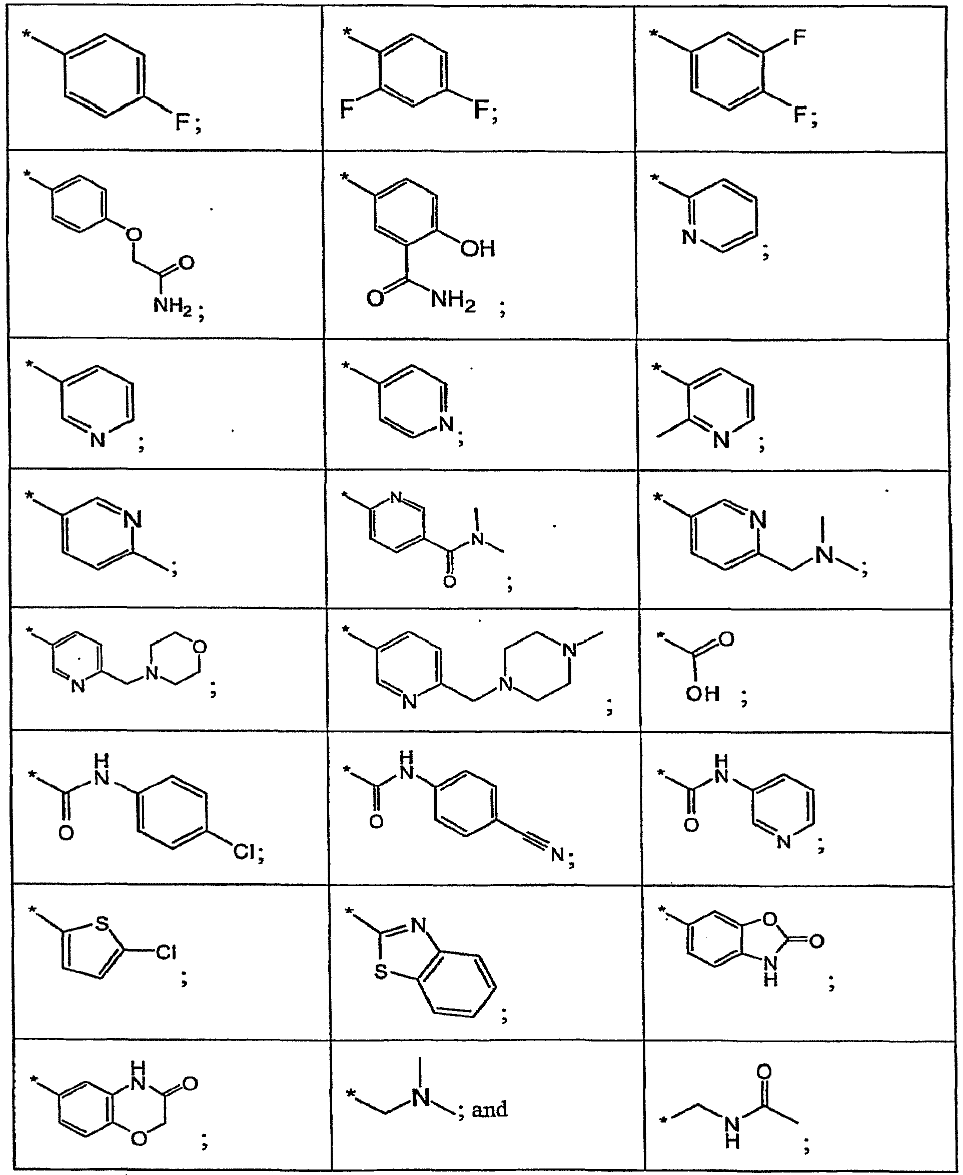

wherein the * indicates the bond of attachment.

### The R₃ variable

The invention includes embodiment in which the variable "A" carries any one of the following definitions.

(1) R₃ is halogen, hydroxyl, amino, methyl, methoxy, dimethylamino, or dimethylaminomethyl.

(2) R₃ is halogen.

(3) R₃ is fluoro.

(4) R₃ is hydrogen.

The invention includes all stable compounds of Formula I having any combination of the above definitions for the variables A, R, and R₁ to R₃. For example the invention includes compound of Formula I in which the R variable carries definition (5), R₁ and R₂ carry definition (3) and condition (1) is also met for this variable; A carries definition 7(b), and R₃ carries definition (3). Thus the invention includes compounds and salts having the following description: 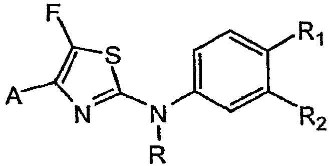 wherein:
R is -C(O)Rₐ or -C(O)CH₂Rₐ,where Rₐ is hydrogen -C(O)NH₂, C₁-C₂alkyl, C₁-C₂alkoxy, (C₁-C₂alkylester)C₀-C₂alkyl, cyclopropyl, or cyclohexyl;
R₁, is C₄-C₁₀alkoxy, C₄-C₁₀alkyl, C₄-C₁₀alkylamino, di-(C₁-C₆)(C₄-C₁₀)alkylamino, or (C₃-C₇cycloalkyl)C₀-C₄alkyl, and R₂ is 0 or 1 substituent chosen from halogen, hydroxyl, amino, cyano, C₁-C₂alkyl, C₁-C₂alkoxy, C₁-C₂haloalkyl, and C₁-C₂haloalkoxy.
A is a group of the formula 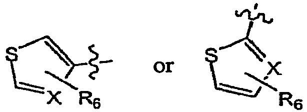 where X is N or CH and R₆ is 0, 1, or 2 substituents independently chosen from halogen, C₁-C₂alkyl, and C₁-C₂alkoxy.

The invention also include compounds of Formula I described by the following sub-formulae.

The invention includes compounds and salts of Formula IA

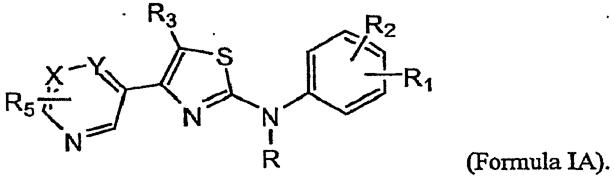

In certain embodiments the invention includes compounds and salts of Formula IA in which
R₁ is in the para position and R₂ is absent or located in the meta position;
R₃ is hydrogen, halogen, or amino;
X and Y are CH; and
R₅ is 0, 1, or 2 substituents independently chosen from hydroxyl, halogen, amino, cyano, -COOH, -CONH₂, C₁-C₂haloalkyl, C₁-C₂haloalkoxy, C₁-C₄alkyl, C₁-C₄alkoxy, mono- and di- C₁-C₄alkylamino, and C₂-C₄alkanoyl.

In other embodiments the invention includes compounds and salts of Formula IA in which:
R₁ is in the para position and is C₄-C₁₀alkoxy, C₄-C₁₀alkyl, C₄-C₁₀alkylamino, or di-(C₁-C₆)(C₄-C₁₀)alkylamino;
R₂ is absent or R₂ is a meta substituent and is halogen, trifluoromethyl, or trifluoromethoxy;
R₃ is hydrogen, fluoro, or amino; and
R₅ is absent or R₅ is 0, 1, or 2 substituents independently chosen from hydroxyl, halogen, cyano, -CONH₂, C₁-C₂alkyl, or C₁-C₂alkoxy.

In certain embodiments R₅ is one (C₃-C₇cycloalkyl)C₀-C₂alkyl or (5- or 6-membered heterocycloalkyl)C₀-C₂alkyl group, each of which is substituted with 0 or 1 or more substituents independently chosen from halogen, hydroxyl, amino, cyano, oxo, C₁-C₂alkoxy, mono- and di- C₁-C₂alkylamino, and R₅ is also 0,1, or 2 substituents independently chosen from hydroxyl, halogen, cyano, -CONH₂, C₁-C₂alkyl, and C₁-C₂alkoxy.

In other embodiments R₅ is (morphonlinyl)C₀-C₂alkyl or (piperazinyl)C₀-C₂alkyl; each of which is substituted with 0 or 1 or more substituents independently chosen from halogen, hydroxyl, amino, cyano, oxo, C₁-C₂alkoxy, mono- and di- C₁-C₂alkylamino.]

The invention includes compounds and salts of Formula IB

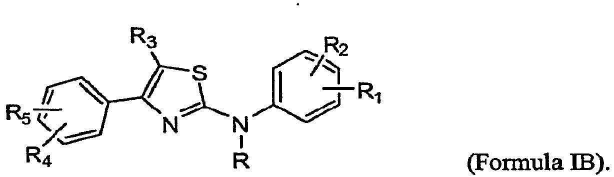

In certain embodiments R₄ is a halogen atom and R₅ is absent or a halogen atom; and the remaining variables, e.g. R and R₁ to R₃ carry any of the definitions set forth above for these variables.

The invention includes compounds and salts of Formula IB in which R₄ is in the para position and R₅ is absent, R₄ is in the meta position and R₅ is absent, or R₄ is in the para position and R₅ is in the meta position.

The invention includes compounds and salts of Formula IC 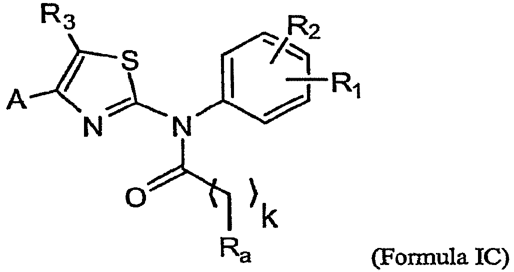 where k is 0 or 1.

In certain embodiments the invention includes compounds and salts of Formula IC in which Rₐ is amino, C₁-C₈ alkyl, a C₁-C₄ alkoxy, or C₁-C₄alkylester.

In other embodiments Rₐ is a C₁ -C₄ alkyl, substituted with one amino or mono- or di-(C₁-C₄alkyl)amino.

The invention includes compounds and salt of Formula IC wherein Rₐ is a (5-10-membered heterocycle)C₀-C₄alkyl, optionally substituted with C₁-C₄alkyl.

The invention also includes compounds and salt of Formula IC wherein 5-10-membered heterocyle is an isoxazole radical, optionally substituted with methyl.

Some preferred compounds of the invention include Compound Nos.: 1, 2, 12, 13,14,15,16, 17, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 69, 70, 71, 72, 75, 76, 77, 81, 83, 84, 85, 86, 87, 89, 91, 93, 94, 96, 97, 98, 99, 100, and 102.

Other preferred compounds include compounds of Formula I in which A is unsubstituted 3-pyridyl, R₁ is a n-octyl at the para position, R₂ is a meta position trifluoromethyl group, R₃ is fluoro, and R carries any of the definitions set forth herein for that variable.

The above compounds are listed only to provide examples that may be used in the methods of the invention. Based upon the instant disclosure, the skilled artisan would recognize other compounds intended to be included within the scope of the presently claimed invention that would be useful in the methods recited herein.

### III. METHODS OF HCV INHIBITION

Another aspect of the invention relates to methods of inhibiting the activity of HCV, comprising the step of treating (e.g., contacting, administering, etc.) a sample suspected of containing HCV with at least one compound of the invention. Compounds of the invention may act as inhibitors of HCV, as intermediates for such inhibitors, or have other utilities as described herein.

Many organisms contain HCV. The compounds of this invention are useful in the treatment or prophylaxis of conditions associated with HCV infection in animals or in man. Thus, in certain aspects, methods for treating or preventing HCV infection are provided, comprising administering a therapeutic or prophylactic amount of at least one compound of the invention to a subject in need thereof

In certain aspects, the inhibitors may generally bind to locations on the surface or in a cavity of the liver where HCV is present. Compositions binding in the liver may bind with varying degrees of reversibility. Those compounds binding substantially irreversibly are ideal candidates for use in this method of the invention. Once labeled, the substantially irreversibly binding compositions are useful as probes for the detection of HCV. Accordingly, the invention relates to methods of detecting HCV in a sample suspected of containing HCV comprising the steps of: treating a sample suspected of containing HCV with a composition comprising a compound of the invention bound to a label; and observing the effect of the sample on the activity of the label. Suitable labels are well known in the diagnostics field and include stable free radicals, fluorophores, radioisotopes, enzymes, chemiluminescent groups and chromogens. The compounds herein are labeled in conventional fashion using functional groups such as hydroxyl or amino.

Within the context of the invention samples suspected of containing HCV include natural or man-made materials such as living organisms; tissue or cell cultures; biological samples such as biological material samples (blood, serum, urine, cerebrospinal fluid, tears, sputum, saliva, tissue samples, and the like); laboratory samples; food, water, or air samples; bioproduct samples such as extracts of cells, particularly recombinant cells synthesizing a desired glycoprotein; and the like. Typically the sample will be suspected of containing HCV. Samples can be contained in any medium including water and organic solvent/water mixtures. Samples include living organisms such as humans, and man made materials such as cell cultures. In certain embodiments, samples include cell and tissue samples, and subjects, including humans.

The treating step of the invention may comprises adding at least one compound of the invention to the sample, or it may comprises adding a precursor of the at least one compound to the sample. The addition step comprises any method of administration as described herein.

If desired, the activity of HCV after application of the compound(s) can be observed by any method, including direct and indirect methods of detecting HCV activity. Quantitative, qualitative, and semiquantitative methods of determining HCV activity are all contemplated. Typically one of the screening methods described herein may be applied, however, any other method such as observation of the physiological properties of a living organism may also be applicable.

However, in screening compounds capable of inhibiting HCV it should be kept in mind that the results of enzyme assays may not correlate with cell culture assays. Thus, in certain aspects, a cell-based assay may preferably be the primary screening tool.

More particularly, as described herein, the compounds of the invention are potent inhibitors of HCV replication and/or proliferation. The activity of the compounds of the invention can be confirmed in *in vitro* assays suitable for measuring inhibition of viral or retroviral replication and/or proliferation. The assays may investigate any parameter that is directly or indirectly under the influence of HCV, including, but not limited to, protein-RNA binding, translation, transcription, genome replication, protein processing, viral particle formation, infectivity, viral transduction, etc. Such assays are well known in the art. Regardless of the parameter being investigated, in one embodiment, to examine the extent of inhibition, samples, cells, tissues, etc. comprising an HCV replicon or HCV RNA are treated with a potential inhibitory compound (test compound) and the value for the parameter compared to control cells (untreated or treated with a vehicle or other placebo). Control samples are assigned a relative activity value of 100%. Inhibition may be achieved when the activity value of the test compound relative to the control is about 90%, preferably 50%, and more preferably 25-0%.

Alternatively, the extent of inhibition may be determined based upon the IC₅₀ of the compound in the particular assay, as will be described in more detail herein.

In one embodiment, the inhibitory activity of the compounds may be confirmed in a replicon assay that assesses the ability of a test compound to block or inhibit HCV replication in replicon cells. One example of a suitable replicon assay is the liver cell-line Huh 7-based replicon assay described in Lohmann et al., 1999, Science 285:110-113. A specific example of this replicon assay utilizes luciferase translation. In one embodiment of this assay, the amount of test compound that yields a 50% reduction in translation as compared to a control cell (IC50, EC50, and/or CC50) may be determined.

Alternatively, the inhibitory activity of the compounds may be confirmed using a quantitative Western immunoblot assay utilizing antibodies specific for HCV non-structural proteins, such as NS3, NS4A NS5A and NS5B. In one embodiment of this assay, replicon cells are treated with varying concentrations of test compound to determine the concentration of test compound that yields a 50% reduction in the amount of a non-structural protein produced as compared to a control sample (IC50, EC50, and/or CC50). A single non-structural protein may be quantified or multiple non-structural proteins may be quantified. Antibodies suitable for carrying out such immunoblot assays are available commercially (e.g., from BIODESIGN International, Saco, Me.).

Alternatively, the inhibitory activity of the compounds may be confirmed in an HCV infection assay, such as the HCV infection assay described in Fournier et al., 1998, J. Gen. Virol. 79(10):2367:2374, the disclosure of which is incorporated herein by reference. In one embodiment of this assay, the amount of test compound that yields a 50% reduction in HCV replication or proliferation as compared to a control cell (IC50, EC50, and/or CC50) may be determined. The extent of HCV replication may be determined by quantifying the amount of HCV RNA present in HCV infected cells.

As yet another example, the inhibitory activity of the compounds may be confirmed using an assay that quantifies the amount of HCV RNA transcribed in treated replicon cells using, for example, a Taqman assay (Roche Molecular, Alameda, Calif.). In one embodiment of this assay, the amount of test compound that yields a 50% reduction in transcription of one or more HCV RNAs as compared to a control sample (IC50, EC50, and/or CC50) may be determined.

Regardless of the assay used, active compounds are generally those which exhibit an IC50, EC50, and/or CC50 in at least one assay in the range of about less than 25 µM, preferably less than about 15 µM, about 10 µM, about 5 µM, or about 1 µM. Compounds which exhibit an IC50, EC50, and/or CC50, for example, in the range of less than about 10 µM, 5 µM, 1 µM, 0.10 µM, 0.50 µM, or even lower, are particularly useful for as therapeutics or prophylactics to treat or prevent HCV infections.

As discussed herein, due to their ability to inhibit HCV replication, the compounds of the invention and/or compositions thereof can be used in a variety of contexts. For example, the compounds of the invention can be used as controls in *in vitro* assays to identify additional more or less potent anti-HCV compounds. As another example, the compounds of the invention and/or compositions thereof can be used as preservatives or disinfectants in clinical settings to prevent medical instruments and supplies from becoming infected with HCV virus. When used in this context, the compound of the invention and/or composition thereof may be applied to the instrument to be disinfected at a concentration that is a multiple of the measured IC50 for the compound.

In a specific embodiment, the compounds and/or compositions may also be used to "disinfect" organs for transplantation. For example, a liver or portion thereof being prepared for transplantation can be perfused with a solution comprising an inhibitory compound of the invention prior to implanting the organ into the recipient. This method has proven successful with lamuvidine (3TC, Epivir™, Epivir-HB™) for reducing the incidence of hepatitis B virus (HBV) infection following liver transplant surgery/therapy. Quite interestingly, it has been found that such perfusion therapy not only protects a liver recipient free of HBV infection (HBV-) from contracting HBV from a liver received from an HBV+ donor, but it also protects a liver from an HBV- donor transplanted into an HBV+ recipient from attack by HBV. The compounds of the invention may be used in a similar manner prior to organ or liver transplantation.

### IV. PHARMACEUTICAL PREPARATIONS

Compounds of the invention can be administered as the neat chemical, but are preferably administered as a pharmaceutical composition. Accordingly, the invention provides pharmaceutical formulations comprising a compound or pharmaceutically acceptable salt of the invention, together with at least one pharmaceutically acceptable carrier.

Compounds of the invention may be administered orally, topically, parenterally, by inhalation or spray, sublingually, transdermally, via buccal administration, rectally, as an ophthalmic solution, or by other means, in dosage unit formulations containing conventional pharmaceutically acceptable carriers. The pharmaceutical composition may be formulated as any pharmaceutically useful form, e.g., as an aerosol, a cream, a gel, a pill, a capsule, a tablet, a syrup, a transdermal patch, or an ophthalmic solution. Some dosage forms, such as tablets and capsules, are subdivided into suitably sized unit doses containing appropriate quantities of the active components, e.g., an effective amount to achieve the desired purpose.

Carriers include excipients and diluents and must be of sufficiently high purity and sufficiently low toxicity to render them suitable for administration to the patient being treated. The carrier can be inert or it can possess pharmaceutical benefits of its own. The amount of carrier employed in conjunction with the compound is sufficient to provide a practical quantity of material for administration per unit dose of the compound.

Classes of carriers include, but are not limited to binders, buffering agents, coloring agents, diluents, disintegrants, emulsifiers, flavorings, glidents, lubricants, preservatives, stabilizers, surfactants, tableting agents, and wetting agents. Some carriers may be listed in more than one class, for example vegetable oil may be used as a lubricant in some formulations and a diluent in others. Exemplary pharmaceutically acceptable carriers include sugars, starches, celluloses, powdered tragacanth, malt, gelatin; talc, and vegetable oils. Optional active agents may be included in a pharmaceutical composition, which do not substantially interfere with the activity of the compound of the present invention.

Binders are substances that bind or "glue" powders together and make them cohesive by forming granules, thus serving as the "adhesive" in the formulation. Binders add cohesive strength to that already available in the diluent or bulking agent. Examples of binders include starch, gelatin, natural sugars, corn sweeteners, natural and synthetic gums such as acacia, sodium alginate, carboxymethylcellulose, polyethylene glycol and waxes. The amount of binder in the composition can range, for example, from about 2 to about 20% by weight of the composition, or from about 3 to about 10% by weight, even more preferably from about 3 to about 6% by weight.

Diluents include sugars such as lactose, sucrose, mannitol and sorbitol; starches derived from wheat, corn, rice and potato; and celluloses such as microcrystalline cellulose. The amount of diluent in the composition may be, for example, about 10 to about 90% by weight of the total composition, about 25 to about 75%, about 30 to about 60% by weight, or about 12 to about 60%.

Disintegrants are materials added to a pharmaceutical composition to help it break apart (disintegrate) and release the active agent. Suitable disintegrants include starches; including "cold water soluble" modified starches such as sodium carboxymethyl starch; natural and synthetic gums such as locust bean, karaya, guar, and tragacanth gum and agar; cellulose derivatives such as methylcellulose and sodium carboxymethylcellulose; microcrystalline celluloses and cross-linked microcrystalline celluloses such as sodium croscarmellose; alginates such as alginic acid and sodium alginate; clays such as bentonites; and effervescent mixtures. The amount of disintegrant in the composition can range, for example, from about 2 to about 15% by weight of the composition or from about 4 to about 10% by weight.

Lubricants are substances added to a pharmaceutical formulation to enable the tablet, granules, etc. after it has been compressed, to release from the; mold or die by reducing friction or wear. Examples of lubricants useful in pharmaceutical dosage forms include boric acid, sodium benzoate, sodium acetate, sodium chloride, and the like. Lubricants are usually added at the very last step before tablet compression, since they must be present on the surfaces of the granules and in between them and the parts of the tablet press. The amount of lubricant in the composition can range, for example, from about 0.1 to about 5% by weight of the composition, from about 0.5 to about 2%, or from about 0.3 to about 1.5% by weight. The amount of compound or salt of the invention in a unit dose may be generally varied or adjusted from about 1.0 milligram to about 1,000 milligrams, from about 1.0 to about 900 milligrams, from about 1.0 to about 500 milligrams, or from about 1 to about 250 milligrams, according to the particular application and the potency of the compound. The actual dosage employed may be varied depending upon the patient's age, sex, weight and severity of the condition being treated.

Pharmaceutical compositions formulated for oral administration are often preferred. These compositions contain between 0.1 and 99% of a compound of the invention and usually at least about 5% (weight %) of a compound of the invention. Some embodiments contain from about 25% to about 50% or from 5% to 75 % of a compound of invention.

### Liquids formulations

Compounds of the invention can be incorporated into oral liquid preparations such as aqueous or oily suspensions, solutions, emulsions, syrups, tinctures, syrups, or elixirs, for example. Moreover, formulations containing these compounds can be presented as a dry product, e.g. as granules or powders, for constitution with water or other suitable vehicle before use. Typical components of carriers for syrups, elixirs, emulsions and suspensions include ethanol, glycerol, propylene glycol, polyethylene glycol, liquid sucrose, sorbitol and water. Liquid preparations can contain conventional additives, such as suspending agents (e.g., sorbitol syrup, methyl cellulose, glucose/sugar, syrup, gelatin, hydroxyethyl cellulose, carboxymethyl cellulose, aluminum stearate gel, and hydrogenated edible fats), emulsifying agents (e.g., lecithin, sorbitan monsoleate, or acacia), non-aqueous vehicles, which can include edible oils (e.g., almond oil, fractionated coconut oil, silyl esters, propylene glycol and ethyl alcohol), and preservatives (e.g., methyl or propyl p-hydroxybenzoate and sorbic acid).. Oral formulations may contain demulcent, flavoring agents, sweetening agents, such as sucrose or saccharin, taste-masking agents, and coloring agents.

### Suspensions

Aqueous suspensions contain the active material(s) in admixture with excipients suitable for the manufacture of aqueous suspensions. Such excipients are suspending agents, for example AVICEL RC-591, sodium carboxymethylcellulose, methylcellulose, hydropropylmethylcellulose, sodium alginate, polyvinylpyrrolidone, gum tragacanth and gum acacia; dispersing or wetting agents, for example lecithin and polysorbate 80. The aqueous suspensions may also contain one or more preservatives, for example ethyl, n- propyl p-hydroxybenzoate, methyl parabens, propyl parabens, and sodium benzoate.

Oily suspensions may be formulated by suspending the active ingredients in a vegetable oil, for example peanut oil, olive oil, sesame oil or coconut oil, or in a mineral oil such as liquid paraffin. The oily suspensions may contain a thickening agent, for example beeswax, hard paraffin or cetyl alcohol. Sweetening agents such as those set forth above, and flavoring agents may be added to provide palatable oral preparations. These compositions may be preserved by the addition of an anti-oxidant such as ascorbic acid.

### Emulsions

Pharmaceutical compositions of the invention may also be in the form of oil-in-water emulsions. The oily phase may be a vegetable oil, for example olive oil or peanut oil, or a mineral oil, for example liquid paraffin or mixtures of these. Suitable emulsifying agents may be naturally-occurring gums, for example gum acacia or gum tragacanth, naturally-occurring phosphatides, for example soy bean, lecithin, and esters or partial esters derived from fatty acids and hexitol, anhydrides, for example sorbitan monoleate, and condensation products of the said partial esters with ethylene oxide, for example polyoxyethylene sorbitan monoleate.

### Tablets and Capsules

Tablets typically comprise conventional pharmaceutically compatible adjuvants as inert diluents, such as calcium carbonate, sodium carbonate, mannitol, lactose and cellulose; binders such as starch, gelatin and sucrose; disintegrants such as starch, alginic acid and croscarmelose; lubricants such as magnesium stearate, stearic acid and talc. Glidants such as silicon dioxide can be used to improve flow characteristics of the powder mixture. Coloring agents, such as the FD&C dyes, can be added for appearance. Sweeteners and flavoring agents, such as aspartame, saccharin, menthol, peppermint, and fruit flavors, are useful adjuvants for chewable tablets. Capsules (including time release and sustained release formulations) typically comprise one or more solid diluents disclosed above. The selection of carrier components often depends on secondary considerations like taste, cost, and shelf stability.

Such compositions may also be coated by conventional methods, typically with pH or time-dependent coatings, such that the subject compound is released in the gastrointestinal tract in the vicinity of the desired topical application, or at various times to extend the desired action. Such dosage forms typically include, but are not limited to, one or more of cellulose acetate phthalate, polyvinylacetate phthalate, hydroxypropyl methylcellulose phthalate, ethyl cellulose, Eudragit coatings, waxes and shellac.

Formulations for oral use may also be presented as hard or soft shell capsules. A capsule is a dosage form administered in a special container or enclosure containing an active agent. The active agent may be present in solid, liquid, gel, or powder form, or any other pharmaceutically acceptable form. A capsule shell may be made of methyl cellulose, polyvinyl alcohols, or denatured gelatins or starch or other material. Hard shell capsules are typically made of blends of relatively high gel strength bone and pork skin gelatins. Soft shell capsule shells are often made of animal or plant gelatins. The capsule itself may contain small amounts of dyes, opaquing agents, plasticizers and preservatives.

The active agent in a capsule may be mixed with an inert solid diluent, for example, calcium carbonate, calcium phosphate or kaolin, or in the case of soft gelatin capsules the active ingredient may be mixed with water or an oil medium, for example peanut oil, liquid paraffin or olive oil.

### Injectable and Parenteral formulations

Pharmaceutical compositions may be in the form of a sterile injectable aqueous or oleaginous suspension. This suspension may be formulated according to the known art using those suitable dispersing or wetting agents and suspending agents that have been mentioned above. The sterile injectable preparation may also be sterile injectable solution or suspension in a non-toxic parentally acceptable diluent or solvent, for example as a solution in 1,3-butanediol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid are useful in the preparation of injectables.

Compounds of the invention may be administered parenterally in a sterile medium. Parenteral administration includes subcutaneous injections, intravenous, intramuscular, intrathecal injection or infusion techniques. The drug, depending on the vehicle and concentration used, can either be suspended or dissolved in the vehicle. Advantageously, adjuvants such as local anesthetics, preservatives and buffering agents can be dissolved in the vehicle. In compositions for parenteral administration the carrier typically comprises least about 90% by weight of the total composition.

### V. METHODS OF TREATMENT

The invention includes methods of preventing and treating hepatitis C infections, by providing an effective amount of a compound of the invention to patient at risk for hepatitis C infection or infected with a hepatitis C virus.

The pharmaceutical combinations disclosed herein are useful for preventing and treating hepatitis C infections in patients. An effective amount of a pharmaceutical combination of the invention may be an amount sufficient to (a) prevent hepatitis C or a symptom of a hepatitis C from occurring in a-patient who may be predisposed to hepatitis C but has not yet been diagnosed as having it or prevent diseases that may be associated with or caused by a primary hepatitis C infection (such as liver fibrosis that can result in the context of chronic HCV infection); (b) inhibit the progression of hepatitis C; and (c) cause a regression of the hepatitis C infection. An amount of a pharmaceutical composition effect to inhibit the progress or cause a regression of hepatitis C includes an amount effective to stop the worsening of symptoms of hepatitis C or reduce the symptoms experienced by a patient infected with the hepatitis C virus. Alternatively a halt in progression or regression of hepatitis C may be indicated by any of several markers for the disease. For example, a lack of increase or reduction in the hepatitis C viral load or a lack of increase or reduction in the number of circulating HCV antibodies in a patient's blood are markers of a halt in progression or regression of hepatitis C infection. Other hepatitis C disease markers include aminotransferase levels, particularly levels of the liver enzymes AST and ALT. Normal levels of AST are from 5 to 40 units per liter of serum (the liquid part of the blood) and normal levels of ALT are from 7 to 56 units per liter of serum. These levels will typically be elevated in a HCV infected patient. Disease regression is usually marked by the return of AST and ALT levels to the normal range.

Symptoms of hepatitis C that may be affected by an effective amount of a pharmaceutical combination of the invention include decreased liver function, fatigue, flu-like symptoms: fever, chills, muscle aches, joint pain, and headaches, nausea, aversion to certain foods, unexplained weight loss, psychological disorders including depression, tenderness in the abdomen, and jaundice.

"Liver function" refers to a normal function of the liver, including, but not limited to, a synthetic function including synthesis of proteins such as serum proteins (e.g., albumin, clotting factors, alkaline phosphatase, aminotransferases (e.g., alanine transaminase, aspartate transaminase), 5'-nucleosidase, y glutaminyltranspeptidase, etc.), synthesis of bilirubin, synthesis of cholesterol, and synthesis of bile acids; a liver metabolic function, including carbohydrate metabolism, amino acid and ammonia metabolism, hormone metabolism, and lipid metabolism; detoxification of exogenous drugs; and a hemodynamic function, including splanchnic and portal hemodynamics.

An effective amount of a combination described herein will also provide a sufficient concentration of the active agents.in the concentration when administered to a patient. A sufficient concentration of an active agent is a concentration of the agent in the patient's body necessary to prevent or combat the infection. Such an amount may be ascertained experimentally, for example by assaying blood concentration of the agent, or theoretically, by calculating bioavailability. The amount of an active agent sufficient to inhibit viral infection *in vitro* may be determined with a conventional assay for viral infectivity such as a replicon based assay, which has been described in the literature.

The invention also includes using pharmaceutical combinations comprising a compound of the invention and at least one additional active agent in prophylactic therapies. In the context of prophylactic or preventative treatment an effective amount of a compound of the invention is an amount sufficient to significantly decrease the patient's risk of contracting a hepatitis C infection.

Methods of treatment include providing certain dosage amounts of a compound of the invention and the at least one additional active agent to a patient. Dosage levels of each active agent of from about 0.1 mg to about 140 mg per kilogram of body weight per day are useful in the treatment of the above-indicated conditions (about 0.5 mg to about 7 g per patient per day). The amount of active ingredient that may be combined with the carrier materials to produce a single dosage form will vary depending upon the patient treated and the particular mode of administration. Dosage unit forms will generally contain between from about 1 mg to about 500 mg of each active agent. In certain embodiments 25 mg to 500 mg, or 25 mg to 200 mg of a compound of the invention are provided daily to a patient. When the additional active agent is NM 283 (valopicitabine), 100 mg to 1000 mg/day, or 200 mg to 800 mg/day, or 200 to 400 mg/ day of either of those agents are typically provided to the patient. When the additional active agent is VX-950,1000 mg to 3750 mg/day, or 1200 mg to 1800 mg/day are administered to the patient. Treatment regiments in which VX-950 is an additional active agent and about 350 to about 450 mg or about 700 to about 800 mg of VX-950 are administered to a patient three times per day or about 350 to about 450 mg or about 700 to about 800 mg is administered every 12 hours are particularly included in the invention.

Frequency of dosage may also vary depending on the compound used and the particular disease treated. However, for treatment of most infectious disorders, a dosage regimen of 4 times daily or less is preferred and a dosage regimen of 1 or 2 times daily is particularly preferred.

It will be understood, however, that the specific dose level for any particular patient will depend upon a variety of factors including the activity of the specific compound employed, the age, body weight, general health, sex, diet, time of administration, route of administration, and rate of excretion, drug combination and the severity of the particular disease undergoing therapy.

### VI. COMBINATION METHODS

Compounds of the invention may also be used in combination with other active ingredients useful in the inhibition of HCV or other co-morbid indications, as recognized by those skilled in the art. Such combinations are selected based on the condition to be treated, cross-reactivities of ingredients and pharmaco-properties of the combination.

The combination therapy may provide "synergy" and "synergistic effect", i.e. the effect achieved when the active ingredients used together is greater than the sum of the effects that results from using the compounds separately. A synergistic effect may be attained when the active ingredients are: (1) co-formulated and administered or delivered simultaneously in a combined formulation; (2) delivered by alternation or in parallel as separate formulations; or (3) by some other regimen. When delivered in alternation therapy, a synergistic effect may be attained when the compounds are administered or delivered sequentially, e.g., in separate tablets, pills or capsules, or by different injections in separate syringes. In general, during alternation therapy, an effective dosage of each active ingredient is administered sequentially, i.e. serially, whereas in combination therapy, effective dosages of two or more active ingredients are administered together.

The invention includes methods of treatment in which a compound or salt of the invention is provided together with one or more additional active agents. In certain embodiments the active agent (or agents) is an HCV protease inhibitor or HCV polymerase inhibitor. For example the protease inhibitor may be telaprevir (VX-950) and the polymerase inhibitor may be valopicitabine, or NM 107, the active agent which valopicitabine is converted into in vivo.

According to the methods of the invention, the compound of the invention and an additional active agent may be: (1) co-formulated and administered or delivered simultaneously in a combined formulation; (2) delivered by alternation or in parallel as separate formulations; or (3) by any other combination therapy regimen known in the art. When delivered in alternation therapy, the methods of the invention may comprise administering or delivering the compound of The invention and an additional active agent sequentially, *e.g.*, in separate solution, emulsion, suspension, tablets, pills or capsules, or by different injections in separate syringes. In general, during alternation therapy, an effective dosage of each active ingredient is administered sequentially, *i*.*e*., serially, whereas in simultaneous therapy, effective dosages of two or more active ingredients are administered together. Various sequences of intermittent combination therapy may also be used.

In certain embodiments method of treatment includes providing a patient with a compound of Formula I and an interferon such as a pegylated interferon or interferon gamma. The interferon may be the only compound provided with the compound of the invention or may be provided with an additional active agent that is not an interferon.

The invention methods of treatment and pharmaceutical combinations including compounds of the invention any one or combination of the following compounds and substances as an additional active agent:
Caspase inhibitors: IDN 6556 (Idun Pharmaceuticals)
Cyclophilin Inhibitors: NIM811 (Novartis) and DEBIO-025 (Debiopharm)
Cytochrome P450 monooxygenase inhibitors: ritonavir (WO 94/14436), ketoconazole, troleandomycin, 4-methyl pyrazole, cyclosporin, clomethiazole, cimetidine, itraconazole, fluconazole, miconazole, fluvoxamine, fluoxetine, nefazodone, sertraline, indinavir, nelfinavir, amprenavir, fosamprenavir, saquinavir, lopinavir, delavirdine, erythromycin, VX-944, and VX-497. Preferred CYP inhibitors include ritonavir, ketoconazole, troleandomycin, 4-methyl pyrazole, cyclosporin, and clomethiazole
Glucocorticoids: hydrocortisone, cortisone, prednisone, prednisolone, methylprednisolone, triamcinolone, paramethasone, betamethasone, and dexamethasone
Hematopoietins: hematopoietin-1 and hematopoietin-2. Other members of the hematopoietin superfamily such as the various colony stimulating factors (e.g. (e.g. G-CSF, GM-CSF, M-CSF), Epo, and SCF (stem cell factor)
Homeopathic Therapies: Milk Thistle, silymarin, ginseng, glycyrrhizin, licorice root, schisandra, vitamin C, vitamin E, beta carotene, and selenium
Immunomodulatory compounds: thalidomide, IL-2, hematopoietins, IMPDH inhibitors, for example Merimepodib (Vertex Pharmaceuticals Inc.), interferon, including natural interferon (such as OMNIFERON, Viragen and SUMIFERON, Sumitomo, a blend of natural interferons), natural interferon alpha (ALFERON, Hemispherx Biopharma, Inc.), interferon alpha nl from lymphblastoid cells (WELLFERON, Glaxo Wellcome), oral alpha interferon, Peg-interferon, Peg-interferon alfa 2a (PEGASYS, Roche), recombinant interferon alfa 2a (ROFERON, Roche), inhaled interferon alpha 2b (AERX, Aradigm), Peg-interferon alpha 2b (ALBUFERON, Human Genome Sciences/ Novartis, PEGINTRON, Schering), recombinant interferon alfa 2b (INTRON A, Schering), pegylated interferon alfa 2b (PEGINTRON, Schering, VIRAFERONPEG, Schering) ,interferon beta-1a (REBIF, Serono, Inc. and Pfizer), consensus interferon alpha (INFERGEN, Valeant Pharmaceutical), interferon gamma-1b (ACTIMMUNE, Intermune, Inc.), un-pegylated interferon alpha, alpha interferon, and its analogs, and synthetic thymosin alpha 1 (ZADAXIN, SciClone Pharmaceuticals Inc.)
Immunosupressants: sirolimus (RAPAMUNE, Wyeth)
Interleukins: (IL-1, IL-3, IL-4, IL-5, IL-6, IL-10, EL-11, IL-12), LIF, TGF-beta, TNF-alpha) and other low molecular weight factors (e.g. AcSDKP, pEEDCK, thymic hormones, and minicytokines)
Interferon Enhancers: EMZ702 (Transition Therapeutics)
IRES inhibitors: VGX-410C (VGX Pharma)
Monoclonal and Polyclonal antibodies: XTL-6865 (XTL), HuMax-HepC (Genmab), Hepatitis C Immune Globin (human) (CIVACIR, Nabi Biopharmceuticals)
Nucleoside analogues: Lamivudine (EPIVIR, 3TC, GlaxoSmithKline), MK-0608 (Merck), zalcitabine (HIVID, Roche US Pharmaceuticals), ribavirin (including COPEGUS (Roche), REBETOL (Schering), VILONA (ICN Pharmaceuticals, and VIRAZOLE (ICN Pharmaceuticals), and viramidine (Valeant Pharmaceuticals), an amidine prodrug of ribavirin. Combinations of nucleoside analogues may also be employed.
Non-nucleoside inhibitors: PSI-6130 (Roche/ Pharmasset), delaviridine (RESCRIPTOR, Pfizer), and HCV-796 (Viropharm)
P7 protein inhibitor: amantadine (SYMMETREL, Endo Pharmaceuticals, Inc.)
Polymerase inhibitors: NM283 (valopicitabine) (Idenix) and NM 107 (Idenix).
Protease inhibitors: BILN-2061 (Boehringer Ingelheim), GW-433908 (prodrug of Amprenavir, Glaxo/ Vertex), indinavir (CRIXIVAN, Merck), ITMN-191 (Intermune/ Array Biopharma), VX950 (Vertex) and combinations comprising one or more of the foregoing protease inhibitors
RNA interference: SIRNA-034 RNAi (Sirna Therapeutics)
Therapeutic Vaccines: IC41 (Intercell), IMN-0101 (Imnogenetics), GI 5005 (Globeimmune), Chronvac-C (Tripep/ Inovio), ED-002 (Imnogenetics), Hepavaxx C (ViRex Medical)
TNF agonists: adalimumab (HUMIRA, Abbott), entanercept (ENBREL, Amgen and Wyeth), infliximab (REMICADE, Centocor, Inc.)
Tubulin inhibitors: Colchicine
Sphingosine-1-phosphate receptor modulators: FTY720 (Novartis)
TLR agonists: ANA-975 (Anadys Pharmaceuticals), TLR7 agonist (Anadys Pharmaceuticals), CPG10101(Coley), andTLR9 agonists including CPG 7909 (Coley)
Cyclophilin Inhibitors: NIM811 (Novartis) and DEBIO-025 (Debiopharm)

Patients receiving hepatitis C medications are typically given interferon together with another active agent. Thus methods of treatment and pharmaceutical combinations in which a compound of The invention is provided together with an interferon, such as pegylated interferon alfa 2a, as the additional active agents are included as embodiments. Similarly methods and pharmaceutical combinations in which ribavirin is an additional active agent are provided herein.

### VI. METABOLITES OF COMPOUNDS OF THE INVENTION

Also falling within the scope of this invention are the in vivo metabolic products of the compounds described herein. Such products may result for example from the oxidation, reduction, hydrolysis, amidation, esterification and the like of the administered compound, primarily due to enzymatic processes. Accordingly, the invention includes compounds produced by a process comprising contacting a compound of this invention with a mammal for a period of time sufficient to yield a metabolic product thereof. Such products typically are identified by preparing a radiolabeled (e.g., C14 or H3) compound of the invention, administering it parenterally in a detectable dose (e.g., greater than about 0.5 mg/kg) to an animal such as rat, mouse, guinea pig, monkey, or to man, allowing sufficient time for metabolism to occur (typically about 30 seconds to 30 hours) and isolating its conversion products from the urine, blood or other biological samples. These products are easily isolated since they are labeled (others are isolated by the use of antibodies capable of binding epitopes surviving in the metabolite). The metabolite structures are determined in conventional fashion, e.g., by MS or NMR analysis. In general, analysis of metabolites is done in the same way as conventional drug metabolism studies well-known to those skilled in the art. The conversion products, so long as they are not otherwise found in vivo, are useful in diagnostic assays for therapeutic dosing of the compounds of the invention even if they possess no HIV -inhibitory activity of their own.

Methods for determining stability of compounds in surrogate gastrointestinal secretions are known. Compounds are defined herein as stable in the gastrointestinal tract where less than about 50 mole percent of the protected groups are deprotected in surrogate intestinal or gastric juice upon incubation for 1 hour at 37 °C. Simply because the compounds are stable to the gastrointestinal tract does not mean that they cannot be hydrolyzed in vivo.

### VII. EXEMPLARY METHODS OF MAKING THE COMPOUNDS OF THE INVENTION.

Generally, the reaction conditions such as temperature, reaction time, solvents, work-up procedures, and the like, will be those common in the art for the particular reaction to be performed. The cited reference material, together with material cited therein, contains detailed descriptions of such conditions. Typically the temperatures will be -100°C to 200°C, solvents will be aprotic or protic, and reaction times will be 10 seconds to 10 days. Work-up typically consists of quenching any unreacted reagents followed by partition between a water/organic layer system (extraction) and separating the layer containing the product.

Oxidation and reduction reactions are typically carried out at temperatures near room temperature (about 20 °C), although for metal hydride reductions frequently the temperature is reduced to 0 °C to -100 °C, solvents are typically aprotic for reductions and may be either protic or aprotic for oxidations. Reaction times are adjusted to achieve desired conversions.

Condensation reactions are typically carried out at temperatures near room temperature, although for non-equilibrating, kinetically controlled condensations reduced temperatures (0 °C to -100 °C) are also common. Solvents can be either protic (common in equilibrating reactions) or aprotic (common in kinetically controlled reactions).

Standard synthetic techniques such as azeotropic removal of reaction byproducts and use of anhydrous reaction conditions (e.g., inert gas environments) are common in the art and will be applied when applicable.

### VIII. SYNTHETIC SCHEMES

General aspects of these exemplary methods are described below and in the Examples. Each of the products of the following processes is optionally separated, isolated, and/or purified prior to its use in subsequent processes.

Generally, the reaction conditions such as temperature, reaction time, solvents, work-up procedures, and the like, will be those common in the art for the particular reaction to be performed. The cited reference material, together with material cited therein, contains detailed descriptions of such conditions. Typically the temperatures will be -100°C to 200°C, solvents will be aprotic or protic, and reaction times will be 10 seconds to 10 days. Work-up typically consists of quenching any unreacted reagents followed by partition between a water/organic layer system (extraction) and separating the layer containing the product.

Oxidation and reduction reactions are typically carried out at temperatures near room temperature (about 20 °C), although for metal hydride reductions frequently the temperature is reduced to 0 °C to -100 °C, solvents are typically aprotic for reductions and may be either protic or aprotic for oxidations. Reaction times are adjusted to achieve desired conversions.

Condensation reactions are typically carried out at temperatures near room temperature, although for non-equilibrating, kinetically controlled condensations reduced temperatures (0 °C to -100 °C) are also common. Solvents can be either protic (common in equilibrating reactions) or aprotic (common in kinetically controlled reactions).

Standard synthetic techniques such as azeotropic removal of reaction byproducts and use of anhydrous reaction conditions (e.g., inert gas environments) are common in the art and will be applied when applicable.

The terms "treated", "treating", "treatment", and the like, when used in connection with a chemical synthetic operation, mean contacting, mixing, reacting, allowing to react, bringing into contact, and other terms common in the art for indicating that one or more chemical entities is treated in such a manner as to convert it to one or more other chemical entities. This means that "treating compound one with compound two" is synonymous with "allowing compound one to react with compound two", "contacting compound one with compound two", "reacting compound one with compound two", and other expressions common in the art of organic synthesis for reasonably indicating that compound one was "treated", "reacted", "allowed to react", etc., with compound two. For example, treating indicates the reasonable and usual manner in which organic chemicals are allowed to react. Normal concentrations (0.01M to 10M, typically 0.1M to 1M), temperatures (-100 °C to 250 °C, typically -78 °C to 150 °C, more typically -78 °C to 100 °C, still more typically 0 °C to 100 °C), reaction vessels (typically glass, plastic, metal), solvents, pressures, atmospheres (typically air for oxygen and water insensitive reactions or nitrogen or argon for oxygen or water sensitive), etc., are intended unless otherwise indicated. The knowledge of similar reactions known in the art of organic synthesis are used in selecting the conditions and apparatus for "treating" in a given process. In particular, one of ordinary skill in the art of organic synthesis selects conditions and apparatus reasonably expected to successfully carry out the chemical reactions of the described processes based on the knowledge in the art.

Modifications of each of the exemplary schemes and in the examples (hereafter "exemplary schemes") leads to various analogs of the specific exemplary materials produce. The above-cited citations describing suitable methods of organic synthesis are applicable to such modifications.

In each of the exemplary schemes it may be advantageous to separate reaction products from one another and/or from starting materials. The desired products of each step or series of steps is separated and/or purified (hereinafter separated) to the desired degree of homogeneity by the techniques common in the art. Typically such separations involve multiphase extraction, crystallization from a solvent or solvent mixture, distillation, sublimation, or chromatography. Chromatography can involve any number of methods including, for example: reverse-phase and normal phase; size exclusion; ion exchange; high, medium, and low pressure liquid chromatography methods and apparatus; small scale analytical; simulated moving bed (SMB) and preparative thin or thick layer chromatography, as well as techniques of small scale thin layer and flash chromatography.

Another class of separation methods involves treatment of a mixture with a reagent selected to bind to or render otherwise separable a desired product, unreacted starting material, reaction by product, or the like. Such reagents include adsorbents or absorbents such as activated carbon, molecular sieves, ion exchange media, or the like. Alternatively, the reagents can be acids in the case of a basic material, bases in the case of an acidic material, binding reagents such as antibodies, binding proteins, selective chelators such as crown ethers, liquid/liquid ion extraction reagents (LIX), or the like.

Selection of appropriate methods of separation depends on the nature of the materials involved. For example, boiling point, and molecular weight in distillation and sublimation, presence or absence of polar functional groups in chromatography, stability of materials in acidic and basic media in multiphase extraction, and the like. One skilled in the art will apply techniques most likely to achieve the desired separation.

More particularly, the compounds of the invention may be produced in any manner known in the art. By way of example, certain compounds of the invention may be produced as generally illustrated. The various substituted aminothiazoles of the invention may be synthesized as shown the schemes below.

As shown in the above reaction schemes, the aminothiazoles may be prepared by the reaction between a alpha-haloketone and thiourea. There are several methods known in literature for the synthesis of alpha-haloketone, and a few have been illustrated in scheme 1. However, the invention is not so limited. The thioureas may be prepared from the corresponding amines or anilines by reacting them with thiocarbonyldiimidazole followed by ammonia. Other methods known in literature may also be used to prepare thioureas. The aminothiazoles may then be converted to their corresponding, i) amides, by reacting it with activated carboxylic acids, ii) carbamates, by reacting it with alkoxy/aryloxy carbonylchlorides, iii) ureas, by reacting it with isocyanates and iv) formyl, by reacting it with Formaic acid/Acetic anhydride as shown in scheme 2.

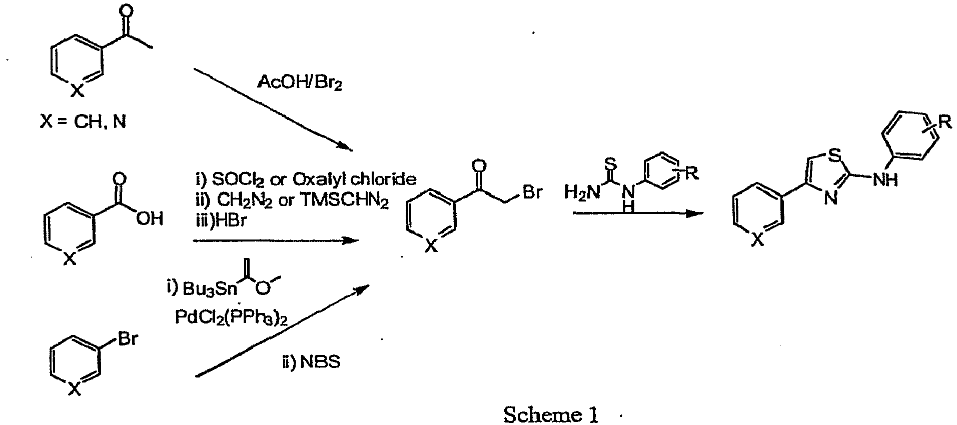

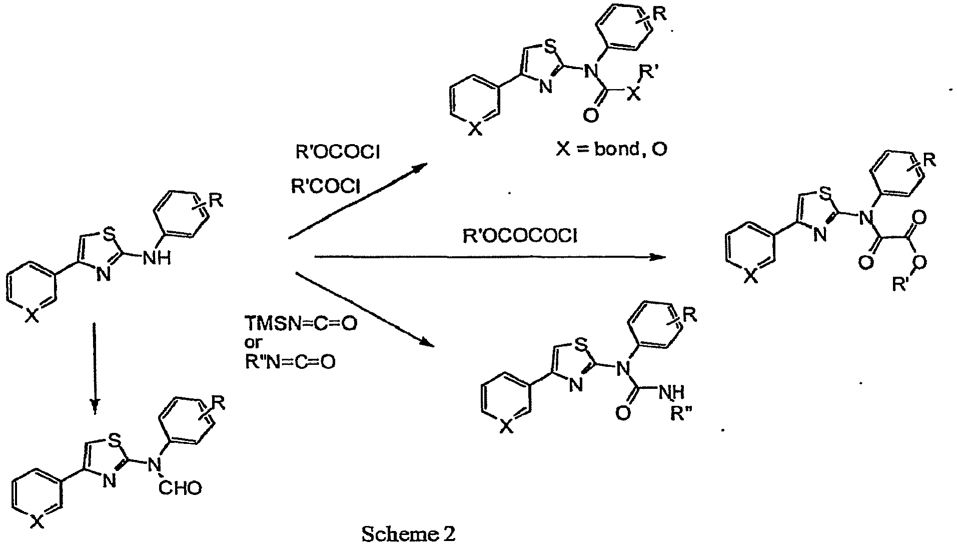

Schemes 3 and 4 then describe the synthesis of carboxylic acid substituted thiazoles and aminomethylene substituted thiazoles.

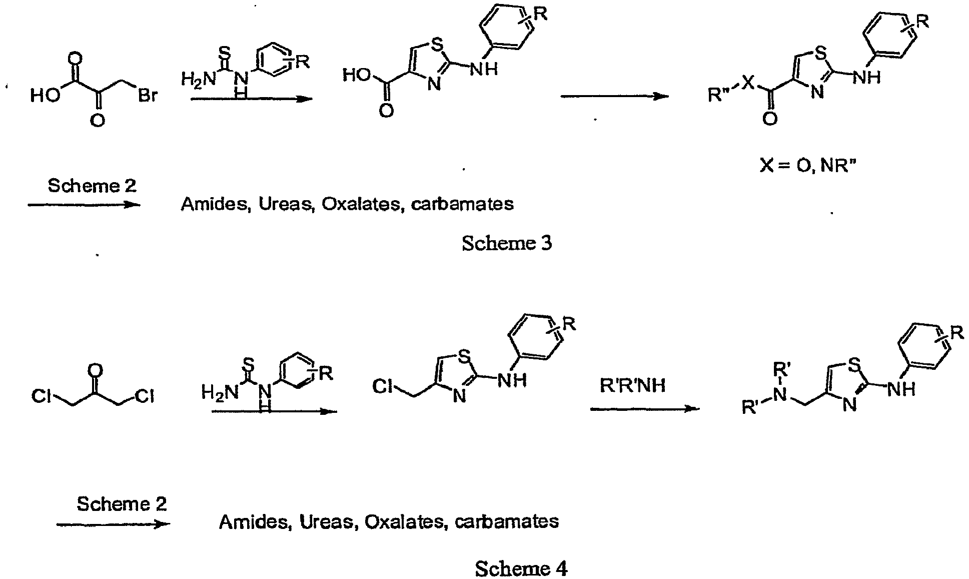

These and other reaction methodologies may be useful in preparing the compounds of the invention, as recognized by one of skill in the art. Various modifications to the above schemes and procedures will be apparent to one of skill in the art, and the invention is not limited specifically by the method of preparing the compounds of the invention.

### EXAMPLES

### EXAMPLE 1. SYNTHETIC AND ANALYTICAL METHODS

All nonaqueous reactions are performed under an atmosphere of dry argon gas (99.99%). NMR spectra are recorded at ambient temperature using a Bruker Avance 300 spectrometer (¹H at 300.1 MHz and ¹³C at 75.5 MHz,). The chemical shifts for ¹H and ¹³C are reported in parts per million (*δ*) relative to external tetramethylsilane and are referenced to signals of residual protons in the deuterated solvent. Analytical HPLC is performed using a Waters X-bridge C18 150 x 4.6 mm 3.5 *µ*m column with a 20-min linear gradient elution of increasing concentrations of acetonitrile in water (5 to 95%) containing 0.1% trifluoroacetic acid with a flow rate of 1.0 mL/min and UV detection at 254 nm. Low-resolution mass spectra are recorded on a Thermo Finnigan Surveyor MSQ instrument (operating in APCI mode) equipped with a Gilson liquid chromatograph. Unless noted otherwise, the quasi-molecular ions, [M + H]⁺, observed in the low-resolution mass spectra are the base peaks.

This invention is further illustrated by the following examples that should not be construed as limiting.

### EXAMPLE 2. SYNTHESIS OF EXEMPLARY COMPOUNDS

General procedures for the synthesis of the compounds below is shown in Scheme 5. Additional compounds were synthesized according to similar procedures, as shown in TABLE A.

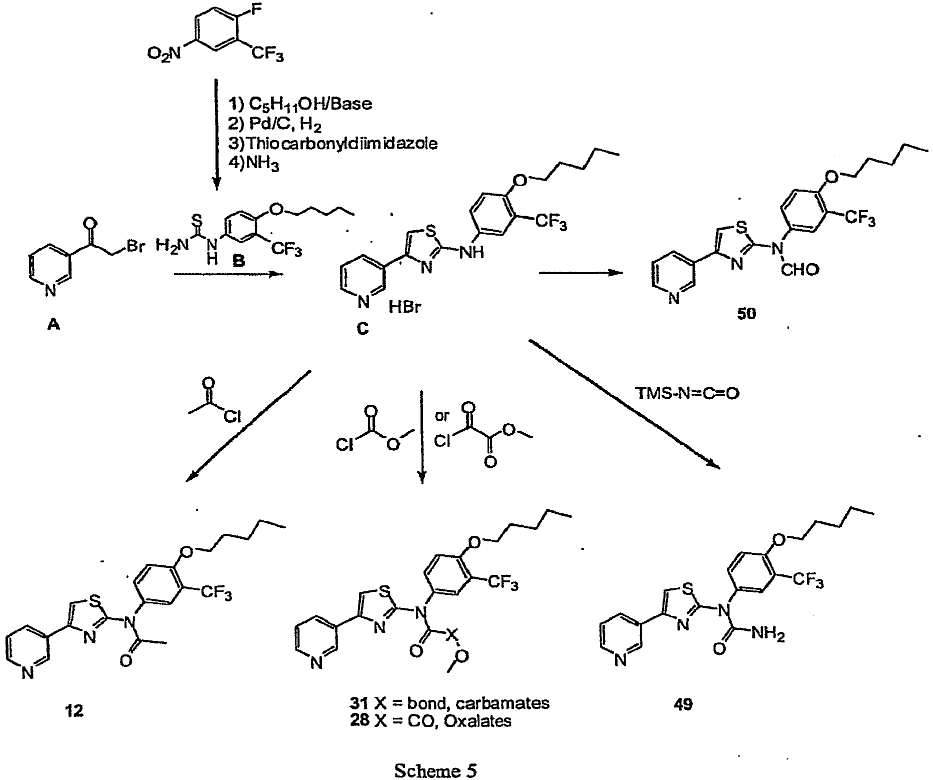

### Compound C

3-Bromoacetylpyridine (**A**, 1 equi) and the thiourea **B** (1 equi) were heated between 50-70 °C in ethanol for 4-8h. On cooling the reaction mixture a yellow solid precipitated. The solid was filtered and washed with minimum ethanol and dried to afford the amniothiazole (**C**) in 70% yield.

¹HNMR (CDCl₃) δ: 8.98 (d, 1H), 8.53 (dd, 1H), 8.06 (dt, 1H), 7.42 (s, 1H), 7.29 (dd, 1H), 7.13 (m,1H), 7.03 (dd, 1H), 6.78 (d, 1H), 3.90 (t, 2H),1.73 (m, 2H), 1.35 (m, 4H), 0.85 (t, 3H). MS: 408 (M⁺+1).

### Compounds 12. 28. 31

Compound C (1 equi) was treated with a slight excess of the corresponding acid chloride (acetyl chloride, methyloxalylchloride, methyl chloroformate) in Methylene chloride in presence of a base like pyridine or triethylamine. DMAP may also be added to the reaction. The reaction can also be done in Pyridine. The reaction was stirred at room temperature over night. The solvent was evaporated, residue suspended in water and extracted with methylene chloride, dried and evaporated. The residue was purified by crystallization or by column chromatography over silca gel or by preparative HPLC.

### Compound 12

NMR (CDCl3)δ: 0.87 (t, 3H), 1.39 (m, 4H), 1.80 (m, 2H), 2.02 (s, 3H), 4.06 (t, 2H), 7.04 (d, 1H), 7.13 (dd, 1H), 7.19 (s, 1H), 7.38 (dd, 1H), 7.47 (brS, 1H), 7.78 (dt, 1H), 8.36 (d, 1H), 8.81 (s, 1H). MS: 450 (M⁺+1)

### Compound 28

¹HNMR (CDCl3) δ: 9.06(d, 1H), 8.66 (dd, 1H), 8.40 (dd, 1H), 7.70 (dd, 1H), 7.52 (m, 2H), 7.61 (s, 1H), 7.08 (d,1H), 4.09 (t, 2H), 3.62 (s, 3H), 1.82 (m, 2H), 1.40 (m, 4H), 0.83 (t, 3H).

### Compound 31

¹HNMR (CDCl3) δ: 9.12 (s, 1H), 8.71 (d, 1H), 8.42 (dt,1H), 7.78 (dd, 1H), 7.60 (s, 1H), 7.52 (d, 1H), 7.45 (dd,1H), 7.13(d, 1H), 4.15 (t, 2H), 3.87 (s, 3H), 1.95 (m, 2H), 1.50 (m, 4H), 1.04 (t, 3H) ppm. MS: 507 (M⁺+1)

### Compound 49

The aminothiazole C (1 equi) and trimethylsilyl isocynate (4 equi) were heated at 60°C in THF overnight. The solvent was removed and the remaining residue was purified by HPLC to give **49**.

¹HNMR (CDCl₃): 9.13 (d, 1H), 8.71 (dd, 1H), 8.42 (dt,1H), 7.75 (dd, 1H), 7.64 (d, 1H), 7.57 (dd, 1H), 7.41 (s,1H,), 7.18(d, 1H), 5.96 (s, 2H), 4.20 (t, 2H), 1.95 (m, 2H), 1.50 (m, 4H), 1.04 (t, 3H) ppm. MS: 451 (M⁺+1)

### Compound 50

The aminothiazole C (1 equi) and formaldehyde and acetic anhydride were heated at 60°C overnight. The solvent was removed and the remaining residue was purified by HPLC to give **50**.

¹HNMR (CDCl₃): 9.16 (s,1H), 8.72 (2H), 8.45 (d,1H), 7.73 (m, 1H,), 7.61 (d-like, 3H), 7.21 (d, 1H), 4.20 (t, 2H), 1.95 (m, 2H), 1.50 (m, 4H), 1.04 (t, 3H) ppm. MS: 436 (M⁺+1)

### Compounds 20 and 30

Thiazole carboxylic acid and chloromethylene thiazole were prepared by reacting the corresponding bromoketones with thiourea as shown in schemes 2 and 3 following procedure described for compound C. The acids were then converted to the corresponding amides following standard conditions. The chloromethylene thiazoles were reacted with different amines in presence of a base in DMF followed by purification by HPLC to afford the animo compounds. These were derivatized as described above.

### Compound 20:

MS: 430 (M⁺+1)

### Compound 30:

¹HNMR (CDCl₃) δ: 9.11 (s, 1H), 9.05 (s, 1H), 8.6 (d, 1H), 8.46 (d, 1H), 8.09 (s, 1H), 7.7 (m, 2H), 7.53 (d, 1H), 7.11 (d, 1H), 4.09 (t, 2H), 3.62 (s, 3H), 1.82 (m, 2H), 1.39 (m, 4H), 0.87 (t, 3H). MS: 537 (M⁺+1)

### EXAMPLE 3. ASSAY FOR IDENTIFYING COMPOUNDS WHICH INHIBIT HCV REPLICATION

Compounds claimed herein are tested for the ability to inhibit viral replication of the Hepatitis C replicon in cultured cells in which the HCV replicon construct has been incorporated. The HCV replicon system was described by Bartenschlager, et. al (Science, 285, pp. 110-113 (1999)). The replicon system is predictive of in vivo anti-HCV activity; compounds that are active in humans uniformly evidence activity in the replicon assay.

In this assay HCV replicon containing cells are treated with different concentrations of the test compound to ascertain the ability of the test compound to suppress replication of the HCV replicon. As a positive control, HCV replicon-containing cells are treated with different concentrations of interferon alpha, a known inhibitor of HCV replication. The replicon assay system includes Neomycin Phosphotransferase (NPT) as a component of the replicon itself in order to detect the transcription of replicon gene products in the host cell. Cells in which the HCV replicon is actively replicating have high levels of NPT; the level of NPT is proportional to HCV replication. Cells in which the HCV replicon is not replicating also have low levels of NPT and thus do not survive when treated with Neomycin. The NPT level of each sample is measured using a captured ELISA.

A protocol for testing compounds for the ability to inhibit viral replication of the Hepatitis C replicon cultured cells in which the replicon construct has been incorporated, follows.

### 3A. HCV Replicon and Replicon Expression

The HCV genome consists of a single ORF that encodes a 3000 amino acid polyprotein. The ORF is flanked on the 5' side by an untranslated region that serves as an internal ribosome entry site (IRES) and at the 3' side by a highly conserved sequence necessary for viral replication (3'-NTR). The structural proteins, necessary for viral infection, are located near the 5' end of the ORF. The non-structural proteins, designated NS2 to NS5B comprise the remainder of the ORF.

The HCV replicon contains, 5'-3', the HCV-IRES, the neomycin phosphotransferase (neo) gene, the IRES of encephalomyocarditis virus, which directs translation of HCV sequences NS3 to NS5B, and the 3'-NTR. The sequence of the HCV replicon has been deposited in GenBank (Accession no. AJ242652).

The replicon is transfected into Huh-7 cells using standard methods such as electroporation.

### 3B. Cell Maintenance

The equipment and materials include, but are not limited to, Huh-7 HCV replicon-containing cells, maintenance media (DMEM (Dulbecco's modified Eagle media) supplemented with 10% FBS, L-glutamine, non-essential amino acids, penicillin (100 units/ml), streptomycin (100 micrograms/ml), and 500 micrograms/ml of Geneticin (G418), screening media (DMEM supplemented with 10% FBS, L-glutamine, non-essential amino acids, penicillin (100 units/ml) and streptomycin (100 micrograms/ml)), 96 well tissue culture plates (flat bottom), 96 well plates (U bottom for drug dilution), Interferon alpha for positive control, fixation reagent (such as methanol: acetone), primary antibody (rabbit anti-NPTII), secondary antibody: Eu-N1 l, and enhancement solution.

HCV replicon-containing cells support high levels of viral RNA replicon replication when their density is suitable. Over-confluency causes decreased viral RNA replication. Therefore, cells must be kept growing in log phase in the presence of 500 micrograms/ml of G418. Generally, cells should be passed twice a week at 1: 4-6 dilution. Cell maintenance is conducted as follows:

HCV replicon-containing cells are examined under a microscope to ensure that cells growing well. Cells are rinsed once with PBS and 2 ml trypsin is added. The cell/trypsin mixture is incubated at 37 °C in a CO₂ incubator for 3-5 minutes. After incubation 10 ml of complete media is added to stop the trypsinization reaction. Cells are blown gently, put into a 15 ml tube, and spun at 1200 rpm for 4 minutes. The trypsin/ medium solution is removed. Medium (5 ml) is added and the cells are mixed carefully. The cells are counted.

The cells are then seeded onto 96-well plates at a density of 6000-7500 cells/100 microliters/ well (6-7.5 x 10⁵ cells/10 ml/plate). The plates are then incubated at 37 oC in a 5% CO₂ incubator.

Cells are examined under a microscope approximated 24 hours after seeding and prior to adding drugs. If counting and dilution were performed correctly, cells are 60-70% confluent and nearly all cells should attach and spread evenly in the well.

### 3C. Treatment of HCV-replicon containing cells with Test Compound

HCV replicon-containing cells are rinsed with once PBS once; 2 mls of trypsin are then added. Cells are incubated at 37°C in a 5% CO₂ incubator for 3-5 minutes. 10 mls of complete medium is added to stop the reaction. Cells are blown gently, put into a 15 ml tube, and spun at 1200 rpm for four minutes. The trypsin/medium solution is removed and 5 mls of medium (500 ml DMEM (high glucose)) from BRL catalog #12430-054; 50 mls 10% FBS, 5% Geneticin G418 (50 mg/ml, BRL catalog #10131-035), 5 ml MEM non-essential amino acids (100x BRL #11140-050) and 5 ml pen-strep (BRL #15140-148) is added. The cells and media are mixed carefully

Cells are plated with screening medium (500 ml DMEM (BRL #21063-029), 50 ml FBS (BRL #10082-147) and 5 ml MEM non-essential amino acid (BRL #11140-050) at 6000-7500 cells/100 µl/well of 96 well plate (6-7.5x105 cells/10 ml/plate). Plates are placed into 37°C 5% CO₂ incubator overnight.

### 3D. Assay

The following morning, drugs (test compounds or interferon alpha) are diluted in 96 well U bottom plates with media or DMSO/media, depending on the final concentration chosen for screening. Generally for 6 concentrations of each test compounds ranging from 10 micromolar to 0.03 micromolar are applied. 100 µl of the test compound dilution is placed in wells of the 96 well plate containing the HCV replicon cells. Media without drug is added to some wells as a negative controls. DMSO is known to affect cell growth. Therefore, if drugs diluted in DMSO are used, all wells, including negative control (media only) and positive control (interferon alpha) wells, must contain the same concentration of DMSO, for single dose screening. The plates are incubated at 37°C in a humidified 5% CO₂ environment for three days.

On day four, the NTPII assay is quantitated. The medium is poured from the plates and the plates are washed once in 200 µl of PBS. The PBS is then decanted and the plates tapped in a paper towel to remove any remaining PBS. Cells are fixed in situ with 100 µl/well of pre-cooled (-20°C) methanol: acetone (1:1) and the plates are placed at -20°C for 30 minutes.

The fixing solution is poured from the plates and the plates allowed to air-dry completely (approximately one hour). The appearance of the dried cell layer is recorded and the density of the cells in the toxic wells is scored with the naked eye. Alternatively cell viability may be assessed using the MTS assay described below.

The wells are blocked with 200 µl of blocking solution (10% FBS; 3% NGS in PBS) for 30 minutes at room temperature. The blocking solution is removed and 100 µl of rabbit anti-NPTII diluted 1:1000 in blocking solution is added to each well. The plates are then incubated 45-60 minutes at room temperature. After incubation, wells are washed six times with PBS-0.05% Tween-20 solution. 100 µl of 1:15,000 diluted Europium (EU)-conjugated goat anti-rabbit in blocking buffer is added to each well and incubated at room temperature for 30-45 minutes. The plates are washed again and 100 µl of enhancement solution (Perkin Elmer #4001-0010) is added to each well. Each plate is shaken (approx. 30 rpm) in a plate shaker for three minutes. 95 µl is transferred from each well to a black plate; the EU signal is quantitated in a Perkin-Elmer VICTOR plate reader (EU-Lance).

*Test Results:*

Compounds described in TABLE A have been tested in an HCV replication assay, essentially as described in this example. In that table +++ indicates inhibition of HCV replicon replication of less than 1 micromolar, ++ indicates inhibition of less than 10 micromolar to 1 micromolar, and + indicates inhibition greater than 10 micromolar.

### EXAMPLE 4. CYTOTOXICITY ASSAYS

To insure that the decrease in replicon replication is due to compound activity against the HCV replicon rather than nonspecific toxicity assays are used to quantitate compound cytotoxicity.

### Example 4A. Cellular protein albumin assay for cytotoxicity

Cellular protein albumin measurements provide one marker of cytotoxicity. The protein levels obtained from cellular albumin assays may also be used to provide a normalization reference for antiviral activity of compounds. In the protein albumin assay HCV replicon-containing cells are treated for three days with different concentrations of helioxanthin; a compound that is known to be cytotoxic at high concentrations. The cells are lysed and the cell lysate used to bind plate-bound goat anti-albumin antibody at room temperature (25 °C to 28 °C) for 3 hours. The plate is then washed 6 times with 1X PBS. After washing away the unbound proteins, mouse monoclonal anti-human serum albumin is applied to bind the albumin on the plate. The complex is then detected using phosphatase-labeled anti-mouse IgG as a second antibody.

### Example 4B. MTS Assay for Cytotoxicity

Cell *viability* may also be determined by CELLTITER 96 AQUEOUS ONE Solution Cell Proliferation Assay (Promega, Madison WI), a colorimetric assay for determining the number of viable cells. In this method, before fixing the cells, 10-20 µl MTS reagent is added to each well according to manufacturer's instructions, plates are incubated at 37°C and read at OD 490 nm. During the incubation period living cells covert the MTS reagent to a formazan product which absorbs at 490 nm. Thus the 490nm absorbance is directly proportional to the number of living cells in culture.

A direct comparison of the Cellular Album and MTS methods for determining cytotoxicity may be obtained as follows: Cells are treated with different concentrations of test compound or Helioxanthin for a three day-period. Prior to lysis for detection album as described above, the MTS reagent is added according to manufacturer's instruction to each well and incubate at 37 °C and read at OD 490 nm. The cellular album quantitation is then performed as described above.

**TABLE A**

| **No.** | **Compound** | **NAME** | **Ret. ime** | **M+1** | **EC50** |
|---|---|---|---|---|---|
| **1** | 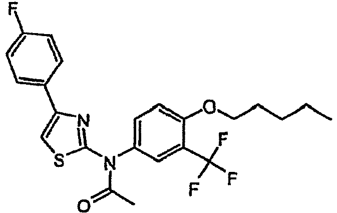 | N-(4-(4-fluorophenyl)thiazol-2-yl)-N-(4-(pentyloxy)-3-(trifluoromethyl)phenyl)acetamide | 5.03 | 467 | **+++** |
| **2** | 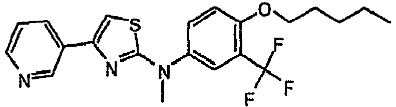 | N-methyl-N-(4-(pentyloxy)-3-(trifluoromethyl)phenyl)-4-(pyridin-3-yl)thiazol-2-amine | 1.54 | 422 | **+++** |
| **3** | 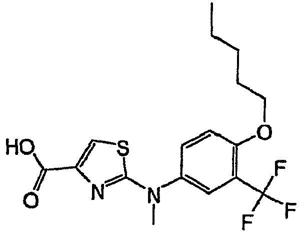 | 2-(methyl(4-(pentyloxy)-3-(trifluoromethyl)phenyl)amino)thiazole-4-carboxylic acid | 2.01 | 389 | **+** |
| **4** | 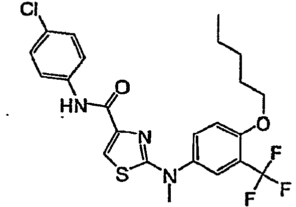 | N-(4-chlorophenyl)-2-(methyl(4-(pentyloxy)-3- (trifluoromethyl)phenyl)amino)thiazole-4-carboxamide | 3.06 | 499 | **++** |
| **5** | 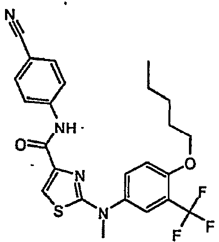 | N-(4-cyanophenyl) 2-(methyl(4.. (pentyloxy)-3-(trifluoromethyl)phenyl)amino)thiazole-4-carboxamide | 2.77 | 489 | **+** |
| **6** | 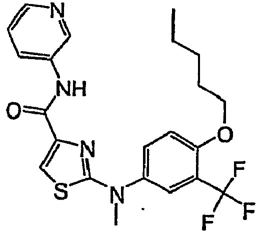 | 2-(methyl(4-(pentyloxy)-3-(trifluoromethyl)phenyl)amino)-N-(pyridin-3-yl)thiazole-4-carboxamide | 1.59 | 465 | **++** |
| **7** | 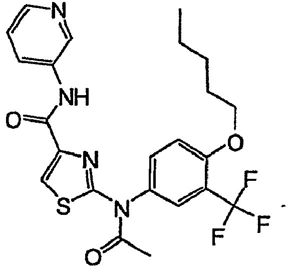 | 2-(N-(4-(pentyloxy)-3-(trifluoromethyl)phenyl)acetamido)-N-(pyridin-3-yl)thiazole-4-carboxamide | 1.42 | 493 | **++** |
| **8** | 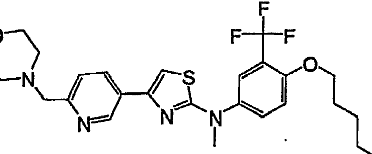 | N-methyl-4-(6-(morpholinomethyl)pyridin-3-yl)-N-(4-(pentyloxy)-3-(trifluoromethyl)phenyl)thiazol-2-amine | 1.60 | 521 | **++** |
| **9** | 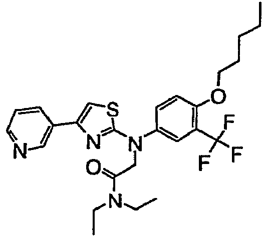 | N,N-diethyl-2-((4-(pentyloxy)-3-(trifluoromethyl)phenyl)(4-(pyridin-3-yl)thiazol-2-yl)amino)acetamide | 2.38 | 521 | **+** |
| **10** | 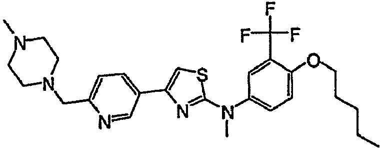 | N-methyl-4-(6-((4-methylpiperazin-1-yl)methyl)pyridin-3-yl)-N-(4-(pentyloxy)-3-(trifluoromethyl)phenyl)thiazol-2-amine | 1.31 | 534 | **+++** |
| **11** | 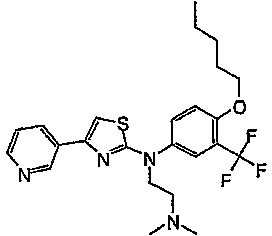 | N1,N1-dimethyl-N2-(4-(pentyloxy)-3-(trifluoromethyl)phenyl)-N2-(4-(pyridin-3-yl)thiazol-2-yl)ethane-1,2-diamine | 2.34 | 478 | **++** |
| **12** | 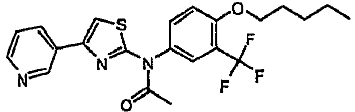 | N-(4-(pentyloxy)-3-(trifluoromethyl)phenyl)-N-(4-(pyridin-3-yl)thiazol-2-yl)acetamide | 2.27 | 450 | **+++** |
| **13** | 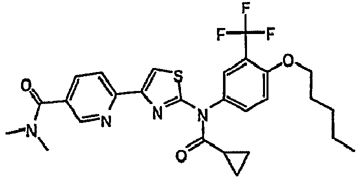 | N,N-dimethyl-6-(2-(N-(4-(pentyloxy)-3-(trifluoromethyl)phenyl)cyclopropaneca rboxamido)thiazol-4-yl)nicotinamide | 2.38 | 547 | **+** |
| **14** | 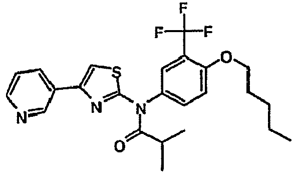 | N-(4-(pentyloxy)-3-(trifluoromethyl)phenyl)-N-(4-(pyridin-3-yl)thiazol-2-yl)isobutyramide | 1.93 | 478 | **+++** |
| **15** | 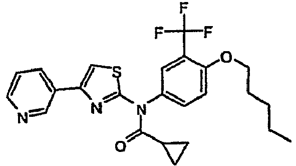 | N-(4-(pentyloxy)-3-(trifluoromethyl)phenyl)-N-(4-(pyridin-3-yl)thiazol-2-yl)cyclopropanecarboxamide | 1.82 | 476 | **+++** |
| **16** | 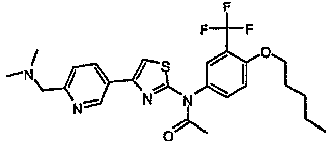 | N-(4-(6-((dimethylamino)methyl)pyridin-3-yl)thiazol-2-yl)-N-(4-(pentyloxy)-3-(trifluoromethyl)phenyl)acetamide | 1.52 | 507 | **++** |
| **17** | 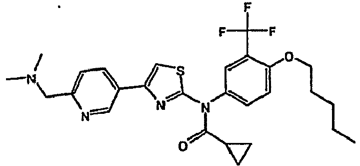 | N-(4-(6-((dimethylamino)methyl)pyridin-3-yl)thiazol-2-yl)-N-(4-(pentyloxy)-3-(trifluoromethyl)phenyl)cyclopropane-carboxamide | 1.65 | 533 | **++** |
| **18** | 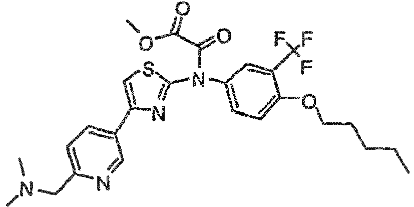 | methyl 2-((4-(6-((dimethylamino)methyl)pyridin-3-yl)thiaxol-2-yl)(4-(pentyloxy)-3-(trifluoromethyl)phenyl)amino)-2-oxoacetate | 1.58 | 551 | |
| **19** | 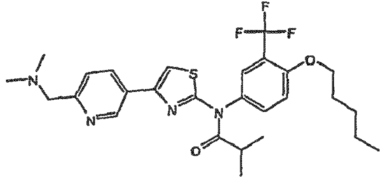 | N-(4-(6-((dimethylamino)methyl)pyridin-3-yl)thiazol-2-yl)-N-(4-(pentyloxy)-3-(trifluoromethyl)phenyl)isobutyramide | 1.77 | 535 | **++** |
| **20** | 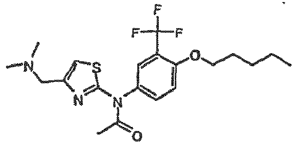 | N-(4-((dimethylamino)methyl)thiazol-2-yl)-N-(4-(pentyloxy)-3-(trifluoromethyl)phenyl)acetamide | 1.40 | 430 | **++** |
| **21** | 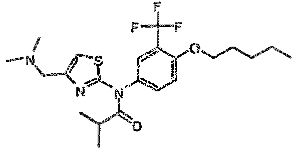 | N-(4-((dimethylamino)methyl)thiazol-2-yl)-N-(4-(pentyloxy)-3-(triluoromethyl)plienyl)isobutyramide | 1.56 | 458 | **++** |
| **22** | 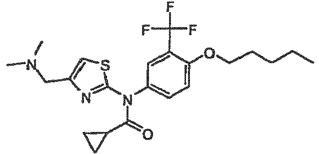 | N-(4-((dimethylammo)methyl)thiazol-2-yl)-N-(4-(pentyloxy)-3-carboxamide | 1.52 | 456 | **++** |
| **23** | 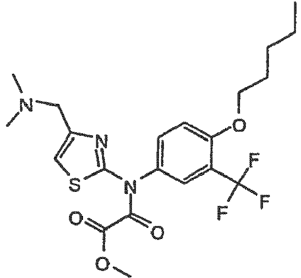 | methyl 2-((4-((dimethylamino)methyl)thiazol-2-yl)(4-(pentyloxy)-3-(trifluoromethyl)phenyl)amino)-2-oxoacetate | 1.48 | 474 | **++** |
| **24** | 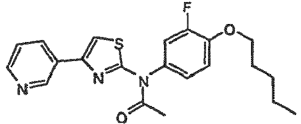 | N-(3-fluoro-4-(pentyloxy)phenyl)-N-(4-(pyridin-3-yl)thiazol-2-yl)acetamide | 1.43 | 400 | **+++** |
| **25** | 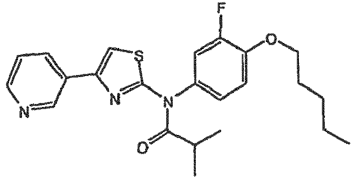 | N-(3-fluoro-4-(pentyloxy)phenyl)-N-(4-(pyridin-3-yl)thiazol-2-yl)isobutyramide | 1.63 | 428 | **+++** |
| **26** | 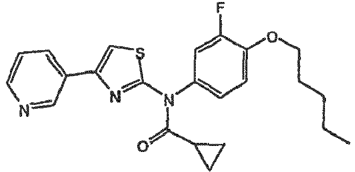 | N-(3-fluoro-4-(pentyloxy)phenyl)-N-(4-(pyridin-3-yl)thiazol-2-yl)cyclopropanecarboxamide | 1.59 | 426 | **+++** |
| **27** | 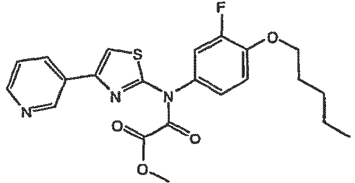 | methyl 2-((3-fluoro-4-(pentyloxy)phenyl)(4-(pyridin-3-yl)thiazol-2-yl)amino)-2-oxoacetate | 1.47 | 444 | **+++** |
| **28** | 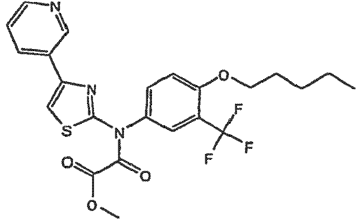 | methyl 2-oxo-2-((4-(pentyloxy)-3-(trifluoromethyl)phenyl)(4-(pyridin-3-yl)thiazol-2-yl)amino)acetate | 1.62 | 494 | **+++** |
| **29** | 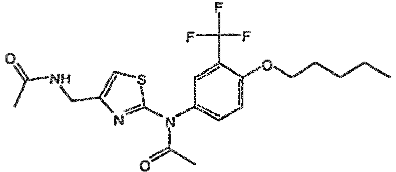 | N-(4-(acetamidomethyl)thiazol-2-yl)-N-(4-(pentyloxy)-3-(trifluoromethyl)phenyl)acetamide | 1.79 | 444 | **++** |
| **30** | 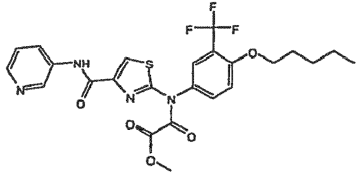 | methyl 2-oxo-2-((4-(pentyloxy)-3-(trifluoromethyl)phenyl)(4-(pyridin-3-ylcarbamoyl)thiazol-2-yl)amino)acetate | 1.47 | 537 | **+++** |
| **31** | 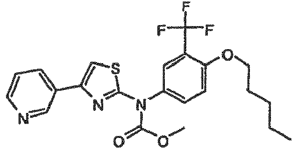 | methyl 4-(pentyloxy)-3-(trifluoromethyl)phenyl(4-(pyridin-3-yl)thiazol-2-yl)carbamate | 1.77 | 466 | **+++** |
| **32** | 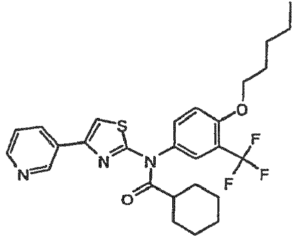 | N-(4-(pentyloxy)-3-(trifluoromethyl)phenyl)-N-(4-(pyridin-3-yl)thiazol-2-yl)cyclohexanecarboxamide | 7.74 | 518 | **+++** |
| **33** | 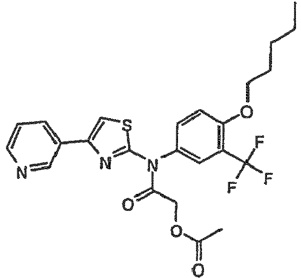 | 2-oxo-2-((4-(pentyloxy)-3-(trifluoromethyl)phenyl)(4-(pyridin-3-yl)thiazol-2-yl)amino)ethyl acetate | 4.99 | 508 | **+++** |
| **34** | 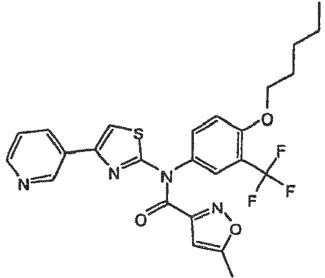 | 5-methyl-N-(4-(pentyloxy)-3-(trifluoromethyl)phenyl-N-(4-(pyridin-3-yl)thiazol-2-yl)isoxazole-3-carboxamide | 1.53 | 517 | **+++** |
| **35** | 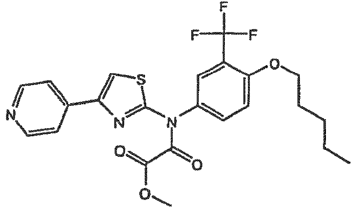 | methyl 2-oxo-2-((4-(pentyloxy)-3-(trifluoromethyl)phenyl)(4-(pyridin-4-yl)thiazol-2-yl)amino)acetate | 1.46 | 494 | **+++** |
| **36** | 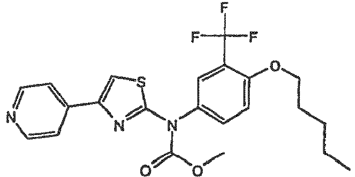 | methyl 4- entyloxy) 3-(trifluoromethyl)phenyl(4-(pyridin-4-yl)thiazol-2-yl)carbamate | 1.49 | 466 | **+++** |
| **37** | 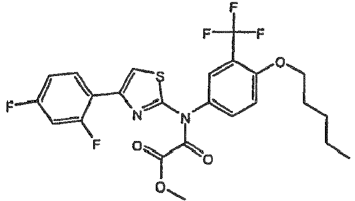 | methyl 2-((4-(2,4-difluorophenyl)thiazol-2-yl)(4-(pentyloxy)-3-(trifluoromethyl)phenyl)amino)-2-oxoacetate | 2.89 | 529 | **+++** |
| **38** | 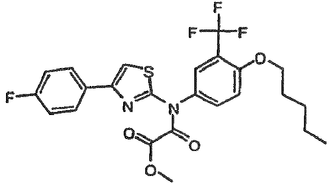 | methyl 2-((4-(4-fluorophenyl)thiazol-2-yl)(4-(pentyloxy)-3-(trifluoromethyl)phenyl)amino)-2-oxoacetate | 2.74 | 511 | **+++** |
| **39** | 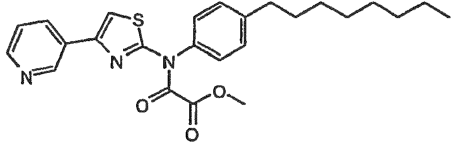 | methyl 2-((4-octylphenyl)(4-(pyridin-3-yl)thiazol-2-yl)amino)-2-oxoacetate | | | **+++** |
| **40** | 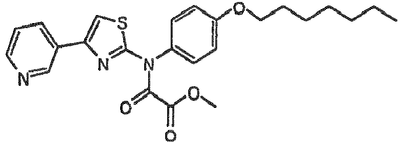 | methyl 2-((4-(heptyloxy)phenyl)(4-(pyridin-3-yl)thiazol-2-yl)amino)-2-oxoacetate | 1.57 | 454 | **+++** |
| **41** | 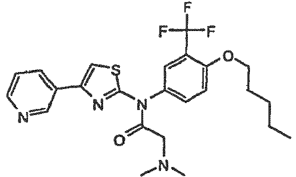 | 2-(dimethylamino)-N-(4-(pentyloxy)-3-(trifluoromethyl)phenyl)-N-(4-(pyridin-3-yl)thiazol-2-yl)acetamide | 0.58 | 493 | **+++** |
| **42** | 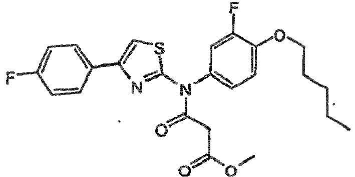 | methyl 3-((3-fluoro-4-(pentyloxy)phenyl)(4-(4-fluorophenyl)thiazol-2-yl)amino)-3-oxopropanoate | 2.63 | 475 | **+++** |
| **43** | 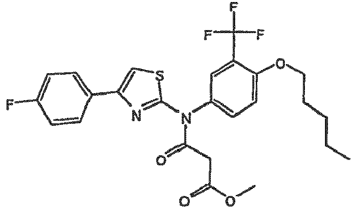 | methyl 3-((4-(4-fluorophenyl)thiazol-2-yl)(4-(pentyloxy)-3-(trifluoromethyl)phenyl)amino)-3-oxopropanoate | 2.76 | 525 | **+++** |
| **44** | 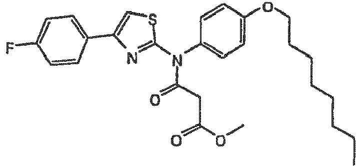 | methyl 3-((4-(4-fluorophenyl)thiazol-2-yl(octyloxy)phenyl)amino)-3-oxopropanoate | 3.33 | 499 | **+++** |
| **45** | 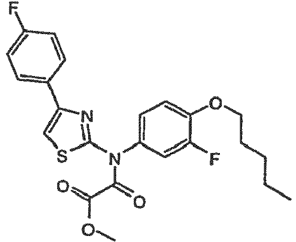 | methyl 2-((3-fluoro-4-(pentyloxy)phenyl)(4-(4-fluorophenyl)thiazol-2-yl)amino)-2-oxoacetate | 2.71 | 461 | **+++** |
| **46** | 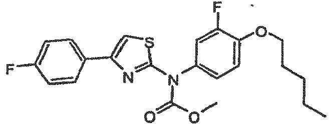 | methyl 3-fluoro-4-(pentyloxy)phenyl(4-(4-fluorophenyl)thiazol-2-yl)carbamate | 2.82 | 433 | **++** |
| **47** | 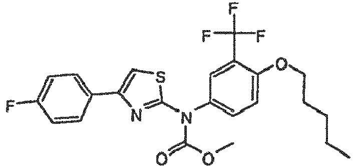 | methyl 4-(4-fluorophenyl)thiazol-2-yl(4-(pentyloxy)-3-(trifluoromethyl)phenyl)carbamate | 3.02 | 483 | **+++** |
| **48** | 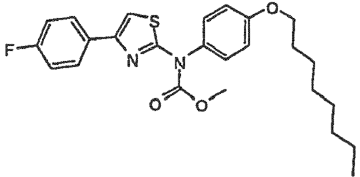 | methyl 4-(4-fluorophenyl)thiazol-2-yl(4-(octyloxy)phenyl)carbamate | 3.82 | 457 | **++** |
| **49** | 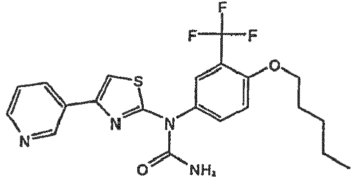 | 1-(4-(pentyloxy)-3-(trifluoromethyl)phenyl)-1-(4-(pyridin-3-yl)thiazol-2-yl)urea | 1.39 | 451 | **+++** |
| **50** | 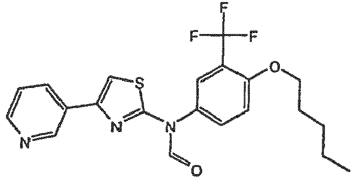 | N-(4-(pentyloxy)-3-(trifluoromethyl)phenyl)-N-(4-(pyridin-3-yl)thiazol-2-yl)formamide | 1.73 | 436 | **+++** |
| **51** | 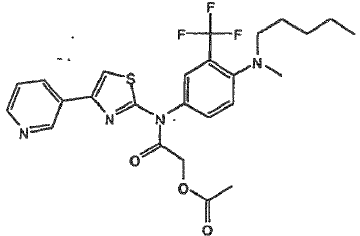 | 2-((4-(methyl(pentyl)amino)-3-(trifluoromethyl)phenyl)(4-(pyridin-3-yl)thiazol-2-yl)amino)-2-oxoethyl acetate | 1.54 | 521 | **+++** |
| **52** | 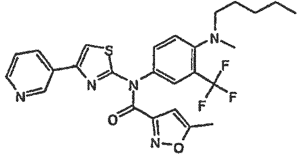 | 5-methyl-N-(4-(methyl(pentyl)amino)-3-(trifluoromethyl)phenyl)-N-(4-(pyridin-3-yl)thiazol-2-yl)isoxazole-3-carboxamide | 1.18 | 530 | **+++** |
| **53** | 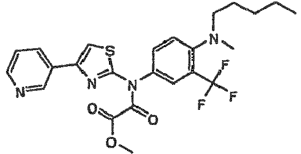 | methyl 2-((4-(methyl(pentyl)amino)-3-(trifluoromethyl)phenyl)(4-(pyridin-3-yl)thiazol-2-yl)amino)-2-oxoacetate | 1.61 | 507 | **+++** |
| **54** | 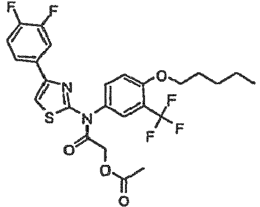 | 2-((4-(3,4-difluorophenyl)thiazol-2-yl)(4-(pentyloxy)-3-(trifluoromethyl)phenyl)amino)-2-oxoethyl acetate | 2.36 | 543.6 | **+++** |
| **55** | 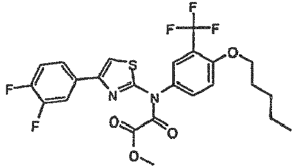 | methyl 2-((4-(methyl(pentyl)amino)-3-(trifluoromethyl)phenyl)(4-pyridin-3-yl) thiazol-2-yl)amino)-2-oxoacetate | 2.4 | 529.65 | **+++** |
| **56** | 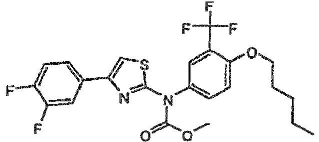 | methyl 4-(3,4-difluorophenyl)thiazol-2-yl(4-(pentyloxy)-3-(trifluoromethyl)phenyl)carbamate | 2.47 | 501 | **+++** |
| **57** | 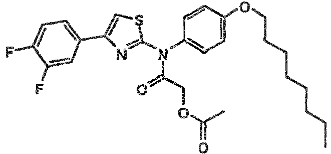 | 2-((4-(3,4-difluorophenyl)thiazol-2-yl)(4-(octyloxy)phenyl)amino)-2-oxoethyl acetate | 2.69 | 517 | **+++** |
| **58** | 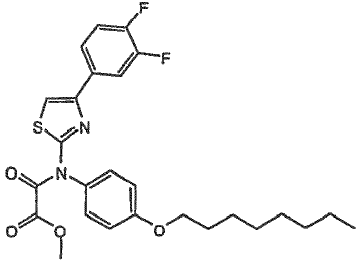 | methyl 2-((4-(3,4-difluorophenyl)thiazol-2-yl)(4-(octyloxy)phenyl)amino)-2-oxoacetate | 2.75 | 503 | **+++** |
| **59** | 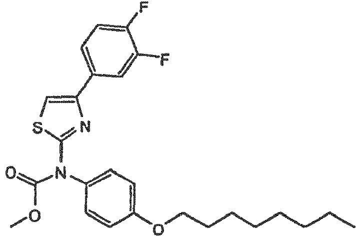 | methyl 4-(3,4-difluorophenyl)thiazol-2-yl(4-(octyloxy)phenyl)carbamate | 2.87 | 475 | **++** |
| **60** | 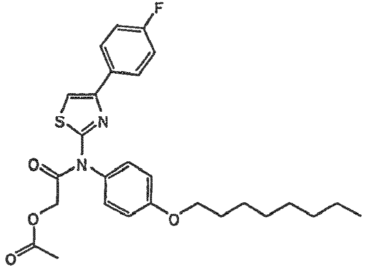 | 2-((4-(4-fluorophenyl)thiazol-2-yl)(4-(octyloxy)phenyl)amino)-2-oxoethyl acetate | 2.61 | 499 | **+++** |
| **61** | 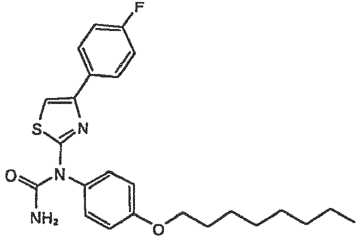 | 1-(4-(4-fluorophenyl)thiazol-2-yl)-1-(4-(octyloxy)phenyl)urea | 2.48 | 442 | **++** |
| **62** | 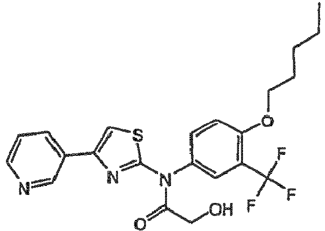 | 2-hydroxy-N-(4-(pentyloxy)-3-(trifluoromethyl)phenyl)-N-(4-(pyridin-3-yl)thiazol-2-yl)acetamide | 2.12 | 466 | **+++** |
| **63** | 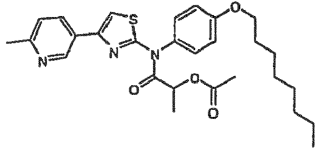 | 1-((4-(6-methylpyridin-3-yl)thiazol-2-yl)(4-(octyloxy)phenyl)amino)-1-oxopropan-2-yl acetate | 1.96 | 510 | **+++** |
| **64** | 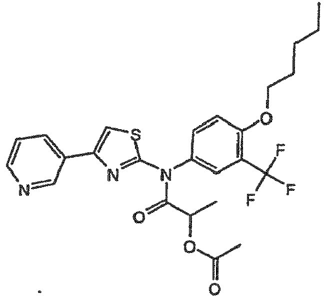 | 1-oxo-1-((4-(pentyloxy)-3-(trifluoromethyl)phenyl)(4-(pyridin-3-yl)thiazol-2-yl)amino)propan-2-yl acetate | 1.54 | 522 | **+++** |
| **65** | 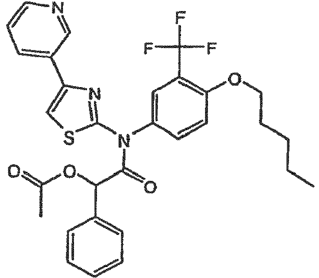 | 2-oxo-2-((4-(pentyloxy)-3-(trifluoromethyl)phenyl)(4-(pyridin-3-yl)thiazol-2-yl)amino)-1-phenylethyl acetate | 1.68 | 584 | **+++** |
| **66** | 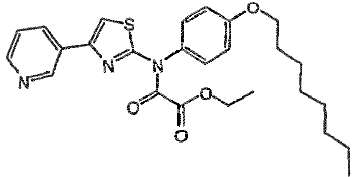 | 2-((4-(octyloxy)phenyl)(4-(pyridin-3-yl)thiazol-2-yl)amino)-2-oxoacetate | 2.09 | 482 | **+++** |
| **67** | 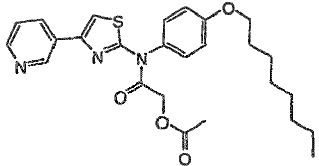 | 2-((4-(octyloxy)phenyl)(4-(pyridin-3-yl)thiazol-2-yl)amino)-2-oxoethyl acetate | 1.68 | 482 | **+++** |
| **68** | 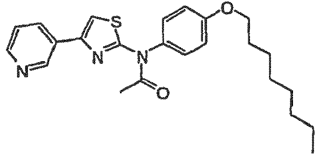 | N-(4-(octyloxy)phenyl)-N-(4-(pyridin-3-yl)thiazol-2-y!)acetamide | 1.71 | 424 | **+++** |
| **69** | 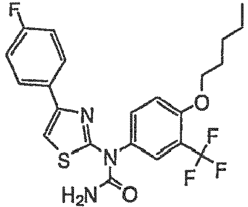 | 1-(4-(4-fluorophenyl)thiazol-2-yl)-1-(4-(pentyloxy)-3-(trifluoromethyl)phenyl)urea | 2.47 | 468 | **+++** |
| **70** | 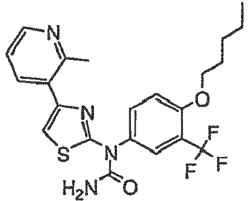 | 1-(4-(2-methylpyridin-3-yl)thiazol-2-yl)-1-(4-(pentyloxy)-3-(trifluoromethyl)phenyl)urea | 1.21 | 465 | **+++** |
| **71** | 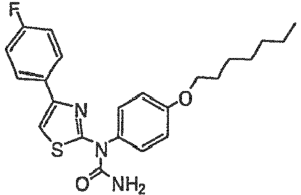 | 1-(4-(4-fluorophenyl)thiazol-2-yl)-1-(4-(heptyloxy)phenyl)urea | 2.81 | 428 | **+++** |
| **72** | 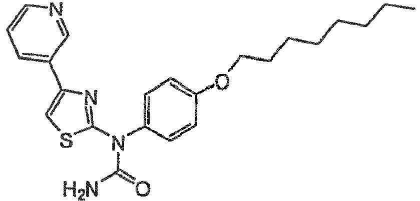 | 1-(4-(octyloxy)phenyl)-1-(4-(pyridin-3-yl)thiazol-2-yl)urea | 1.44 | 425 | **+++** |
| **73** | 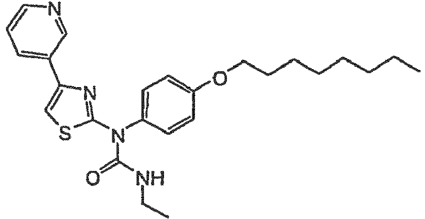 | 3-ethyl-1-(4-(octyloxy)phenyl)-1-(4-(pyridin-3-yl)thiazol-2-yl)urea | 3.37 | 453 | **+** |
| **74** | 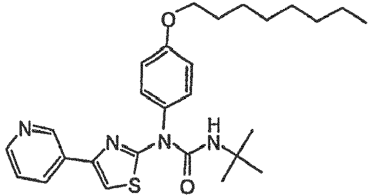 | 3-tert-butyl-1-(4-(octyloxy)phenyl)-1-(4-(pyridin-3-yl)thiazol-2-yl)urea | 2.09 | 481 | **++** |
| **75** | 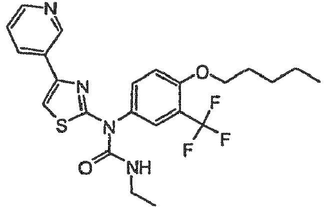 | 3-ethyl-1-(4-entyloxy)-3-(trifluoromethyl)phenyl)-1-(4-(pyridin-3-yl)thiazol-2-yl)urea | 1.46 | 479 | **+++** |
| **76** | 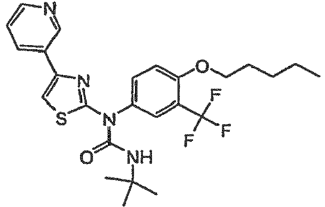 | 3-tert-butyl-1-(4-(pentyloxy)-3-(trifluoromethyl)phenyl)-1-(4-(pyridin-3-yl)thiazol-2-yl)urea | 2.76 | 507 | **+++** |
| **77** | 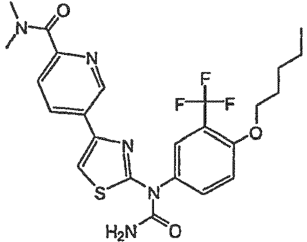 | N,N-dimethyl-5-(2-(1-(4-(pentyloxy)-3-(trifluoromethyl)phenyl)ureido)thiazol-4-yl)picolinamide | 1.74 | 522 | **+++** |
| **78** | 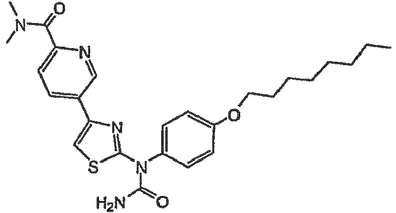 | N,N-dimethyl-5-(2-(1-(4-(octyloxy)phenyl)ureido)thiazol-4-yl)picolinamide | 2.06 | 496 | **+** |
| **79** | 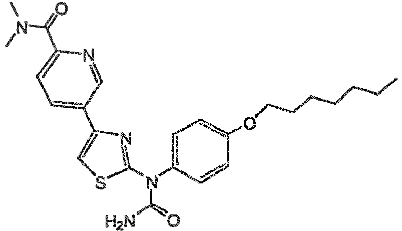 | 5-(2-(1-(4-(heptyloxy)phenyl)ureido)thiazol-4-yl)-N,N-dimethylpicolinamide | 1.89 | 482 | **+** |
| **80** | 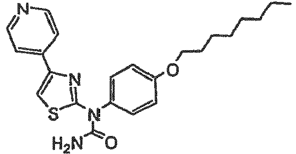 | 1-(4-(octyloxy)phenyl)-1-(4-(pyridin-4-yl)thiazol-2-yl)urea | 1.32 | 425 | **+** |
| **81** | 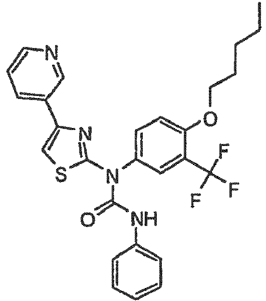 | 1-(4-(pentyloxy)-3-(trifluoromethyl)phenyl)-3-phenyl-1-(4 (pyridin-3-yl)thiazol-2-yl)urea | 2.71 | 527 | **+++** |
| **82** | 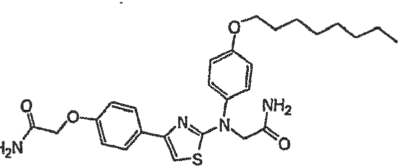 | 2-((4-(4-(2-amino-2-oxoethoxy)phenyl)thiazol-2-yl)(4-(octyloxy)phenyl)amino)acetamide | | | **+** |
| **83** | 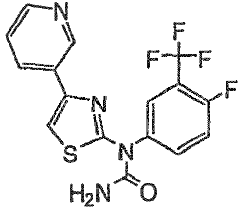 | 1-(4-fluoro-3-(trifluoromethyl)phenyl)-1-(4-(pyridin-3-yl)thiazol-2-yl)urea | 1.07 | 383 | **+++** |
| **84** | 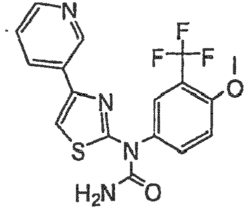 | 1-(4-methoxy-3-(trifluoromethyl)phenyl)-1-(4-pyridin-3-yl)thiazol-2-yl)urea | 0.82 | 395 | **+++** |
| **85** | 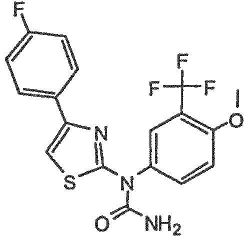 | 1-(4-(4-fluorophenyl)thiazol-2-yl)-1-(4-methoxy-3-(trifluoromethyl)phenyl)urea | 2.08 | 412 | **+++** |
| **86** | 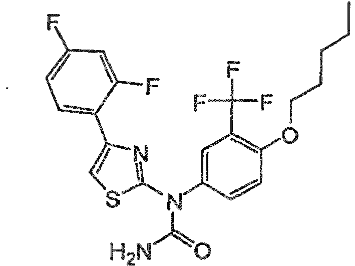 | 1-(4-(2,4-difluorophenyl)thiazol-2-yl)-1-(4-(pentyloxy)-3-(trifluoromethyl)phenyl)urea | 2.52 | 486 | +++ |
| **87** | 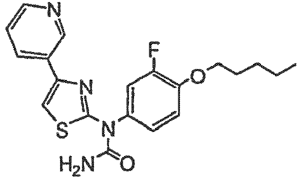 | 1-(3-fluoro-4-(pentyloxy)phenyl)-1-(4-(pyridin-3-yl)thiazol-2-yl)urea | 1.24 | 401.41 | +++ |
| **88** | 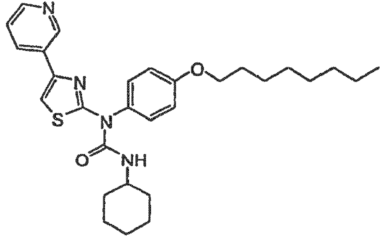 | 3-cyclohexyl-1-(4-(octyloxy)phenyl)-1-(4-(pyridin-3-yl)thiazol-2-yl)urea | 3.11 | 507 | ++ |
| **89** | 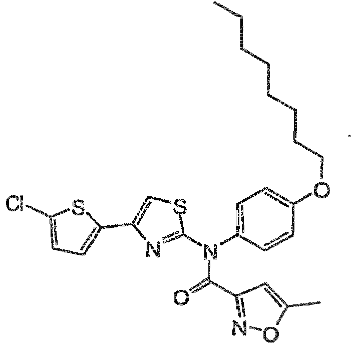 | N-(4-(5-chlorothiophen-2-yl)thiazol-2-yl)-5-methyl-N-(4-(octyloxy)phenyl)isoxazole-3-carboxamide | 4.17 | 531 | **+++** |
| **90** | 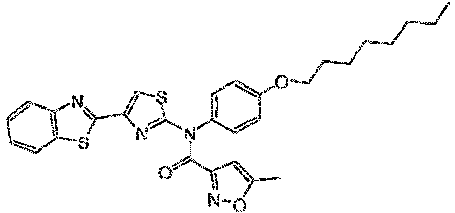 | N-(4-(5-chlorothiophen-2-yl)thiazol-2-yl)-5-methyl-N-(4-(octyloxy)phenyl)isoxazole-3-carboxamide | 3.74 | 547 | **++** |
| **91** | 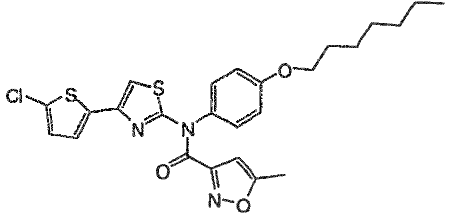 | N-(4-(5-chlorothiophen-2-yl)thiazol-2-yl)-N-(4-(heptyloxy)phenyl)-5-methylisoxazole-3-carboxamide | 3.81 | 517 | **+++** |
| **92** | 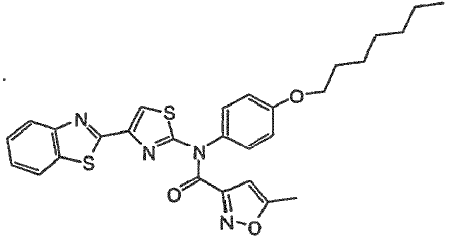 | N-(4-(benzo[d]thiazol-2-yl)thiazol-2-yl)-N-(4-(heptyloxy)phenyl)-5-methylisoxazole-3-carboxamide | 3.41 | 533 | **++** |
| **93** | 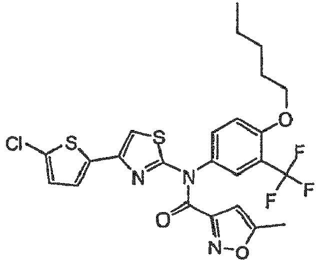 | N-(4-(benzo[d]thiazol-2-yl)thiazol-2-yl)-N-(4-(heptyloxy)phenyl)-5-methylisoxazole-3-carboxamide | 3.31 | 557 | **+++** |
| **94** | 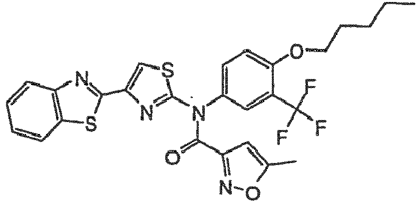 | N-(4-(benzo[d]thiazol-2-yl)thiazol-2-yl)-5-methyl-N-(4-(pentyloxy)-3-(trifluoromethyl)phenyl)isoxazole-3-carboxamide | 3.02 | 573 | **+++** |
| **95** | 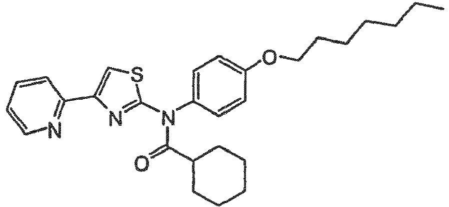 | N-(4-(heptyloxy)phenyl)-N-(4-(pyridin-2-yl)thiazol-2-yl)cyclohexanecarboxamide | 2.08 | 478 | **++** |
| **96** | 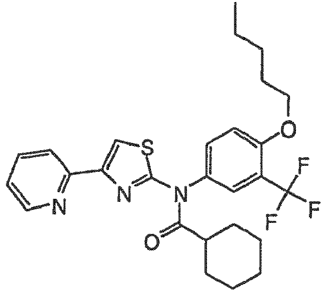 | N-(4-(pentyloxy)-3-(trifluoromethyl)phenyl)-N-(4-(pyridin-2-yl)thiazol-2-yl)cyclohexanecarboxamide | 1.94 | 518 | **+++** |
| **97** | 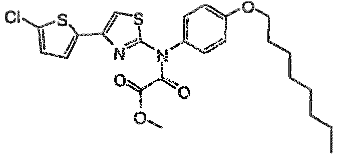 | methyl 2-((4-(5-chlorothiophen-2-yl)thiazol-2-yl)(4-(octyloxy)phenyl)amino)-2-oxoacetate | 4.17 | 508 | **+++** |
| **98** | 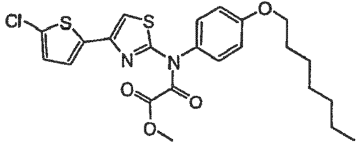 | methyl 2-((4-(5-chlorothiophen-2-yl)thiazol-2-yl)(4-(heptyloxy)phenyl)amino)-2-oxoacetate | 3.83 | 494 | **+++** |
| **99** | 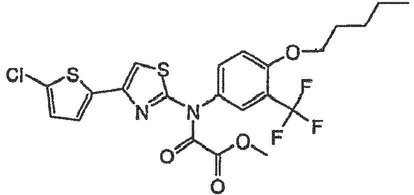 | methyl 2-((4-(5-chlorothiophen-2-yl)thiazol-2-yl)(4-(pentyloxy)-3-(trifluoromethyl)phenyl)amino)-2-oxoacetate | 3.35 | 533 | **+++** |
| **100** | 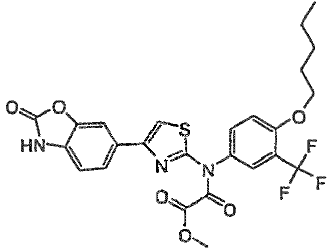 | methyl 2-oxo-2-((4-(2-oxo-2,3-dihydrobenzo[d]oxazol-6-yl)thiazol-2-yl)(4-(pentyloxy)-3-(trifluoromethyl)phenyl)amino)acetate | 2.25 | 550 | **+++** |
| **101** | 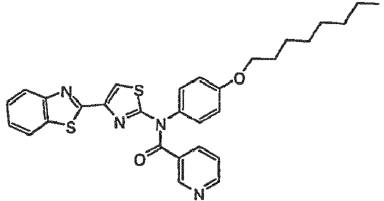 | N-(4-(benzo[d]thiazol-2-yl)thiazol-2-yl)-N-(4-(octyloxy)phenyl)nicotinamide | 2.91 | 543 | **++** |
| **102** | 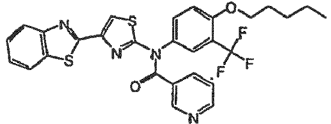 | N-(4-(benzo[d]thiazol-2-yl)-N-(4-(pentyloxy)-3-(trifluoromethyl)phenyl)nicotinamide | 2.42 | 569 | **+++** |
| **103** | 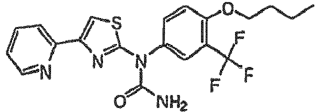 | 1-(4-butoxy-3-(trifluoromethyl)phenyl)-1-(4-(pyridin-2-yl)thiazol-2-yl)urea | 1.14 | 437 | **+++** |
| **104** | 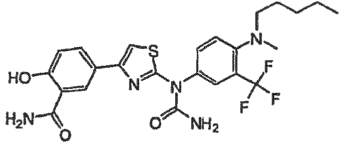 | 1-(4-butoxy-3-(trifluoromethyl)phenyl)-1-(4-(pyridin-2-yl)thiazol-2-yl)urea | 2.07 | 522 | **+++** |
| **105** | 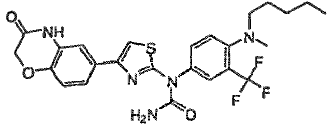 | 1-(4-(methyl(pentyl)amino)-3-(trifluoromethyl)phenyl)-1-(4-(3-oxo-3,4-dihydro-2H-benzo[b][1,4]oxazin-6-yl)thiazol-2-yl)urea | 2.09 | 534 | **++** |
| **106** | 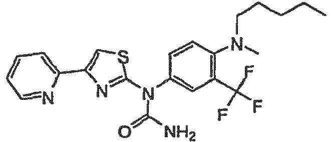 | 1-(4-(methyl(pentyl)amino)-3-(trifluoromethyl)phenyl)-1-(4-(pyridin-2-yl)thiazol-2-yl)urea | 1.34 | 464 | **+++** |
| **107** | 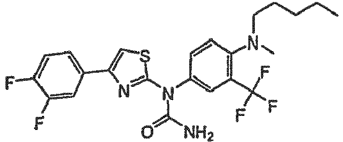 | 1-(4-(methyl)(pentyl)amino-3-(trifluoromethyl)phenyl)-1-(4-(pyridin-2-yl)thiazol-2-yl)urea | 2.7 | 499 | **+++** |
| **108** | 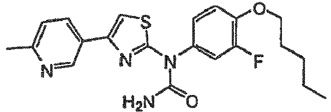 | 1-(4-(methyl(pentyl)amino)-3-(trifluoromethyl)phenyl)-1-(4-(pyridin-2-yl)thiazol-2-yl)urea | 1.04 | 415 | **+++** |
| **109** | 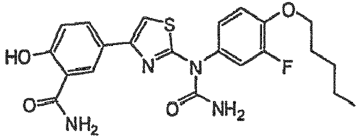 | 5-(2-(1-(3-fluoro-4-(pentyloxy)phenyl)ureido)thiazol-4-yl)-2-hydroxybenzamide | 1.78 | 459 | **++** |
| **110** | 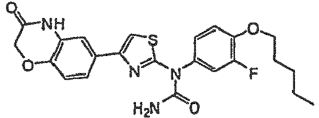 | 1-(3-fluoro-4-(pentyloxy)phenyl)-1-(4-(3-oxo-3,4-dihydro-2H-benzo[b][1,4]oxazin-6-yl)thiazol-2-yl)urea | 1.79 | 471 | **+** |
| **111** | 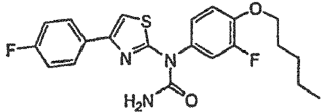 | 1-(3-fluoro-4-(pentyloxy)phenyl)-1-(4-(4-fluorophenyl)thiazol-2-yl)urea | 2.25 | 418 | **+++** |
| **112** | 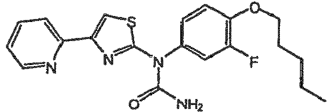 | 1-(3-fluoro-4-(pentyloxy)phenyl)-1-(4-(pyridin-2-yl)thiazol-2-yl)urea | 1.03 | 401 | **++** |
| **113** | 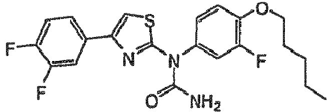 | 1-(4-(3,4-difluorophenyl)thiazol-2-yl)-1-(3-fluoro-4-(pentyloxy)phenyl)urea | 2.35 | 436 | **++** |
| **114** | 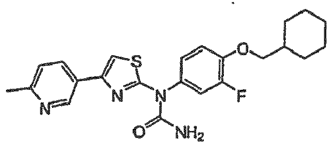 | 1-(4-(cyclohexylmethoxy)-3-fluorophenyl)-1-(4-(6-methylpyridin-3-yl)thiazol-2-yl)urea | 1.19 | 441 | **++** |
| **115** | 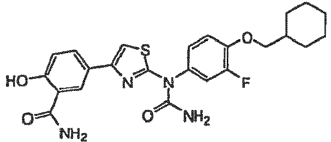 | 5-(2-(1-(4-(cyclohexylmethoxy)-3-fluorophenyl)ureido)thiazol-4-yl)-2-hydroxybenzamide | 1.97 | 485 | **++** |
| **116** | 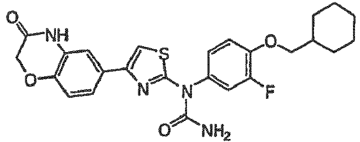 | 1-(4-(cyclohexylmethoxy)-3-fluorophenyl)-1-(4-(3-oxo-3,4-dihydro-2H-benzo[b][1,4]oxazin-6-yl)thiazol-2-yl)urea | 1.98 | 497 | **+** |
| **117** | 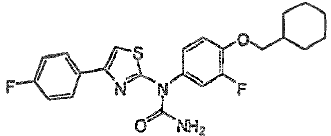 | 1-(4-(cyclohexylmethoxy)-3-fluorophenyl)-1-(4-(4-fluorophenyl)thiazol-2-yl)urea | 2.51 | 444 | **++** |
| **118** | 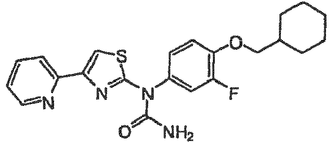 | 1-(4-(cyclohexylmethoxy)-3-fluorophenyl)-1-(4-(pyridin-2-yl)thiazol-2-yl)urea | 1.21 | 427 | **++** |
| **119** | 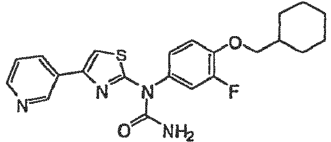 | 1-(4-(cyclohexylmethoxy)-3-fluorophenyl)-1-(4-(pyridin-3-yl)thiazol-2-yl)urea | 1.18 | 427 | **+++** |
| **120** | 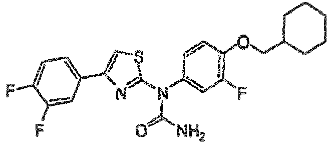 | 1-(4-(cyclohexylmethoxy)-3-fluorophenyl)-1-(4-(3,4-difluorophenyl)thiazol-2-yl)urea | 2.6 | 462 | **+++** |
| **121** | 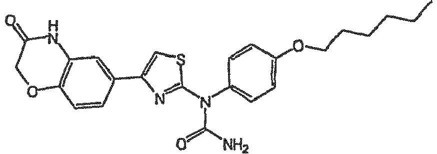 | 1-(4-(hexyloxy)phenyl)-1-(4-(3-oxo-3,4-dihydro-2H-benzo[b][1,4]oxazin-6-yl)thiazol-2-yl)urea | 2.01 | 467 | **++** |
| **122** | 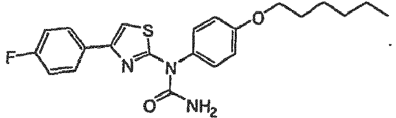 | 1-(4-(4-fluorophenyl)thiazol-2-yl)-1-(4-(hexyloxy)phenyl)urea | 2.51 | 414 | **+++** |
| **123** | 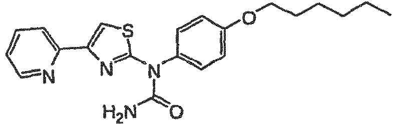 | 1-(4-(hexyloxy)phenyl)-1-(4-(pyridin-2-yl)thiazol-2-yl)urea | 1.16 | 397 | **+++** |
| **124** | 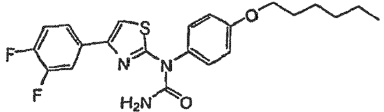 | 1-(4-(3,4-difluorophenyl)thiazol-2-yl)-1-(4-(hexyloxy)phenyl)urea | 2.59 | 432 | **+++** |
| **125** | 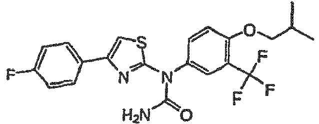 | 1-(4-(4-fluorophenyl)thiazol-2-yl)-1-(4-isobutoxy-3-(trifluoromethyl)phenyl)urea | 2.3 | 454 | **+++** |
| **126** | 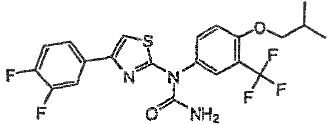 | 1-(4-(3,4-difluorophenyl)thiazol-2-yl)-1-(4-isobutoxy-3-(trifluoromethyl)phenyl)urea | 2.36 | 472 | **+++** |
| **127** | 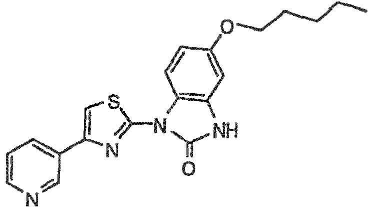 | 5-(pentyloxy)-1-(4-(pyridin-3-yl)thiazol-2-yl)-1H-benzo[d]imidazol-2(3H)-one | 1.32 | 381 | **++** |
| **128** | 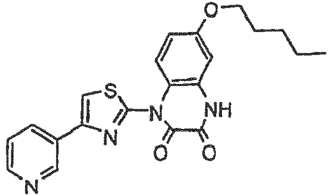 | 6-(pentyloxy)-1-(4-(pyridin-3-yl)thiazol-2-yl)quinoxaline-2,3(1H,4H)-dione | 1.15 | 409 | **+** |
| **129** | 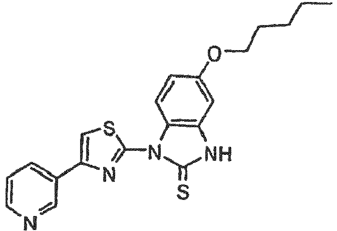 | 5-(pentyloxy)-1-(4-(pyridin-3-yl)thiazol-2-yl)-1H-benzo[d]imidazole-2(3H)-thione | 1.5 | 438.61 | **+** |
| **130** | 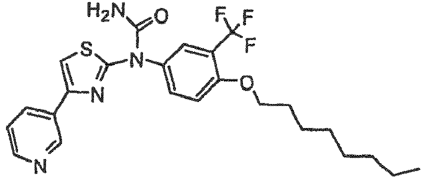 | 1-(4-(octyloxy)-3-(trifluoromethyl)phenyl)-1-(4-(pyridin-3-yl)thiazol-2-yl)urea | 1.76 | M492 | **+++** |
| **131** | 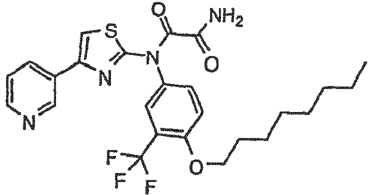 | N1-(4-(octyloxy)-3-(trifluoromethyl)phenyl)-N1-(4-(pyridin-3-yl)thiazol-2-yl)oxalamide | 1.62 | 521 | **+++** |
| **132** | 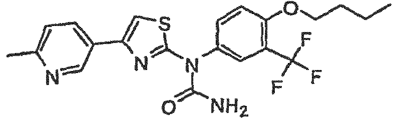 | 1-(4-butoxy-3-(trifluoromethyl)phenyl)-1-(4-(6-methylpyridin-3-yl)thiazol-2-yl)urea | 1.24 | 451 | **+++** |
| **133** | 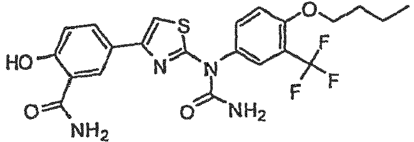 | 5-(2-(1-(4-butoxy-3-(trifluoromethyl)phenyl)ureido)thiazol-4-yl)-2-hydroxybenzamide | 1.85 | 495 | **+++** |
| **134** | 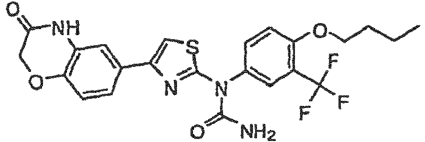 | 1-(4-butoxy-3-(trifluoromethyl)phenyl)-1-(4-(3-oxo-3,4-dihydro-2H-benzo[b][1,4]oxazin-6-yl)thiazol-2-yl)urea | 1.86 | 507 | **++** |
| **135** | 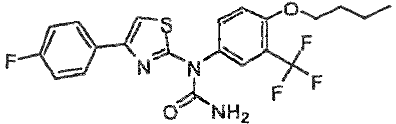 | 1-(4-butoxy-3-(trifluoromethyl)phenyl)-1-(4-(4-fluorophenyl)thiazol-2-yl)urea | 2.29 | 454 | **+++** |
| **136** | 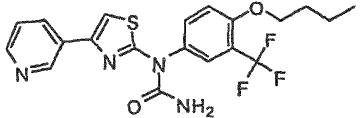 | 1-(4-butoxy-3-(trifluoromethyl)phenyl)-1-(4-(pyridin-3-yl)thiazol-2-yl)urea | 1.13 | 437 | **+++** |
| **137** | 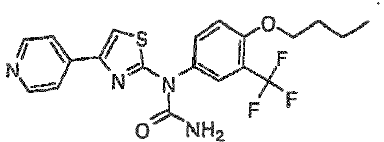 | 1-(4-butoxy-3-(trifluoromethyl)phenyl)-1-(4-(pyridin-4-yl)thiazol-2-yl)urea | 1.1 | 437 | **+++** |
| **138** | 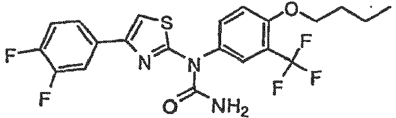 | 1-(4-butoxy-3-(trifluoromethyl)phenyl)-1-(4-(3,4-difluorophenyl)thiazol-2-yl)urea | 2.34 | 472 | **+++** |
| **139** | 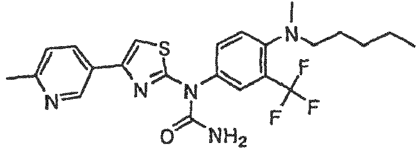 | 1-(4-(methyl(pentyl)amino)-3-(trifluoromethyl)phenyl)-1-(4-(6-methylpyridin-3-yl)thiazol-2-yl)urea | 1.34 | 478 | **+++** |
| **140** | 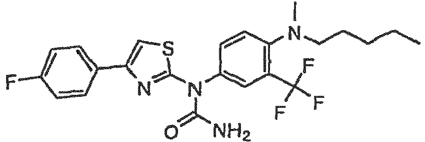 | 1-(4-(4-fluorophenyl)thiazol-2-yl)-1-(4-(methyl(pentyl)amino)-3-(trifluoromethyl)phenyl)urea | 2.64 | 481 | **+++** |
| **141** | 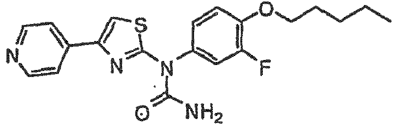 | 1-(3-fluoro-4-(pentyloxy)phenyl)-1-(4-(pyridin-4-yl)thiazol-2-yl)urea | 1.02 | 401 | **+** |
| **142** | 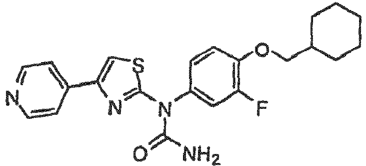 | 1-(4-(cyclohexylmethoxy)-3-fluorophenyl)-1-(4-(pyridin-4-yl)thiazol-2-yl)urea | 1.17 | 427 | **++** |
| **143** | 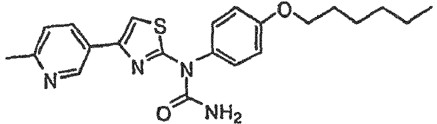 | 1-(4-(hexyloxy)phenyl)-1-(4-(6-methylpyridin-3-yl)thiazol-2-yl)urea | 1.22 | 411 | **++** |
| **144** | 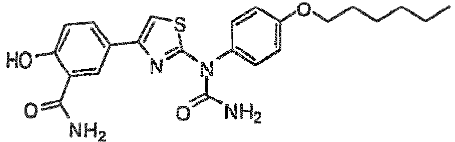 | 5-(2-(1-(4-(hexyloxy)phenyl)ureido)thiazol-4-yl)-2-hydroxybenzamide | 1.96 | 455 | **++** |
| **145** | 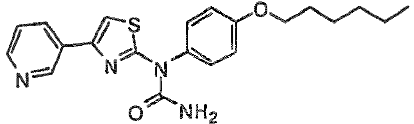 | 1-(4-(hexyloxy)phenyl)-1-(4-(pyridin-3-yl)thiazol-2-yl)urea | 1.2 | 397 | **+++** |
| **146** | 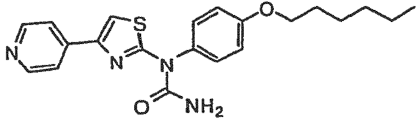 | 1-(4-(hexyloxy)phenyl)-1-(4-(pyridin-4-yl)thiazol-2-yl)urea | 1.17 | 397 | **++** |
| **147** | 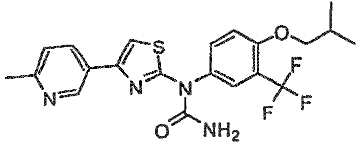 | 1-(4-isobutoxy-3-(trifluoromethyl)phenyl)-1-(4-(6-methylpyridin-3-yl)thiazol-2-yl)urea | 1.15 | 451 | **+++** |
| **148** | 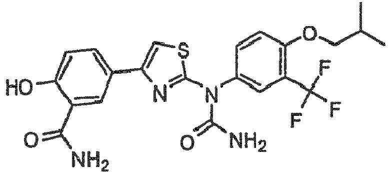 | 2-hydroxy-5-(2-(1-(4-isobutoxy-3-(trifluoromethyl)phenyl)ureido)thiazol-4-yl)benzamide | 1.83 | 495 | **+++** |
| **149** | 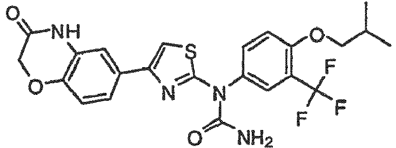 | 1-(4-isobutoxy-3-(trifluoromethyl)phenyl)-1-(4-(3-oxo-3,4-dihydro-2H-benzo[b][1,4]oxazin-6-yl)thiazol-2-yl)urea | 1.85 | 507 | **+++** |
| **150** | 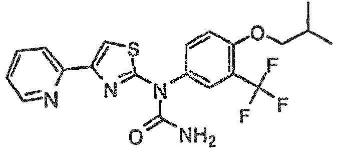 | 1-(4-isobutoxy-3-(trifluoromethyl)phenyl)-1-(4-(pyridin-2-yl)thiazol-2-yl)urea | 1.16 | 437 | **+++** |
| **151** | 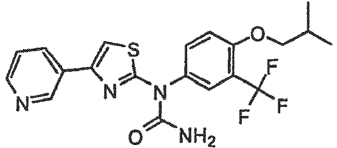 | 1-(4-isobutoxy-3-(trifluoromethyl)phenyl)-1-(4-(pyridin-3-yl)thiazol-2-yl)urea | 1.12 | 437 | **+++** |
| **152** | 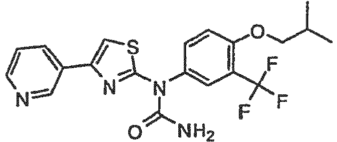 | 1-(4-isobutoxy-3-(trifluoromethyl)phenyl)-1-(4-(pyridin-3-yl)thiazol-2-yl)urea | 1.08 | 437 | **++** |
| **153** | 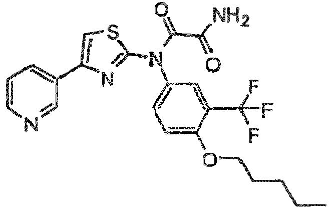 | N1-(4-(pentyloxy)-3-(trifluoromethyl)phenyl)-N1-(4-(pyridin-3-yl)thiazol-2-yl)oxalamide | 1.24 | 479 | **+++** |
| **154** | 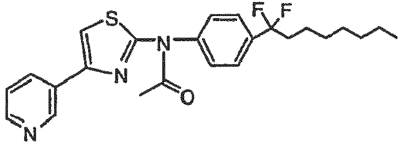 | N-(4-(1,1-difluorooctyl)phenyl)-N-(4-(pyridin-3-yl)thiazol-2-yl)acetamide | 1.73 | 445 | **+++** |
| **155** | 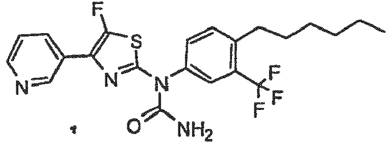 | 1-(5-fluoro-4-(pyridin-3-yl)thiazol-2-yl)-1-(4-hexyl-3-(trifluoromethyl)phenyl)urea | 1.58 | 467 | **+++** |
| **156** | 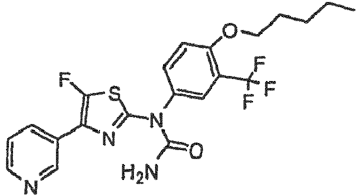 | 1-(5-fluoro-4-(pyridin-3-yl)thiazol-2-yl)-1-(4-(pentyloxy)-3-(trifluoromethyl)phenyl)urea | 1.45 | 469 | **+++** |
| **157** | 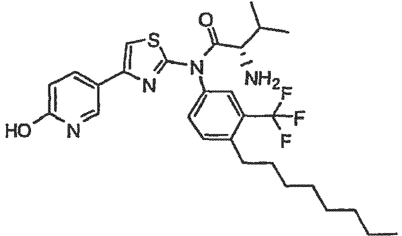 | (S)-2-amino-N-(4-(6-hydroxypyridin-3-yl)thiazol-2-yl)-3-methyl-N-(4-octyl-3-(trifluoromethyl)phenyl)butanamide | 1.57 | 549 | **++** |
| **158** | 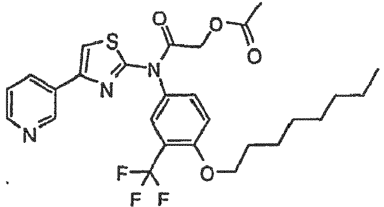 | 2-((4-(octyloxy)-3-(tiifluoromethyl)phenyl)(4-(pyridin-3-yl)thiazol-2-yl)amino)-2-oxoethyl acetate | 1.81 | 550 | **+++** |
| **159** | 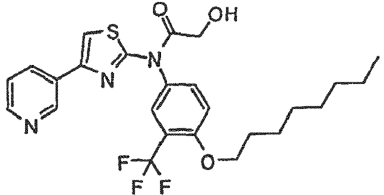 | 2-hydroxy-N-(4-(octyloxy)-3-(trifluoromethyl)phenyl)-N-(4-(pyridin-3-yl)thiazol-2-yl)acetamide | 1.69 | 508 | **+++** |
| **160** | 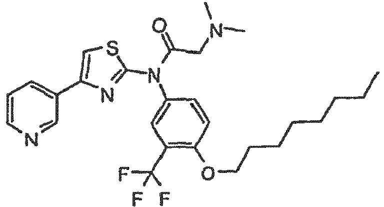 | 2-(dimethylamino)-N-(4-(octyloxy)-3-(trifluoromethyl)phenyl)-N-(4-(pyridin-3-yl)thiazol-2-yl)acetamide | 1.31 | 535 | **+++** |
| **161** | 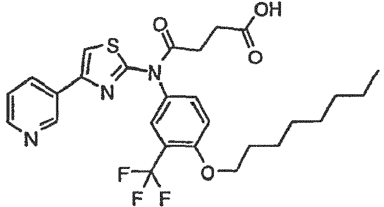 | 4-((4-(octyloxy)-3-(trifluoromethyl)phenyl)(4-(pyridin-3-yl)thiazol-2-yl)amino)-4-oxobutanoic acid | 1.61 | 550 | **+++** |
| **162** | 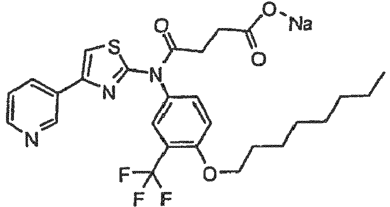 | sodium 4-((4-(octyloxy)-3-(trifluoromethyl)phenyl)(4-(pyridin-3-yl)thiazol-2-yl)amino)-4-oxobutanoate | 1.6 | 550 | **+++** |
| **163** | 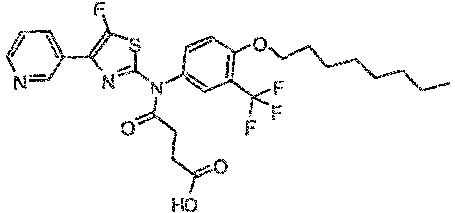 | 4-((5-fluoro-4-(pyridin-3-yl)thiazol-2-yl)(4-(octyloxy)-3-(trifluoromethyl)phenyl)amino)4-oxobutanoic acid | 1.68 | 568 | **+++** |
| **164** | 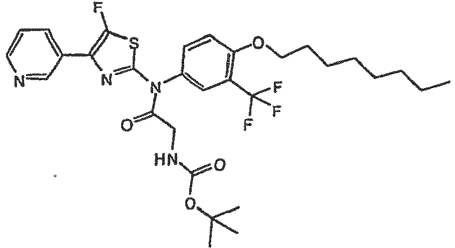 | tert-butyl 2-((5-fluoro-4-(pyridin-3-yl)thiazol-2-yl)(4-(octyloxy)-3-(trifluoromethyl)phenyl)amino)-2-oxoethylcarbamate | | | **+++** |
| **165** | 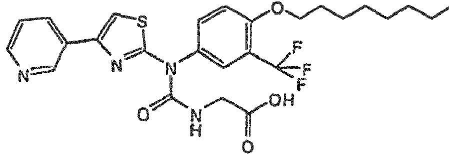 | 2-(3-(4-(octyloxy)-3-(trifluoromethyl)phenyl)-3-(4-(pyridin-3-yl)thiazol-2-yl)ureido)acetic acid | 1.59 | 551 | **+++** |
| **166** | 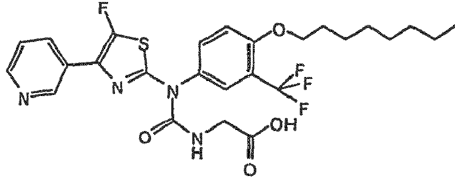 | 2-(3-(5-fluoro-4-(pyridin-3-yl)thiazol-2-yl)-3-(4-(octyloxy)-3-acid | 1.63 | 569 | **+++** |
| **167** | 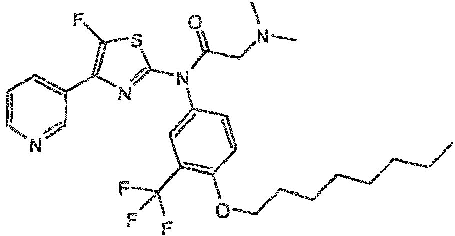 | 2-(dimethylamino)-N-(5-fluoro-4-(pyridin-3-yl)thiazol-2-yl)-N-(4-(octyloxy)-3-(trifluoromethyl)phenyl)acetamide | 1.22 | 553 | **+++** |
| **168** | 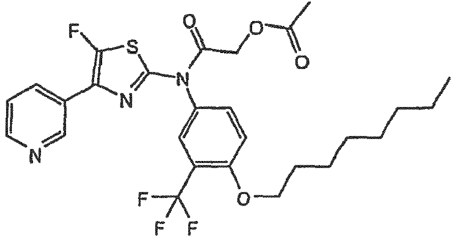 | 2-((5-fluoro-4-(pyridin-3-yl)thiazol-2-yl)(4-(octyloxy)-3-(trifluoromethyl)phenyl)amino)-2-oxoethyl acetate | 1.9 | 568 | **+++** |
| **169** | 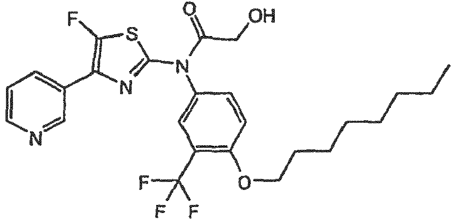 | N-(5-fluoro-4-(pyridin-3-yl)thiazol-2-yl)-2-hydroxy-N-(4-(octyloxy)-3-(trifluoromethyl)phenyl)acetamide | 1.58 | 508 | **+++** |
| **170** | 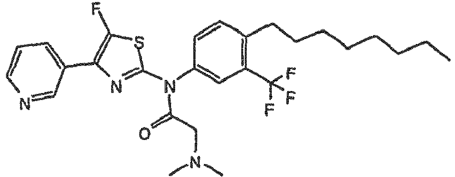 | 2-(dimethylamino)-N-(5-fluoro-4-(pyridin-3-yl)thiazol-2-yl)-N-(4-octyl-3-(trifluoromethyl)phenyl)acetamide | 1.29 | 537 | **+++** |
| **171** | 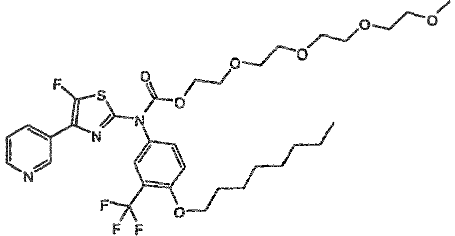 | 2,5,8,11-tetraoxatridecan-13-yl 5-fluoro-4-(pyridin-3-yl)thiazol-2-yl(4-(octyloxy)-3-(trifluoromethyl)phenyl)carbamate | 1.92 | 702 | **+++** |
| **172** | 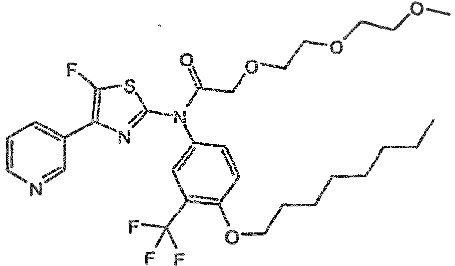 | N-(5-fluoro-4-(pyridin-3-yl)thiazol-2-yl)-2-(2-(2-methoxyethoxy)ethoxy)-N-(4-(octyloxy)-3-(trifluoromethyl)phenyl)acetamide | 1.8 | 628 | **+++** |
| **173** | 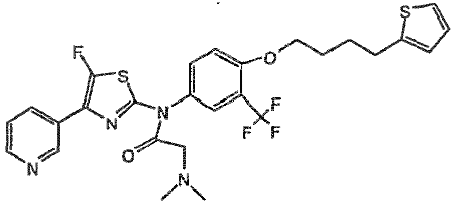 | 2-(dimethylamino)-N-(5-fluoro-4-(pyridin-3-yl)thiazol-2-yl)-N-(4-(4-(thiophen-2-yl)butoxy)-3-(trifluoromethyl)phenyl)acetamide | 1.1 | 579 | **+++** |
| **174** | 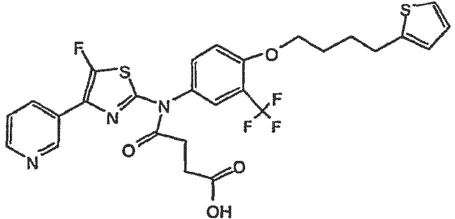 | 4-((5-fluoro-4-(pyridin-3-yl)thiazol-2-yl)(4-(4-(thiophen-2-yl)butoxy)-3-(trifluoromethyl)phenyl)amino)-4-oxobutanoic acid | 1.6 | 594 | **+++** |
| **175** | 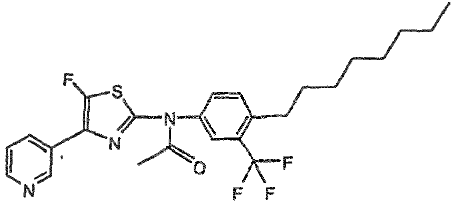 | N-(5-fluoro-4-(pyridin-3-yl)thiazol-2-yl)-N-(4-octyl-3-(trifluoromethyl)phenyl)acetamide | 4.04 | 495 | **+++** |
| **176** | 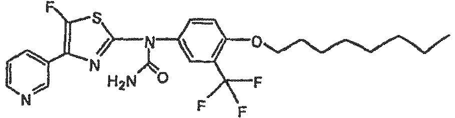 | 1-(5-fluoro-4-(pyridin-3-yl)thiazol-2-yl)-1-(4-(octyloxy)-3-(trifluoromethyl)phenyl)urea | 2.01 | 512 | **+++** |
| **177** | 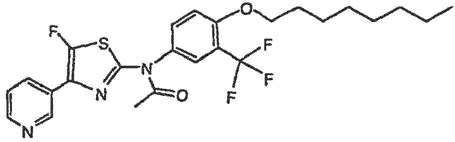 | N-(5-fluoro-4-(pyridin-3-yl)thiazol-2-yl)-N-(4-(octyloxy)-3-(trifluoromethyl)phenyl)acetamide | 2.57 | 5150 | **+++** |
| **178** | 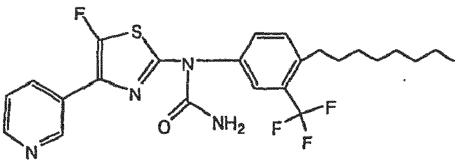 | 1-(5-fluoro-4-(pyridin-3-yl)thiazol-2-yl)-1-(4-octyl-3-(trifluoromethyl)phenyl)urea | | | **+++** |
| **179** | 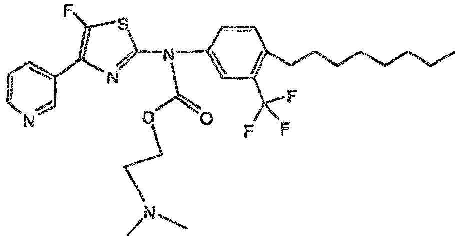 | 2-(dimethylamino)ethyl 5-fluoro-4-(pyridin-3-yl)thiazol-2-yl(4-octyl-3-(trifluoromethyl)phenyl)carbamate | 1.24 | 567 | **+++** |
| **180** | 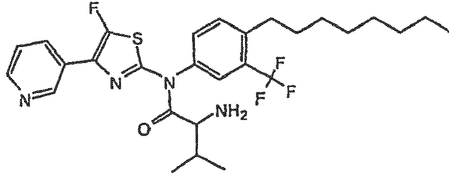 | 2-amino-N-(5-fluoro4-(pyridin-3-yl)thiazol-2-yl)-3-methyl-N-(4-octyl-3-(trifluoromethyl)phenyl)butanamide | 1.52 | 551 | **+++** |
| **181** | 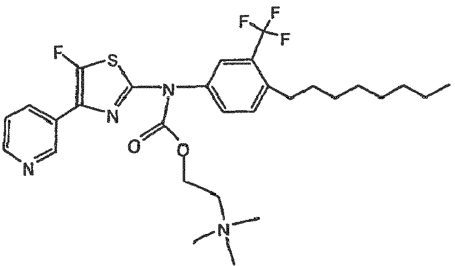 | | 1.39 | | **+++** |
| **182** | 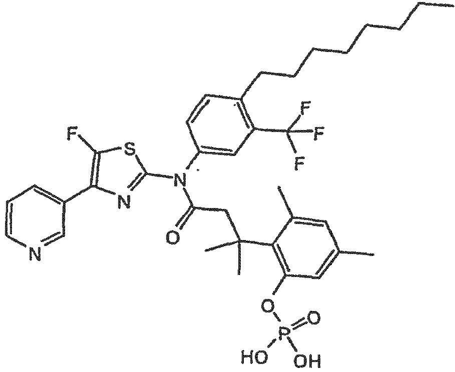 | 2-(4-((5-fluoro-4-(pyridin-3-yl)thiazol-2-yl)(4-octyl-3-(trifluoromethyl)phenyl)amino)-2-methyl-4-oxobutan-2-yl)-3,5-dimethylphenyl dihydrogen phosphate | 2.02 | 736 | **++** |
| **183** | 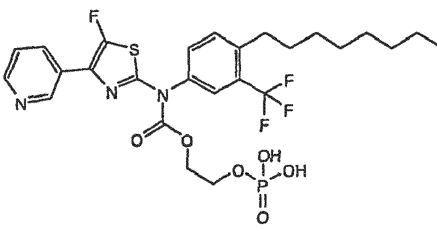 | 2-(phosphonooxy)ethyl 5-fluoro-4-(pyridin-3-yl)thiazol-2-yl(4-octyl-3-(trifluoromethyl)phenyl)carbamate | 1.54 | 621 | **+++** |
| **184** | 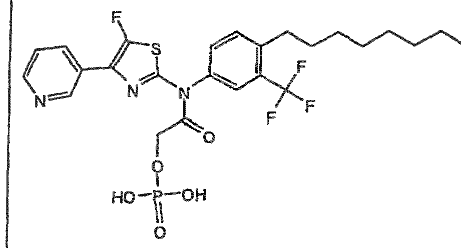 | 2-((5-fluoro-4-(pyridin-3-yl)thiazol-2-yl)(4-octyl-3-(trifluoromethyl)phenyl)amino)-2-oxoethyl dihydrogen phosphate | 1.32 | 590 | |
| **185** | 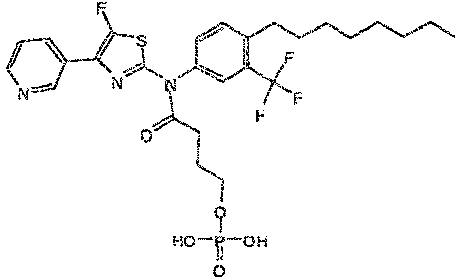 | 4-((5-fluoro-4-(pyridin-3-yl)thiazol-2-yl)(4-octyl-3-(trifluoromethyl)phenyl)amino)-4-oxobutyl dihydrogen phosphate | 1.47 | 618 | |
| **186** | 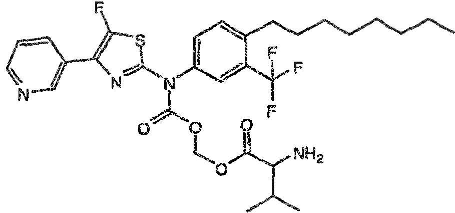 | ((5-fluoro-4-(pyridin-3-yl)thiazol-2-yl)(4-octyl-3-(trifluoromethyl)phenyl)carbamoyloxy) methyl 2-amino-3-methylbutanoate | 1.48 | 625 | **+++** |
| **187** | 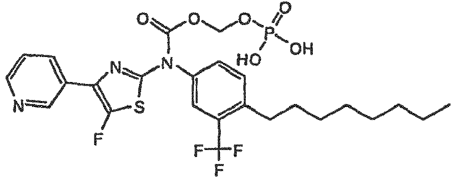 | phosphonooxymethyl 5-fluoro-4-(pyridin-3-yl)thiazol-2-yl(4-octyl-3-(trifluoromethyl)phenyl)carbamate | 1.79 | 606 | **+++** |
| **188** | 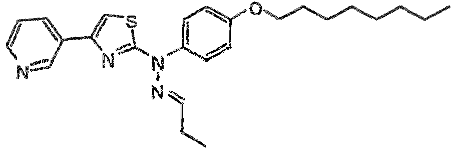 | (E)-2-(1-(4-(octyloxy)phenyl)-2-propylidenehydrazinyl)-4-(pyridin-3-yl)thiazolo | | | **+++** |
| **189** | 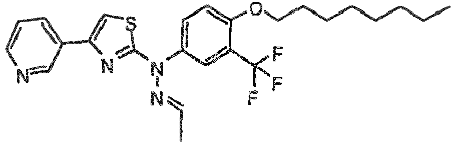 | (E)-2-(2-ethylidene-1-(4-(octyloxy)-3-(trifluoromethyl)phenyl)hydrazinyl)-4-(pyridin-3-yl)thiazolo | | | **+++** |
| **190** | 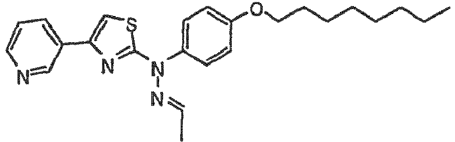 | (E)-2-(2-ethylidene-1-(4-(octyloxy)phenyl)hydrazinyl)-4-(pyridin-3-yl)thiazolo | | | **+++** |
| **191** | 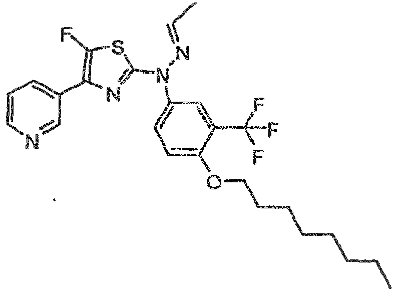 | (E)-2-(2-ethylidene-1-(4-(octyloxy)-3-(trifluoromethyl)phenyl)hydrazinyl)-5-fluoro-4-(pyridin-3-yl)thiazolo | | | **++** |
| **192** | 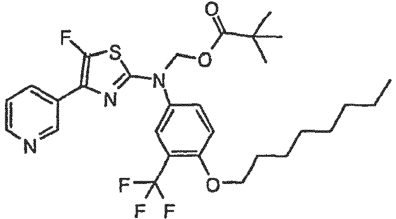 | ((5-fluoro-4-(pyridin-3-yl)thiazol-2-yl)(4-(octyloxy)-3-(trifluoromethyl)phenyl)amino)methyl pivalate | | | **+++** |
| **193** | 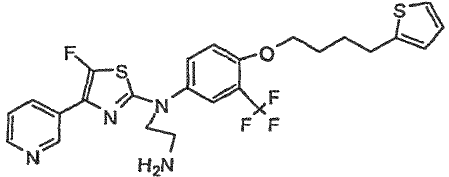 | Nl-(5-fluoro-4-(pyridin-3-yl)thiazol-2-yl)-N1-(4-(4-(thiophen-2-yl)butoxy)-3-(trifluoromethyl)phenyl)ethane-1,2-diamine | | | **++** |
| **194** | 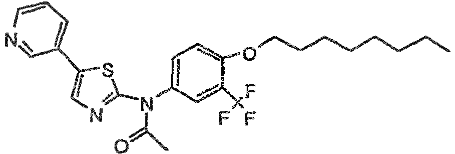 | N-(4-(octyloxy)-3-(trifluoromethyl)phenyl)-N-(5-(pyridin-3-yl)thiazol-2-yl)acetamide | | | **+++** |

| | | | | | |
|---|---|---|---|---|---|
| Scale: < 1 µM = +++; 1-10 µM = ++; > 10 µM = + | | | | | |

### CLAUSES

1. A compound or pharmaceutically acceptable salt of Formula (I) 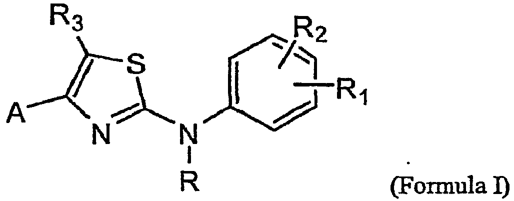 in which A is: 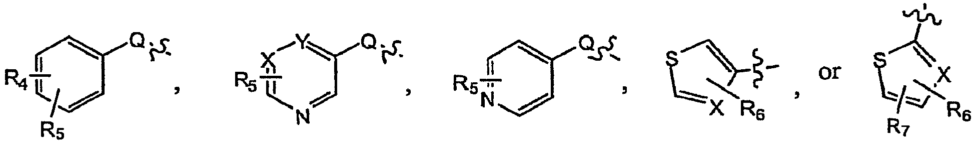 where
   Q is -NHC(O)-, -C(O)-, O, S, NH, CH₂, or absent;
   X and Y are independently N or CH;
   R is -C(O)Rₐ, -C(O)CH₂Rₐ, -N=CHR_{b}, mono- or di-(C₁-C₄alkylamino)C₀-C₄alkyl, or substituted C₁-C₄ alkyl;
   R₁ is C₁-C₂haloalkyl, C₁-C₂haloalkoxy, or R₁ is C₄-C₁₀alkoxy, C₄-C₁₀alkyl, C₄-C₁₀alkylamino, di-(C₁-C₆)(C₄-C₁₀alkylamino, (C₃-C₇cycloalkyl)C₀-C₄alkyl, (C₃-C₇cycloalkyl)C₀-C₄alkoxy, (5- or 6-membered heterocycloalkyl)C₀-C₂alkyl, (5- or 6-membered heterocycloalkyl)C₀-C₂alkoxy, (5- or 6-membered heteroaryl)C₀-C₄alkyl, (5- or 6-membered heteroaryl)C₀-C₄alkoxy, (aryl)C₀-C₂alkyl, or (aryl)C₀-C₂alkoxy, each of which is optionally substituted;
   R₂ is 0, 1, or 2 substituents independently chosen from halogen, hydroxyl, amino, cyano, C₁-C₂alkyl, C₁-C₂alkoxy, C₁-C₂haloalkyl, and C₁-C₂haloalkoxy;
   Rₐ is hydrogen, R_{c}, -C(O)OH, -C(O)NH₂, or Rₐ is C₁-C₆alkoxy, (C₁-C₆alkylester)C₀-C₄alkyl, (C₁-C₆alkylester)C₁-C₄alkoxy, (mono- or di-C₁-C₆alkylcarbamate)C₀-C₄alkyl, (C₃-C₇cycloalkyl)C₀-C₄alkyl, (5-10-membered heterocycle)C₀-C₄alkyl, C₆-C₁₂polyethylene oxide, or C₁-C₁₀ alkyl, each of which is optionally substituted;
   R_{b} is hydrogen or optionally substituted C₁-C₆alkyl;
   R_{c} is optionally substituted amino;
   R₃ is hydrogen, halogen, hydroxyl, amino, cyano, C₁-C₂alkyl, or (mono- or di-C₁-C₂alkylamino)C₀-C₂alkyl;
   R₄ is
   (a) halogen, hydroxyl, amino, cyano, -C(O)OH, -C(O)NH₂, -PO₄, C₁-C₂haloalkyl, or C₁-C₂haloalkoxy, or
   (b) mono- or di- C₁-C₄alkylamino, mono- or di-(C₁-C₄)alkylcarboxamide, C₂-C₄alkanoyl, C₁-C₄aminoalkyl, C₁-C₄aminoalkoxy, C₁-C₄hydroxyalkyl, or C₁-C₄alkylester, each of which is substituted with 0 or 1 or more substituents independently chosen from halogen, hydroxyl, amino, cyano, oxo, C₁-C₂alkyl, C₁-C₂alkoxy, mono- and di- C₁-C₂alkylamino;
   R₅ is 0 or 1 or more substituents independently chosen from
   (i) hydroxyl, halogen, amino, cyano, nitro, -COOH, -CONH₂, -PO₄, C₁-C₂haloalkyl, and C₁-C₂haloalkoxy; and
   (ii) C₁-C₄alkyl, C₁-C₄alkoxy, mono- and di- C₁-C₄alkylamino, C₂-C₄alkanoyl, mono- and di-C₁-C₄alkylphosphate, C₁-C₄alkylester, (C₃-C₇cycloalkyl)C₀-C₂alkyl, or (5- or 6-membered heterocycloalkyl)C₀-C₂alkyl, each of which is substituted with 0 or 1 or more substituents independently chosen from halogen, hydroxyl, amino, cyano, oxo, C₁-C₂alkoxy, mono- and di- C₁-C₂alkylamino; or
   any two R₄ and R₅ bound to adjacent carbon atoms may be joined to form a 5- or 6-membered ring having 0, 1, or 2 ring heteroatoms chosen from N, O, and S; which 5-or 6-membered ring is optionally substituted with 1 or 2 substituents independently chosen from halogen, hydroxyl, oxo, C₁-C₂alkyl, and C₁-C₂alkoxy;
   R₆ is 0 or 1 or more substituents independently chosen from
   (iii) hydroxyl, halogen, amino, cyano, -COOH, -CONH₂, -PO₄, C₁-C₂haloalkyl, and C₁-C₂haloalkoxy;
   (iv) C₁-C₄alkyl, C₁-C₄alkoxy, mono- and di- C₁-C₄alkylamino, C₂-C₄alkanoyl, C₁-C₄alkylphosphate, and C₁-C₄alkylester, each of which is substituted with 0 or 1 or more substituents independently chosen from halogen, hydroxyl, amino, cyano, C₁-C₂alkoxy, mono- and di- C₁-C₂alkylamino; or
   R₆ is joined with R₇ to form a 6-membered aryl or heteroaryl ring, which is substituted with 0 or 1 or more substituents independently chosen from halogen, hydroxyl, amino, cyano, C₁-C₂alkyl, C₁-C₂alkoxy, mono- and di- C₁-C₂alkylamino, C₁-C₂haloalkyl, and C₁-C₂haloalkoxy.
2. A compound or salt of Clause 1, wherein
   R is -C(O)Rₐ, -C(O)CH₂Rₐ, -N=CHR_{b}, or
   R is C₁-C₄alkyl substituted with 1 or 2 substituents independently chosen from hydroxyl, amino, -C(O)OH, -C(O)NH₂, -C(O)ONa, or C₁-C₄alkylester; where
   Rₐ is hydrogen, R_{c}, -C(O)OH, -C(O)NH₂, C₁-C₄alkyl, C₁-C₄alkoxy, (C₁-C₄alkylester)C₀-C₂alkyl, (mono- or di-C₁-C₆alkylcarbamate)C₀-C₂alkyl, C₆-C₁₂polyethylene oxide, C₃-C₇cycloalkyl, (phenyl)C₀-C₄alkyl, 5-or 6-membered heteroaryl containing 1 or 2 heteroatoms independently chosen from N, S, and O, each or which is substituted with 0, or 1 or more substituents independently chosen from halogen, hydroxyl, amino, phosphate, C₁-C₂alkyl, C₁-C₂alkoxy, mono- and di-(C₁-C₂alkyl)amino, trifluoromethyl, trifluoromethoxy, and phenyl;
   R_{b} is C₁-C₄alkyl; and
   R_{c} is amino, mono- or di(C₁-C₄alkyl amino, mono- or di(C₁-C₄alkyl amino substituted with -C(O)OH, C₃-C₇cycloalkylamino, or phenylamino.
3. A compound or salt of Clause 1, wherein
   R is -C(O)Rₐ, -C(O)CH₂Rₐ, -N=CHR_{b};
   Rₐ is hydrogen, R_{c}, -C(O)OH, -C(O)NH₂, C₁-C₄alkyl, C₁-C₄alkoxy, (C₁-C₄alkylester)C₀-C₂alkyl, (mono- or di-C₁-C₆alkylcarbamate)C₀-C₂alkyl, C₆-C₁₂polyethylene oxide, or C₃-C₆cycloalkyl, or
   Rₐ is phenyl, isoxazolyl, thienyl, imidazolyl, or pyridyl, each of which is substituted with 0, 1, or 2 substituents independently chosen from halogen, hydroxyl, C₁-C₂alkyl, and C₁-C₂alkoxy;
   R_{b} is C₁-C₄alkyl; and
   R_{c} is amino, mono- or di(C₁-C₄alkyl amino, mono- or di(C₁-C₄alkyl)amino substituted with -C(O)OH, C₃-C₇cycloalkylamino, or phenylamino.
4. A compound or salt of Clause 1, wherein
   R is -C(O)Rₐ or -C(O)CH₂Rₐ, where
   Rₐ is hydrogen, C₁-C₄alkyl, C₁-C₄alkoxy, (C₁-C₄alkylester)C₀-C₂alkyl, or C₃-C₆cycloalkyl, or R is -C(O)NH₂.
5. A compound or salt of Clause 1, wherein
   R is -C(O)Rₐ, -C(O)CH₂Rₐ, or -N=CHR_{b},
   Rₐ is hydrogen, R_{c}, -C(O)OH, -C(O)NH₂, C₁-C₄alkyl, C₁-C₄alkoxy, (C₁-C₄alkylester)C₀-C₂alkyl, (mono- or di-C₁-C₆alkylcarbamate)C₀-C₂alkyl, C₆-C₁₂polyethylene oxide, C₃-C₆cyloalkyl, or
   Rₐ is phenyl, isoxazolyl, thienyl, imidazolyl, or pyridyl, each of which is substituted with 0, 1, or 2 substituents independently chosen from halogen, hydroxyl, C₁-C₂alkyl, and C₁-C₂alkoxy;
   R_{b} is C₁-C₄alkyl; and
   R_{c} is amino, mono- or di(C₁-C₄alkyl amino, mono- or di(C₁-C₄alkyl)amino substituted with -C(O)OH, C₃-C₇cycloalkylamino, or phenylamino.
6. A compound or salt of Clause 1, wherein
   R is -C(O)Rₐ or -C(O)CH₂Rₐ, where
   Rₐ is hydrogen -C(O)NH₂, C₁-C₂alkyl, C₁-C₂alkoxy, (C₁-C₂alkylester)C₀-C₂alkyl, cyclopropyl, or cyclohexyl.
7. A compound or salt of Clause 1, wherein R is a group of the formula: 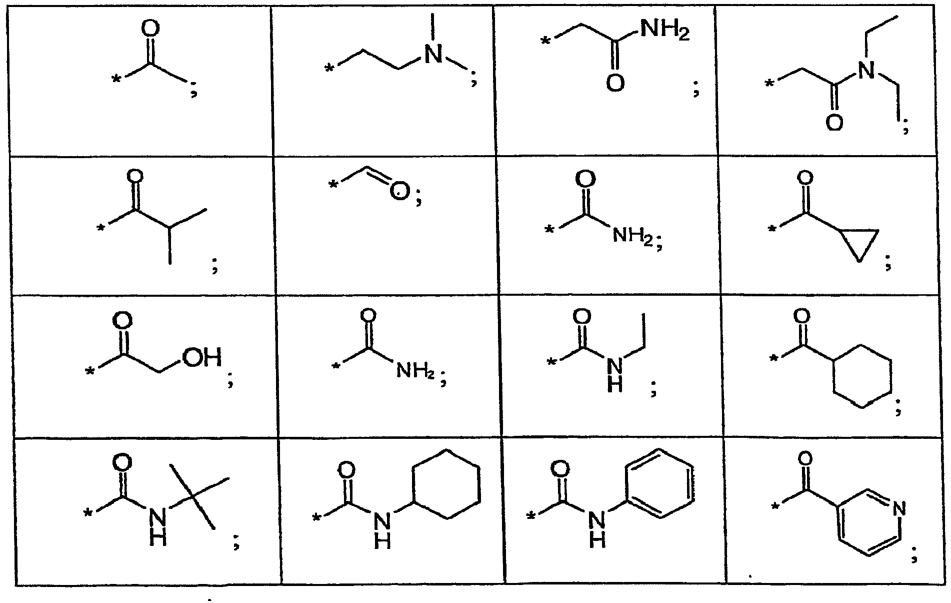 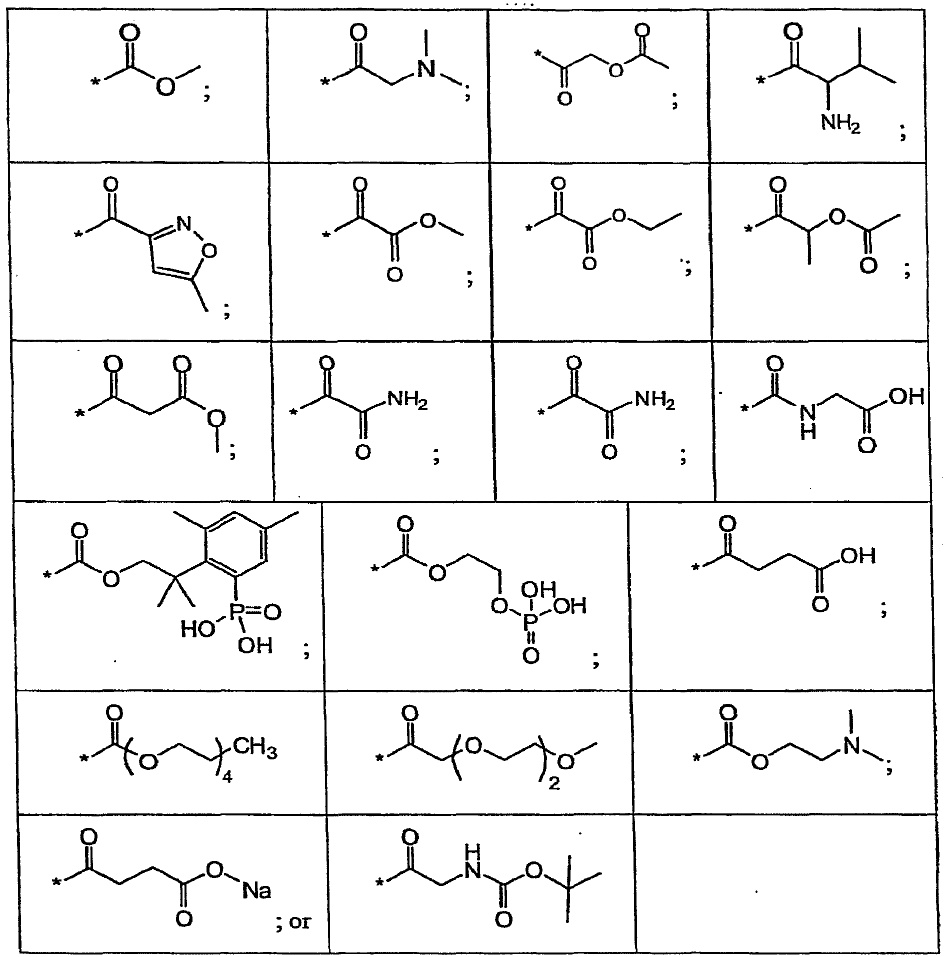 wherein the * indicates the bond of attachment.
8. A compound or salt of Clause 1, wherein R₁ is in the para position and R₂ is absent or in which R₁ is in the para position and R₂ is a single substituent in the meta position.
9. A compound or salt of Clause 1, wherein
   R₁ is in the para position and (5- or 6-membered heteroaryl)C₀-C₄alkyl or (5- or 6-membered heteroaryl)C₀-C₄alkoxy, wherein the 5- or 6-membered heteroaryl is thienyl, thiazolyl, imidazolyl, oxazolyl, or pyridyl.
10. A compound or salt of Clause 1, wherein
   R₁ is C₄-C₁₀alkoxy, C₄-C₁₀alkyl, C₄-C₁₀alkylamino, di-(C₁-C₆)(C₄-C₁₀)alkylamino, or (C₃-C₇xycloalkyl)C₀-C₄alkyl, and
   R₂ is 0 or 1 substituent chosen from halogen, hydroxyl, amino, cyano, C₁-C₂alkyl, C₁-C₂alkoxy, C₁-C₂haloalkyl, and C₁-C₂haloalkoxy.
11. A compound or salt of Clause 1, wherein
   R₁ is a para position and is C₄-C₁₀alkoxy, C₄-C₁₀alkyl, C₄-C₁₀alkylamino, or di-(C₁-C₆)(C₄-C₁₀)alkylamino, and
   R₂ is absent or R₂ is a meta substituent and is halogen, trifluoromethyl, or trifluoromethoxy.
12. A compound or salt of Clause 1, wherein R₁ is n-octyl, n-butoxy, isobutoxy, n-pentoxy, n-hexoxy, n-heptoxy, n-octyloxy, (cyclohexyl)methoxy, trifluoromethyl, or N-methyl-N-pentylamino.
13. A compound or salt of Clause 1, wherein Q is -NHC(O)- or absent.
14. A compound or salt of Clause 12, wherein Q is absent.
15. A compound or salt of Clause 13, wherein A is 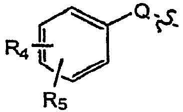
16. A compound or salt of Clause 15, wherein
   R₄ is halogen, -C(O)NH₂, C₁-C₂haloalkyl, mono- or di- C₁-C₄alkylamino, mono- or di-(C₁-C₄)alkylcarboxamide, C₂-C₄alkanoyl, C₁-C₄aminoalkyl, C₁-C₄aminoalkoxy, C₁-C₄hydroxyalkyl, or C₁-C₄alkylester, and
   R₅ is 0 or 1 or more substituents independently chosen from
   hydroxyl, halogen, amino, -CONH₂, trifluoromethyl, trifluoromethoxy, C₁-C₂alkyl, and C₁-C₂alkoxy.
17. A compound or salt of Clause 15, wherein
   R₄ and R₅ bound to adjacent carbon atoms are joined to form a 5- or 6-membered ring having 0, 1, or 2 ring heteroatoms chosen from N, O, and S; which 5- or 6-membered ring is optionally substituted with 1 or 2 substituents independently chosen from halogen, hydroxyl, oxo, C₁-C₂alkyl, and C₁-C₂alkoxy.
18. A compound or salt of Clause 1, wherein A is a group of the formula 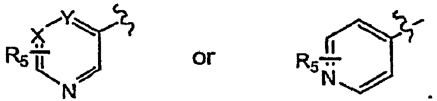
19. A compound or salt of Clause 18, wherein
   R₅ is 0 or 1 or more substituents independently chosen from
   (i) hydroxyl, halogen, amino, cyano, nitro, -COOH, -CONH₂, -PO₄, C₁-C₂haloalkyl, and C₁-C₂haloalkoxy; and
   (ii) C₁-C₄alkyl, C₁-C₄alkoxy, mono- and di- C₁-C₄alkylamino, C₂-C₄alkanoyl, and C₁-C₄alkylester, each of which is substituted with 0 or 1 or more substituents independently chosen from halogen, hydroxyl, amino, cyano, oxo, C₁-C₂alkoxy, mono- and di- C₁-C₂alkylamino.
20. A compound or salt of Clause 18, wherein
   R₅ is 0, 1, or 2 substituents independently chosen from hydroxyl, halogen, amino, cyano, -COOH, -CONH₂, C₁-C₂haloalkyl, C₁-C₂haloalkoxy, C₁-C₄alkyl, C₁-C₄alkoxy, mono- and di- C₁-C₄alkylamino, and C₂-C₄alkanoyl.
21. A compound or salt of Clause 18, wherein:
   R₅ is absent or R₅ is 0, 1, or 2 substituents independently chosen from hydroxyl, halogen, cyano, -CONH₂, C₁-C₂alkyl, and C₁-C₂alkoxy.
22. A compound or salt of Clause 18, wherein
   A is 3-pyridyl and R₅ is absent or R₅ is 0, 1, or 2 substituents independently chosen from hydroxy, fluoro, chloro, cyano, -CONH₂, methyl, and methoxy.
23. A compound or salt of Clause 22, wherein A is 3-pyridyl and R₅ is absent.
24. A compound or salt of Clause 1, wherein A is a group of the formula 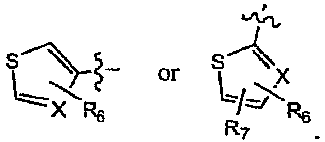
25. A compound or salt of Clause 24, wherein
   R₆ is 0 or 1 or more substituents independently chosen from
   hydroxyl, halogen, amino, cyano, -COOH, -CONH₂, C₁-C₂haloalkyl, C₁-C₂haloalkoxy; C₁-C₄alkyl, C₁-C₄alkoxy, mono- and di- C₁-C₄alkylamino, C₂-C₄alkanoyl, and C₁-C₄alkylester.
26. A compound or salt of Clause 25, wherein
   R₆ is 0, 1, or 2 substituents independently chosen from
   halogen, C₁-C₂alkyl, and C₁-C₂alkoxy.
27. A compound or salt of Clause 24, wherein
   R₆ is joined with R₇ to form a 6-membered aryl or heteroaryl ring, which ring is substituted with 0 or 1 or more substituents independently chosen from halogen, hydroxyl, C₁-C₂alkyl, and C₁-C₂alkoxy.
28. A compound or salt of Clause 1, wherein A is a group of the formula: 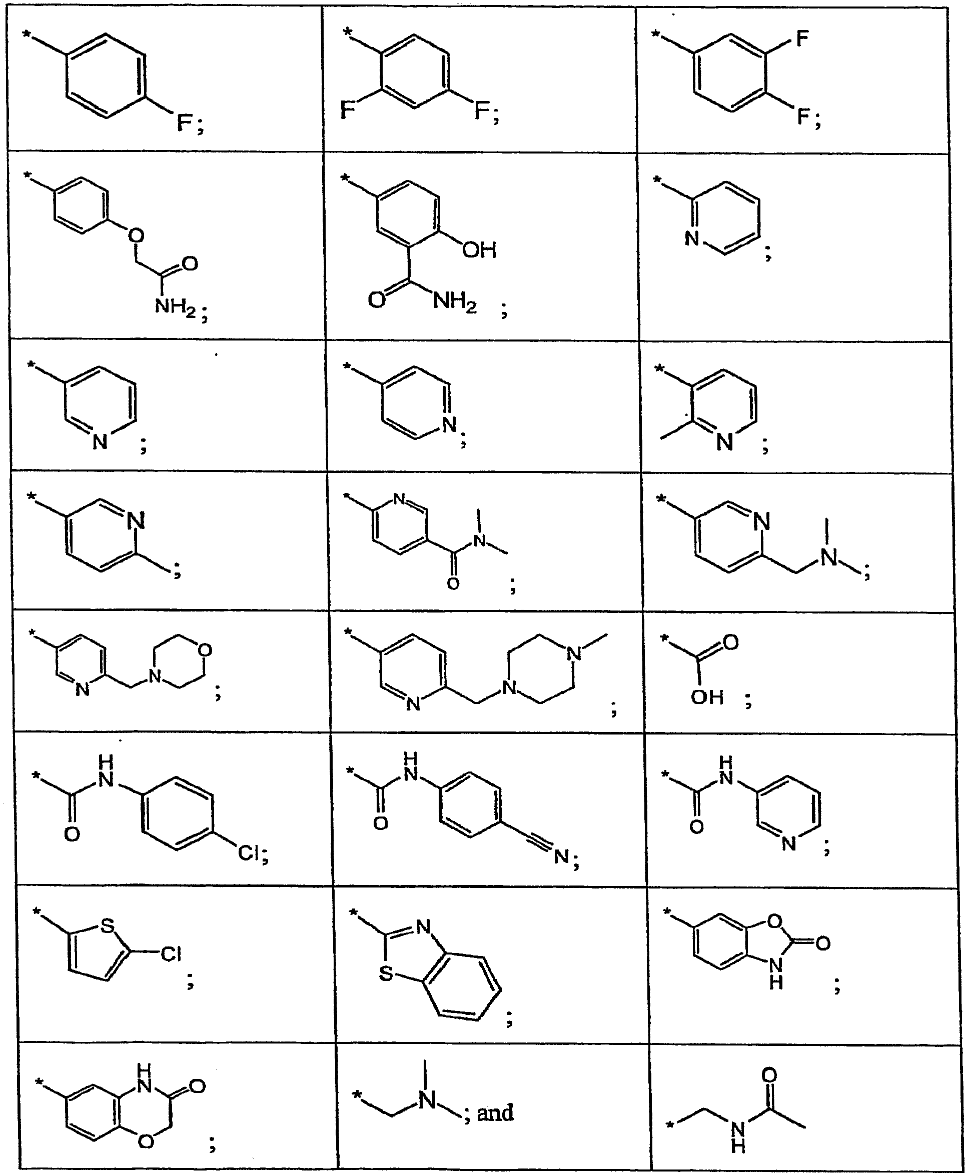 wherein the * indicates the bond of attachment.
29. A compound or salt of Clause 1, wherein
   R₃ is halogen, hydroxyl, amino, methyl, methoxy, dimethylamino, or dimethylaminomethyl.
30. A compound or salt of Clause 29, wherein R₃ is halogen.
31. A compound or salt of Clause 30, wherein R₃ is fluoro.
32. A compound or salt of Clause 1, wherein R₃ is hydrogen.
33. A compound or salt of Clause 1 of Formula IA 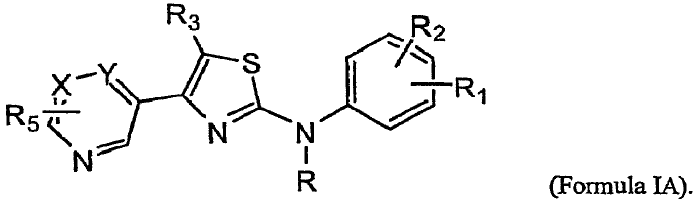
34. A compound or salt of Clause 33, wherein
   R₁ is in the para position and R₂ is absent or located in the meta position;
   R₃ is hydrogen, halogen, or amino;
   X and Y are CH; and
   R₅ is 0, 1, or 2 substituents independently chosen from hydroxyl, halogen, amino, cyano, -COOH, -CONH₂, C₁-C₂haloalkyl, C₁-C₂haloalkoxy, C₁-C₄alkyl, C₁-C₄alkoxy, mono- and di- C₁-C₄alkylamino, and C₂-C₄alkanoyl.
35. A compound or salt of Clause 34, wherein
   R₁ is in the para position and is C₄-C₁₀alkoxy, C₄-C₁₀alkyl, C₄-C₁₀alkylamino, or di-(C₁-C₆)(C₄-C₁₀)alkylamino;
   R₂ is absent or R₂ is a meta substituent and is halogen, trifluoromethyl, or trifluoromethoxy;
   R₃ is hydrogen, fluoro, or amino;
   R₅ is absent or R₅ is 0, 1, or 2 substituents independently chosen from hydroxyl, halogen, cyano, -CONH₂, C₁-C₂alkyl, and C₁-C₂alkoxy.
36. A compound or salt of Clause 1, of Formula IB 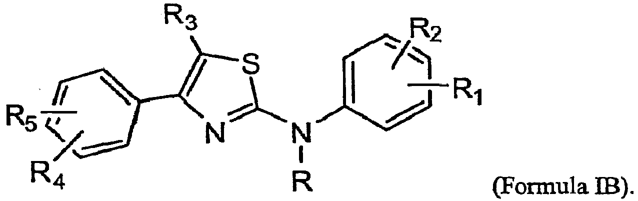
37. A compound or salt of Clause 36, wherein R₄ is a halogen atom and R₅ is absent or a halogen atom.
38. A compound or salt of Clause 37, wherein R₄ is in the para position and R₅ is absent, R₄ is in the meta position and R₅ is absent, or R₄ is in the para position and R₅ is in the meta position.
39. A compound or salt of Clause 1, of Formula IC 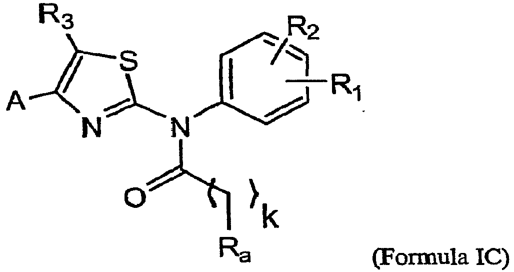 where k is 0 or 1.
40. A compound or salt of Clause 39, wherein Rₐ is amino, C₁-C₈ alkyl, a C₁-C₄ alkoxy, or C₁-C₄alkylester.
41. A compound or salt of Clause 1, wherein the compound is
   N-(4-(4-fluorophenyl)thiazol-2-yl)-N-(4-(pentyloxy)-3-(trifluoromethyl)phenyl)acetamide;
   N-(4-(pentyloxy)-3-(trifluoromethyl)phenyl)-N-(4-(pyridin-3-yl)thiazol-2-yl)acetamide;
   N-(4-(pentyloxy)-3-(trifluoromethyl)phenyl)-N-(4-(pyridin-3-yl)thiazol-2-yl)isobutyramide;
   N-(4-(pentyloxy)-3-(trifluoromethyl)phenyl)-N-(4-(pyridin-3-yl)thiazol-2-yl)cyclopropanecarboxamide;
   N-(3-fluoro-4-(pentyloxy)phenyl)-N-(4-(pyridin-3-yl)thiazol-2-yl)acetamide;
   N-(3-fluoro-4-(pentyloxy)phenyl)-N-(4-(pyridin-3-yl)thiazol-2-yl)isobutyramide;
   N-(3-fluoro-4-(pentyloxy)phenyl)-N-(4-(pyridin-3-yl)thiazol-2-yl)cyclopropanecarboxamide; methyl 2-((3-fluoro-4-(pentyloxy)phenyl)(4-(pyridin-3-yl)thiazol-2-yl)amino)-2-oxoacetate; methyl 2-oxo-2-((4-(pentyloxy)-3-(trifluoromethyl)phenyl)(4-(pyridin-3-yl)thiazol-2-yl)amino)acetate;
   methyl 2-oxo-2-((4-(pentyloxy)-3-(trifluoromethyl)phenyl)(4-(pyridin-3-ylcarbamoyl)thiazol-2-yl)amino)acetate; methyl 4-(pentyloxy)-3-(trifluoromethyl)phenyl(4-(pyridin-3-yl)thiazol-2-yl)carbamate;
   N-(4-(pentyloxy)-3-(trifluoromethyl)phenyl)-N-(4-(pyridin-3-yl)thiazol-2-yl)cyclohexanecarboxamide;
   2-oxo-2-((4-(pentyloxy)-3-(trifluoromethyl)phenyl)(4-(pyridin-3-yl)thiazol-2-yl)amino)ethyl acetate;
   5-methyl-N-(4-(pentyloxy)-3-(trifluoromethyl)phenyl)-N-(4-(pyridin-3-yl)thiazol-2-yl)isoxazole-3-carboxamide;
   methyl 2-oxo-2-((4-(pentyloxy)-3-(trifluoromethyl)phenyl)(4-(pyridin-4-yl)thiazol-2-yl)amino)acetate;
   methyl 4-(pentyloxy)-3-(trifluoromethyl)phenyl(4-(pyridin-4-yl)thiazol-2-yl)carbamate;
   methyl 2-((4-(2,4-difluorophenyl)thiazol-2-yl)(4-(pentyloxy)-3-(trifluoromethyl)phenyl)amino)-2-oxoacetate;
   methyl 2-((4-(4-fluorophenyl)thiazol-2-yl)(4-(pentyloxy)-3-(trifluoromethyl)phenyl)amino)-2-oxoacetate;
   methyl 2-((4-octylphenyl)(4-(pyridin-3-yl)thiazol-2-yl)amino)-2-oxoacetate;
   methyl 2-((4-(heptyloxy)phenyl)(4-(pyridin-3-yl)thiazol-2-yl)amino)-2-oxoacetate; 2-(dimethylamino)-N-(4-(pentyloxy)-3-(trifluoromethyl)phenyl)-N-(4-(pyridin-3-yl)thiazol-2-yl)acetamide;
   methyl 3-((3-fluoro-4-(pentyloxy)phenyl)(4-(4-fluorophenyl)thiazol-2-yl)amino)-3-oxopropanoate;
   methyl 3-((4-(4-fluorophenyl)thiazol-2-yl)(4-(pentyloxy)-3-(trifluoromethyl)phenyl)amino)-3-oxopropanoate;
   methyl 3-((4-(4-fluorophenyl)thiazol-2-yl)(4-(octyloxy)phenyl)amino)-3-oxopropanoate;
   methyl 2-((3-fluoro-4-(pentyloxy)phenyl)(4-(4-fluorophenyl)thiazol-2-yl)amino)-2-oxoacetate;
   methyl 4-(4-fluorophenyl)thiazol-2-yl(4-(pentyloxy)-3-(trifluoromethyl)phenyl)carbamate;
   1-(4-(pentyloxy)-3-(trifluoromethyl)phenyl)-1-(4-(pyridin-3-yl)thiazol-2-yl)urea;
   N-(4-(pentyloxy)-3-(trifluoromethyl)phenyl)-N-(4-(pyridin-3-yl)thiazol-2-yl)formamide;
   2-((4-(methyl(pentyl)amino)-3-(trifluoromethyl)phenyl)(4-(pyridin-3-yl)thiazol-2-yl)amino)-2-oxoethyl acetate;
   5-methyl-N-(4-(methyl(pentyl)amino)-3-(trifluoromethyl)phenyl)-N-(4-(pyridin-3-yl)thiazol-2-yl)isoxazole-3-carboxamide;
   methyl 2-((4-(methyl(pentyl)amino)-3-(trifluoromethyl)phenyl)(4-(pyridin-3-yl)thiazol-2-yl)amino)-2-oxoacetate;
   2-((4-(3,4-difluorophenyl)thiazol-2-yl)(4-(pentyloxy)-3-(trifluoromethyl)phenyl)amino)-2-oxoethyl acetate;
   methyl 2-((4-(methyl(pentyl)amino)-3-(trifluoromethyl)phenyl)(4-(pyridin-3-yl)thiazol-2-yl)amino)-2-oxoacetate;
   methyl 4-(3,4-difluorophenyl)thiazol-2-yl(4-(pentyloxy)-3-(trifluoromethyl)phenyl)carbamate; .
   2-((4-(3,4-difluorophenyl)thiazol-2-yl)(4-(octyloxy)phenyl)amino)-2-oxoethyl acetate; methyl 2-((4-(3,4-difluorophenyl)thiazol-2-yl)(4-(octyloxy)phenyl)amino)-2-axoacetate;
   2-((4-(4-fluorophenyl)thiazol-2-yl)(4-(octyloxy)phenyl)amino)-2-oxoethyl acetate;
   2-hydroxy-N-(4-(pentyloxy)-3-(trifluoromethyl)phenyl)-N-(4-(pyridin-3-yl)thiazol-2-yl)acetamide;
   1-((4-(6-methylpyridin-3-yl)thiazol-2-yl)(4-(octyloxy)phenyl)amino)-1-oxopropan-2-yl acetate;
   1-oxo-1-((4-(pentyloxy)-3-(trifluoromethyl)phenyl)(4-(pyridin-3-yl)thiazol-2-yl)amino)propan-2-yl acetate;
   2-oxo-2-((4-(pentyloxy)-3-(trifluoromethyl)phenyl)(4-(pyridin-3-yl)thiazol-2-yl)amino)-1-phenylethyl acetate;
   ethyl 2-((4-(octyloxy)phenyl)(4-(pyridin-3-yl)thiazol-2-yl)amino)-2-oxoacetate;
   2-((4-(octyloxy)phenyl)(4-(pyridin-3-yl)thiazol-2-yl)amino)-2-oxoethyl acetate;
   N-(4-(octyloxy)phenyl)-N-(4-(pyridin-3-yl)thiazol-2-yl)acetamide;
   1-(4-(4-fluorophenyl)thiazol-2-yl)-1-(4-(pentyloxy)-3-(trifluoromethyl)phenyl)urea;
   1-(4-(2-methylpyridin-3-yl)thiazol-2-yl)-1-(4-(pentyloxy)-3-(trifluoromethyl)phenyl)urea;
   1-(4-(4-fluorophenyl)thiazol-2-yl)-1-(4-(heptyloxy)phenyl)urea;
   1-(4-(octyloxy)phenyl)-1-(4-(pyridin-3-yl)thiazol-2-yl)urea;
   3-ethyl-1-(4-(pentyloxy)-3-(trifluoromethyl)phenyl)-1-(4-(pyridin-3-yl)thiazol-2-yl)urea;
   3-tert-butyl-1-(4-(pentyloxy)-3-(trifluoromethyl)phenyl)-1-(4-(pyridin-3-yl)thiazol-2-yl)urea;
   N,N-dimethyl-5-(2-(1-(4-(pentyloxy)-3-(trifluoromethyl)phenyl)ureido)thiazol-4-yl)picolinamide;
   1-(4-(pentyloxy)-3-(trifluoromethyl)phenyl)-3-phenyl-1-(4-(pyridin-3-yl)thiazol-2-yl)urea;
   1-(4-fluoro-3-(trifluoromethyl)phenyl)-1-(4-(pyridin-3-yl)thiazol-2-yl)urea;
   1-(4-methoxy-3-(trifluoromethyl)phenyl)-1-(4-(pyridin-3-yl)thiazol-2-yl)urea;
   1-(4-(4-fluorophenyl)thiazol-2-yl)-1-(4-methoxy-3-(trifluoromethyl)phenyl)urea;
   1-(4-(2,4-difluorophenyl)thiazol-2-yl)-1-(4-(pentyloxy)-3-(trifluoromethyl)phenyl)urea;
   1-(3-fluoro-4-(pentyloxy)phenyl)-1-(4-(pyridin-3-yl)thiazol-2-yl)urea;
   N-(4-(5-chlorothiophen-2-yl)thiazol-2-yl)-5-methyl-N-(4-(octyloxy)phenyl)isoxazole-3-carboxamide;
   N-(4-(5-chlorothiophen-2-yl)thiazol-2-yl)-N-(4-(heptyloxy)phenyl)-5-methylisoxazole-3-carboxamide;
   N-(4-(benzo[d]thiazol-2-yl)thiazol-2-yl)-N-(4-(heptyloxy)phenyl)-5-methylisoxazole-3-carboxamide;
   N-(4-(benzo[d]thiazol-2-yl)thiazol-2-yl)-5-methyl-N-(4-(pentyloxy)-3-(trifluoromethyl)phenyl)isoxazole-3-carboxamide;
   N-(4-(pentyloxy)-3-(trifluoromethyl)phenyl)-N-(4-(pyridin-2-yl)thiazol-2-yl)cyclohexanecarboxamide;
   methyl 2-((4-(5-chlorothiophen-2-yl)thiazol-2-yl)(4-(octyloxy)phenyl)amino)-2-oxoacetate;
   methyl 2-((4-(5-chlorothiophen-2-yl)thiazol-2-yl)(4-(heptyloxy)phenyl)amino)-2-oxoacetate;
   methyl 2-((4-(5-chlorothiophen-2-yl)thiazol-2-yl)(4-(pentyloxy)-3-(trifluoromethyl)phenyl)amino)-2-oxoacetate;
   methyl 2-oxo-2-((4-(2-oxo-2,3-dihydrobenzo[d]oxazol-6-yl)thiazol-2-yl)(4-(pentyloxy)-3-(trifluoromethyl)phenyl)amino)acetate;
   N-(4-(benzo[d]thiazol-2-yl)thiazol-2-yl)-N-(4-(pentyloxy)-3-(trifluoromethyl)phenyl)nicotinamide;
   1-(4-butoxy-3-(trifluoromethyl)phenyl)-1-(4-(pyridin-2-yl)thiazol-2-yl)urea;
   1-(4-butoxy-3-(trifluoromethyl)phenyl)-1-(4-(pyridin-2-yl)thiazol-2-yl)urea;
   1-(4-(methyl(pentyl)amino)-3-(trifluoromethyl)phenyl)-1-(4-(pyridin-2-yl)thiazol-2-yl)urea;
   1-(4-(methyl(pentyl)amino)-3-(trifluoromethyl)phenyl)-1-(4-(pyridin-2-yl)thiazol-2-yl)urea;
   1-(4-(methyl(pentyl)amino)-3-(trifluoromethyl)phenyl)-1-(4-(pyridin-2-yl)thiazol-2-yl)urea;
   1-(3-fluoro-4-(pentyloxy)phenyl)-1-(4-(4-fluorophenyl)thiazol-2-yl)urea;
   1-(4-(cyclohexylmethoxy)-3-fluorophenyl)-1-(4-(pyridin-3-yl)thiazol-2-yl)urea;
   1-(4-(cyclohexylmethoxy)-3-fluorophenyl)-1-(4-(3,4-difluorophenyl)thiazol-2-yl)urea;
   1-(4-(4-fluorophenyl)thiazol-2-yl)-1-(4-(hexyloxy)phenyl)urea;
   1-(4-(hexyloxy)phenyl)-1-(4-(pyridin-2-yl)thiazol-2-yl)urea;
   1-(4-(3,4-difluorophenyl)thiazol-2-yl)-1-(4-(hexyloxy)phenyl)urea;
   1-(4-(4-fluorophenyl)thiazol-2-yl)-1-(4-isobutoxy-3-(trifluoromethyl)phenyl)urea;
   1-(4-(3,4-difluorophenyl)thiazol-2-yl)-1-(4-isobutoxy-3-(trifluoromethyl)phenyl)urea;
   1-(4-(octyloxy)-3-(trifluoromethyl)phenyl)-1-(4-pyridin-3-yl)thiazo1-2-yl)urea;
   N1-(4-(octyloxy)-3-(trifluoromethyl)phenyl)-N1-(4-(pyridin-3-yl)thiazol-2-yl)oxalamide;
   1-(4-butoxy-3-(trifluoromethyl)phenyl)-1-(4-(6-methylpyridin-3-yl)thiazol-2-yl)urea;
   5-(2-(1-(4-butoxy-3-(trifluoromethyl)phenyl)ureido)thiazol-4-yl)-2-hydroxybenzamide;
   1-(4-butoxy-3-(trifluoromethyl)phenyl)-1-(4-(4-fluorophenyl)thiazol-2-yl)urea;
   1-(4-butoxy-3-(trifluoromethyl)phenyl)-1-(4-(pyridin-3-yl)thiazol-2-yl)urea;
   1-(4-butoxy-3-(trifluoromethyl)phenyl)-1-(4-(pyridin-4-yl)thiazol-2-yl)urea;
   1-(4-butoxy-3-(trifluoromethyl)phenyl)-1-(4-(3,4-difluorophenyl)thiazol-2-yl)urea
   1-(4-(methyl(pentyl)amino)-3-(trifluoromethyl)phenyl)-1-(4-(6-methylpyridin-3-yl)thiazol-2-yl)urea;
   1-(4-(4-fluorophenyl)thiazol-2-yl)-1-(4-(methyl(pentyl)amino)-3-(trifluoromethyl)phenyl)urea;
   1-(4-(hexyloxy)phenyl)-1-(4-(pyridin-3-yl)thiazol-2-yl)urea;
   1-(4-isobutoxy-3-(trifluoromethyl)phenyl)-1-(4-(6-methylpyridin-3-yl)thiazol-2-yl)urea;
   2-hydroxy-5-(2-(1-(4-isobutoxy-3-(trifluoromethyl)phenyl)ureido)thiazol-4-yl)benzamide;
   1-(4-isobutoxy-3-(trifluoromethyl)phenyl)-1-(4-(3-oxo-3,4-dihydro-2H-benzo[b][1,4]oxazin-6-yl)thiazol-2-yl)urea;
   1-(4-isobutoxy-3-(trifluoromethyl)phenyl)-1-(4-(pyridin-2-yl)thiazol-2-yl)urea;
   1-(4-isobutoxy-3-(trifluoromethyl)phenyl)-1-(4-(pyridin-3-yl)thiazol-2-yl)urea;
   N1-(4-(pentyloxy)-3-(trifluoromethyl)phenyl)-N1-(4-(pyridin-3-yl)thiazol-2-yl)oxalamide;
   N-(4-(1,1-difluorooctyl)phenyl)-N-(4-(pyridin-3-yl)thiazol-2-yl)acetamide;
   1-(5-fluoro-4-(pyridin-3-yl)thiazol-2-yl)-1-(4-hexyl-3-(trifluoromethyl)phenyl)urea;
   1-(5-fluoro-4-(pyridin-3-yl)thiazol-2-yl)-1-(4-(pentyloxy)-3-(trifluoromethyl)phenyl)urea;
   2-((4-(octyloxy)-3-(trifluoromethyl)phenyl)(4-(pyridin-3-yl)thiazol-2-yl)amino)-2-oxoethyl acetate;
   2-hydroxy-N-(4-(octyloxy)-3-(trifluoromethyl)phenyl)-N-(4-(pyridin-3-yl)thiazol-2-yl)acetamide;
   2-(dimethylamino)-N-(4-(octyloxy)-3-(trifluoromethyl)phenyl)-N-(4-(pyridin-3-yl)thiazol-2-yl)acetamide;
   4-((4-(octyloxy)-3-(trifluoromethyl)phenyl)(4-(pyridin-3-yl)thiazol-2-yl)amino)-4-oxobutanoic acid;
   sodium 4-((4-(octyloxy)-3-(trifluoromethyl)phenyl)(4-(pyridin-3-yl)thiazol-2-yl)amino)-4-oxobutanoate;
   4-((5-fluoro-4-(pyridin-3-yl)thiazol-2-yl)(4-(octyloxy)-3-(trifluoromethyl)phenyl)amino)-4-oxobutanoic acid;
   tert-butyl 2-((5-fluoro-4-(pyrimidin-3-yl)thiazol-2-yl)(4-(octyloxy)-3-(trifluoromethyl)phenyl)amino)-2-oxoethylcarbamate;
   2-(3-(4-(octyloxy)-3-(trifluoromethyl)phenyl)-3-(4-(pyridin-3-yl)thiazol-2-yl)ureido)acetic acid;

   2-(3-(5-fluoro-4-(pyridin-3-yl)thiazol-2-yl)-3-(4-(octyloxy)-3-(trifluoromethyl)phenyl)ureido)acetic acid;
   2-(dimethylamino)-N-(5-fluoro-4-(pyridin-3-yl)thiazol-2-yl)-N-(4-(octyloxy)-3-(trifluoromethyl)phenyl)acetamide;
   2-((5-fluoro-4-(pyridin-3-yl)thiazol-2-yl)(4-(octyloxy)-3-(trifluoromethyl)phenyl)amino)-2-oxoethyl acetate;

   N-(5-fluoro-4-(pyridin-3-yl)thiazol-2-yl)-2-hydroxy-N-(4-(octyloxy)-3-(trifluoromethyl)phenyl)acetamide;
   2-(dimethylamino)-N-(5-fluoro-4-(pyridin-3-yl)thiazol-2-yl)-N-(4-octyl-3-(trifluoromethyl)phenyl)acetamide;
   2,5,8,11-tetraoxatridecan-13-yl 5-fluoro-4-(pyridin-3-yl)thiazol-2-yl(4-(octyloxy)-3-(trifluoromethyl)phenyl)carbamate;
   N-(5-fluoro-4-(pyridin-3-yl)thiazol-2-yl)-2-(2-(2-methoxyethoxy)ethoxy)-N-(4-(octyloxy)-3-(trifluoromethyl)phenyl)acetamide;
   2-(dimethylamino)-N-(5-fluoro-4-(pyridin-3-yl)thiazol-2-yl)-N-(4-(4-(thiophen-2-yl)butoxy)-3-(trifluoromethyl)phenyl)acetamide;
   4-((5-fluoro-4-(pyridin-3-yl)thiazol-2-yl)(4-(4-(thiophen-2-yl)butoxy)-3-(trifluoromethyl)phenyl)amino)-4-oxobutanoic acid;
   N-(5-fluoro-4-(pyridin-3-yl)thiazol-2-yl)-N-(4-octyl-3-(trifluoromethyl)phenyl)acetamide;
   1-(5-fluoro-4-(pyridin-3-yl)thiazol-2-yl)-1-(4-(octyloxy)-3-(trifluoromethyl)phenyl)urea;
   N-(5-fluoro-4-(pyridin-3-yl)thiazol-2-yl)-N-(4-(octyloxy)-3-(trifluoromethyl)phenyl)acetamide;
   1-(5-fluoro-4-(pyridin-3-yl)thiazol-2-yl)-1-(4-octyl-3-(trifluoromethyl)phenyl)urea;
   2-(dimethylamino)ethyl 5-fluoro-4-(pyridin-3-yl)thiazol-2-yl(4-octyl-3-(trifluoromethyl)phenyl)carbamate;
   2-amino-N-(5-fluoro-4-(pyridin-3-yl)thiazol-2-yl)-3-methyl-N-(4-octyl-3-(trifluoromethyl)phenyl)butanamide;
   2-(phosphonooxy)ethyl 5-fluoro-4-(pyridin-3-yl)thiazol-2-yl(4-octyl-3-(trifluoromethyl)phenyl)carbamate;
   2-((5-fluoro-4-(pyridin-3-yl)thiazol-2-yl)(4-octyl-3-(trifluoromethyl)phenyl)amino)-2-oxoethyl dihydrogen phosphate;
   4-((5-fluoro-4-(pyridin-3-yl)thiazol-2-yl)(4-octyl-3-(trifluoromethyl)phenyl)amino)-4-oxobutyl dihydrogen phosphate;
   ((5-fluoro-4-(pyridin-3-yl)thiazol-2-yl)(4-octyl-3-(trifluoromethyl)phenyl)carbamoyloxy)methyl 2-amino-3-methylbutanoate; phosphonooxymethyl 5-fluoro-4-(pyridin-3-yl)thiazol-2-yl(4-octyl-3-(trifluoromethyl)phenyl)carbamate;
   (E)-2-(1-(4-(octyloxy)phenyl)-2-propylidenehydrazinyl)-4-(pyridin-3-yl)thiazolo;
   (E)-2-(2-ethylidene-1-(4-(octyloxy)-3-(trifluoromethyl)phenyl)hydrazinyl)-4-(pyridin-3 yl)thiazolo;
   (E)-2-(2-ethylidene-1-(4-(octyloxy)phenyl)hydrazinyl)-4-(pyridin-3-yl)thiazolo;
   ((5-fluoro-4-(pyridin-3-yl)thiazol-2-yl)(4-(octyloxy)-3-(trifluoromethyl)phenyl)amino)methyl pivalate;
   N1-(5-fluoro-4-(pyridin-3-yl)thiazol-2-yl)-N1-(4-(4-(thiophen-2-yl)butoxy)-3-(trifluoromethyl)phenyl)ethane-1,2-diamine; or
   N-(4-(octyloxy)-3-(trifluoromethyl)phenyl)-N-(5-(pyridin-3-yl)thiazol-2-yl)acetamide.
42. A pharmaceutical composition comprising a compound or salt of Clause 1 and at least one pharmaceutically acceptable carrier.
43. The pharmaceutical composition of Clause 43, containing at least one additional antiviral agent that is not a compound or salt of Clause 1.
44. The pharmaceutical composition of Clause 44, wherein the at least one additional antiviral agent is ribavarin, an interferon, VX-950, or valopicitabine.
45. The pharmaceutical composition of Clause 42, wherein the composition is formulated as an injectable fluid, an aerosol, a cream, a gel, a tablet, a pill, a capsule, a syrup, ophthalmic solution, or a transdermal patch.
46. A package comprising the pharmaceutical composition of Clause 42 in a container and together with instructions for using the composition to treat or prevent HCV infection in a patient.
47. A method for treating hepatitis C infection comprising providing an effective amount of a compound or salt of Clause 1 to a patient in need of such treatment.
48. A method of inhibiting HCV replication *in vivo* comprising providing to a patient infected with HCV a concentration of a compound or salt of Clause **1** sufficient to inhibit HCV replicon replication *in vitro.*
49. The method of Clause 48 wherein the effective amount is an amount sufficient to decrease the number of HCV antibodies in the patient's blood or serum.

## Claims

1. A compound or pharmaceutically acceptable salt of Formula (I) 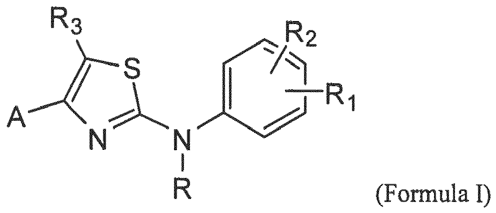 in which A is: 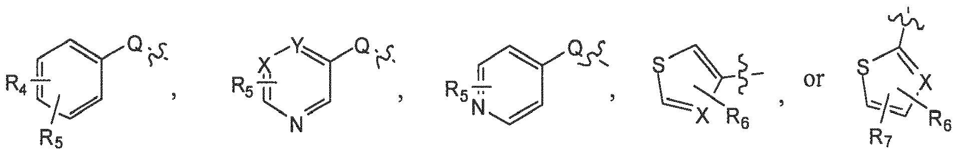 where
Q is absent;
X and Y are independently N or CH;
R is -C(O)Rₐ, -C(O)CH₂Rₐ, mono- or di-(C₁-C₄alkylamino)C₀-C₄alkyl, or substituted C₁-C₄ alkyl;
R₁ is C₁-C₂haloalkyl, C₁-C₂haloalkoxy, or R₁ is C₄-C₁₀alkoxy, C₄-C₁₀alkyl, C₄-C₁₀alkylamino, di-(C₁-C₆)(C₄-C₁₀)alkylamino, (C₃-C₇cycloalkyl)C₀-C₄alkyl, (C₃-C₇cycloalkyl)C₀-C₄alkoxy, (5- or 6-membered heteroaryl)C₀-C₄alkyl, or (5- or 6-membered heteroaryl)C₀-C₄alkoxy;
R₂ is 0, 1, or 2 substituents independently chosen from halogen, amino, C₁-C₂alkyl, C₁-C₂haloalkyl, and C₁-C₂haloalkoxy;
Rₐ is R_{c}, -C(O)OH, -C(O)NH₂, or Rₐ is C₁-C₆alkoxy, (C₁-C₆alkylester)C₀-C₄alkyl, (C₁-C₆alkylester)C₁-C₄alkoxy, (mono- or di-C₁-C₆alkylcarbamate)C₀-C₄alkyl, (C₃-C₇cycloalkyl)C₀-C₄alkyl, (5-10-membered heterocycle)C₀-C₄alkyl, C₃-C₇cycloalkyl, (phenyl)C₀-C₄alkyl, or C₆-C₁₂polyethylene oxide, each of which is substituted with 0, or 1 or more substituents independently chosen from halogen, hydroxyl, amino, phosphate, C₁-C₂alkyl, C₁-C₂alkoxy, mono- and di-(C₁-C₂alkyl)amino, trifluoromethyl, trifluoromethoxy, and phenyl;
R_{c} is amino, mono- or di(C₁-C₄alkyl amino, mono- or di(C₁-C₄alkyl amino substituted with -C(O)OH, C₃-C₇cycloalkylamino, or phenylamino;
R₃ is hydrogen or halogen;
R₄ is
(a) halogen, hydroxyl, amino, cyano, -C(O)OH, -C(O)NH₂, -PO₄, C₁-C₂haloalkyl, or C₁-C₂haloalkoxy, or
(b) mono- or di- C₁-C₄alkylamino, mono- or di-(C₁-C₄)alkylcarboxamide, C₂-C₄alkanoyl, C₁-C₄aminoalkyl, C₁-C₄aminoalkoxy, C₁-C₄hydroxyalkyl, or C₁-C₄alkylester, each of which is substituted with 0 or 1 or more substituents independently chosen from halogen, hydroxyl, amino, cyano, oxo, C₁-C₂alkyl, C₁-C₂alkoxy, mono- and di- C₁-C₂alkylamino;
R₅ is 0 or 1 or more substituents independently chosen from
(i) hydroxyl, halogen, amino, cyano, nitro, -COOH, -CONH₂, -PO₄, C₁-C₂haloalkyl, and C₁-C₂haloalkoxy; and
(ii) C₁-C₄alkyl, C₁-C₄alkoxy, mono- and di- C₁-C₄alkylamino, C₂-C₄alkanoyl, mono- and di-C₁-C₄alkylphosphate, C₁-C₄alkylester, (C₃-C₇cycloalkyl)C₀-C₂alkyl, or (5- or 6-membered heterocycloalkyl)C₀-C₂alkyl, each of which is substituted with 0 or 1 or more substituents independently chosen from halogen, hydroxyl, amino, cyano, oxo, C₁-C₂alkoxy, mono- and di- C₁-C₂alkylamino; or
any two R₄ and R₅ bound to adjacent carbon atoms may be joined to form a 5- or 6-membered ring having 0, 1, or 2 ring heteroatoms chosen from N, O, and S; which 5- or 6-membered ring is optionally substituted with 1 or 2 substituents independently chosen from halogen, hydroxyl, oxo, C₁-C₂alkyl, and C₁-C₂alkoxy;
R₆ is 0 or 1 or more substituents independently chosen from
(iii) hydroxyl, halogen, amino, cyano, -COOH, -CONH₂, -PO₄, C₁-C₂haloalkyl, and C₁-C₂haloalkoxy;
(iv) C₁-C₄alkyl, C₁-C₄alkoxy, mono- and di- C₁-C₄alkylamino, C₂-C₄alkanoyl, C₁-C₄alkylphosphate, and C₁-C₄alkylester, each of which is substituted with 0 or 1 or more substituents independently chosen from halogen, hydroxyl, amino, cyano, C₁-C₂alkoxy, C₁-C₂mono- and di-alkylamino; or
R₆ is joined with R₇ to form a 6-membered aryl or heteroaryl ring, which is substituted with 0 or 1 or more substituents independently chosen from halogen, hydroxyl, amino, cyano, C₁-C₂alkyl, C₁-C₂alkoxy, mono- and di- C₁-C₂alkylamino, C₁-C₂haloalkyl, and C₁-C₂haloalkoxy.

2. A compound or salt of Claim 1, wherein
(A) R is -C(O)Rₐ or -C(O)CH₂Ra, or
R is C₁-C₄alkyl substituted with 1 or 2 substituents independently chosen from hydroxyl, amino, -C(O)OH, -C(O)NH₂, -C(O)ONa, or C₁-C₄alkylester; where Rₐ is R_{c}, -C(O)OH, -C(O)NH₂, C₁-C₄alkoxy, (C₁-C₄alkylester)C₀-C₂alkyl, (mono- or di-C₁-C₆alkylcarbamate)C₀-C₂alkyl, C₆-C₁₂polyethylene oxide, C₃-C₇cycloalkyl, (phenyl)C₀-C₄alkyl, or 5-or 6-membered heteroaryl containing 1 or 2 heteroatoms independently chosen from N, S, and O, each of which Rₐ is substituted with 0, or 1 or more substituents independently chosen from halogen, hydroxyl, amino, phosphate, C₁-C₂alkyl, C₁-C₂alkoxy, mono- and di-(C₁-C₂alkyl)amino, trifluoromethyl, trifluoromethoxy, and phenyl;
and
R_{c} is amino, mono- or di(C₁-C₄alkyl amino, mono- or di(C₁-C₄alkyl amino substituted with -C(O)OH, C₃-C₇cycloalkylamino, or phenylamino;
(B) R is -C(O)Rₐ or -C(O)CH₂Rₐ;
Rₐ is R_{c}, -C(O)OH, -C(O)NH₂, C₁-C₄alkoxy, (C₁-C₄alkylester)C₀-C₂alkyl, (mono-or di-C₁-C₆alkylcarbamate)C₀-C₂alkyl, C₆-C₁₂polyethylene oxide, or C₃-C₆cycloalkyl, or
Rₐ is phenyl, isoxazolyl, thienyl, imidazolyl, or pyridyl, each of which is substituted with 0, 1, or 2 substituents independently chosen from halogen, hydroxyl, C₁-C₂alkyl, and C₁-C₂alkoxy;
and
R_{c} is amino, mono- or di(C₁-C₄alkyl amino, mono- or di(C₁-C₄alkyl)amino substituted with -C(O)OH, C₃-C₇cycloalkylamino, or phenylamino;
(C) R is -C(O)Rₐ or -C(O)CH₂Rₐ, where
Rₐ is C₁-C₄alkoxy, (C₁-C₄alkylester)C₀-C₂alkyl, or C₃-C₆cycloalkyl, or R is -C(O)NH₂;
(D) R is -C(O)Rₐ or -C(O)CH₂Rₐ,
Rₐ is R_{c}, -C(O)OH, -C(O)NH₂, C₁-C₄alkoxy, (C₁-C₄alkylester)C₀-C₂alkyl, (mono- or di-C₁-C₆alkylcarbamate)C₀-C₂alkyl, C₆-C₁₂polyethylene oxide, C₃-C₆cycloalkyl, or
Rₐ is phenyl, isoxazolyl, thienyl, imidazolyl, or pyridyl, each of which is substituted with 0, 1, or 2 substituents independently chosen from halogen, hydroxyl, C₁-C₂alkyl, and C₁-C₂alkoxy; and
R_{c} is amino, mono- or di(C₁-C₄alkyl amino, mono- or di(C₁-C₄alkyl)amino substituted with -C(O)OH, C₃-C₇cycloalkylamino, or phenylamino;
(E) R is -C(O)Rₐ or -C(O)CH₂Rₐ, where
Rₐ is -C(O)NH₂, C₁-C₂alkoxy, (C₁-C₂alkylester)C₀-C₂alkyl, cyclopropyl, or cyclohexyl.
(F) R is a group of the formula: 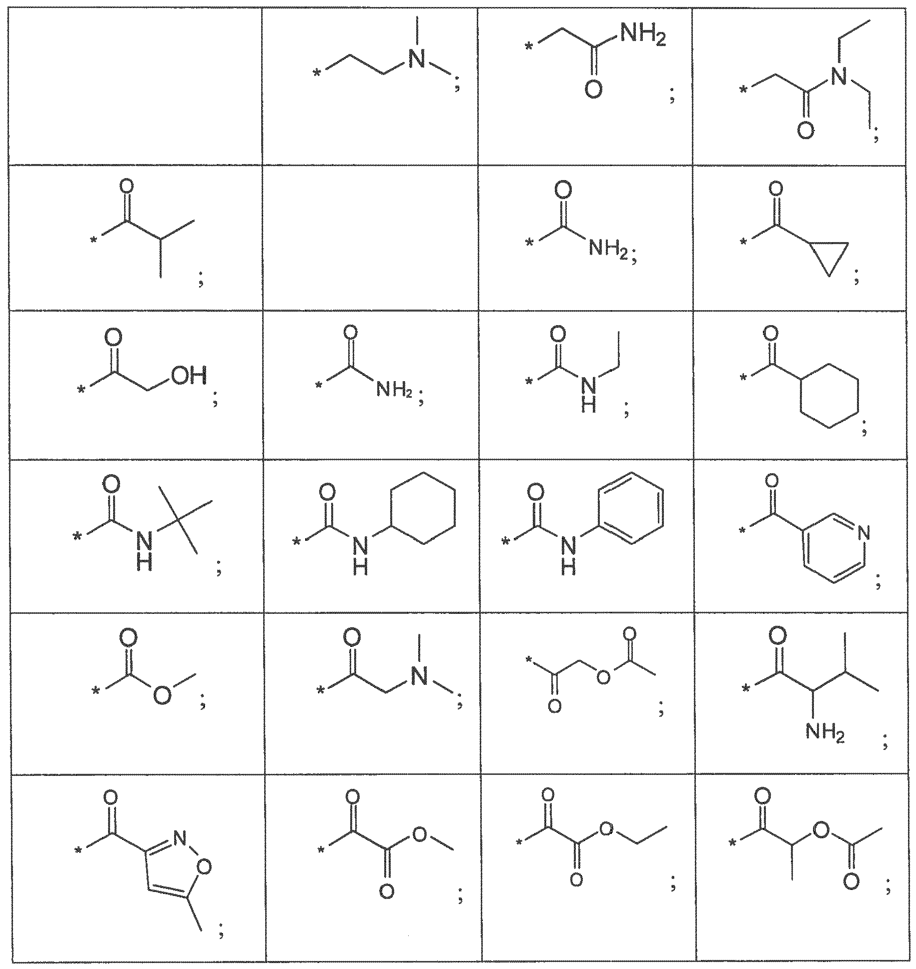 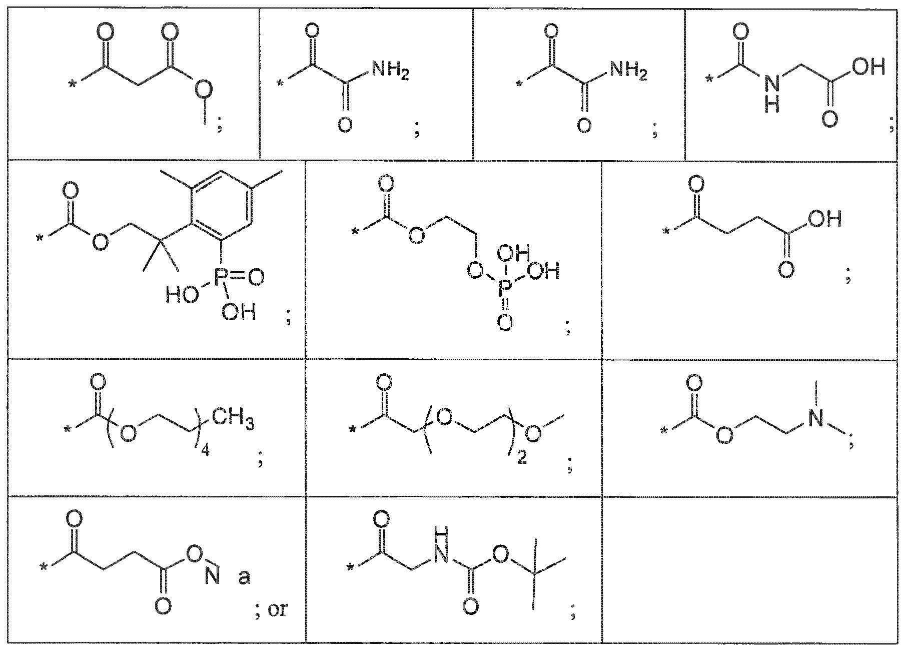 wherein the * indicates the bond of attachment;
(G) R₁ is in the para position and R₂ is absent or R₁ is in the para position and R₂ is a single substituent in the meta position;
(H) R₁ is in the para position and is (5- or 6-membered heteroaryl)C₀-C₄alkyl or (5- or 6-membered heteroaryl)C₀-C₄alkoxy, wherein the 5- or 6-membered heteroaryl is thienyl, thiazolyl, imidazolyl, oxazolyl, or pyridyl;
(I) R₁ is C₄-C₁₀alkoxy, C₄-C₁₀alkyl, C₄-C₁₀alkylamino, or di-(C₁-C₆)(C₄-C₁₀)alkylamino, (C₃-C₇cycloalkyl)C₀-C₄alkyl, and
R₂ is 0 or 1 substituent chosen from halogen, amino, C₁-C₂alkyl, C₁-C₂haloalkyl, and C₁-C₂haloalkoxy;
(J) R₁ is a para position and is C₄-C₁₀alkoxy, C₄-C₁₀alkyl, C₄-C₁₀alkylamino, or di-(C₁-C₆)(C₄-C₁₀)alkylamino, and
R₂ is absent or R₂ is a meta substituent and is halogen, trifluoromethyl, or trifluoromethoxy; or
(K) R₁ is n-octyl, n-butoxy, isobutoxy, n-pentoxy, n-hexoxy, n-heptoxy, n-octyloxy, (cyclohexyl)methoxy, trifluoromethyl, or N-methyl-N-pentylamino.

3. A compound or salt of Claim 1, wherein A is

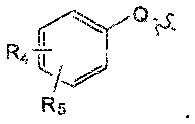

4. A compound or salt of Claim 3, wherein
(L) R₄ is halogen, -C(O)NH₂, C₁-C₂haloalkyl, mono- or di- C₁-C₄alkylamino, mono- or di-(C₁-C₄)alkylcarboxamide, C₂-C₄alkanoyl, C₁-C₄aminoalkyl, C₁-C₄aminoalkoxy, C₁-C₄hydroxyalkyl, or C₁-C₄alkylester, and
R₅ is 0 or 1 or more substituents independently chosen from hydroxyl, halogen, amino, -CONH₂, trifluoromethyl, trifluoromethoxy, C₁-C₂alkyl, C₁-C₂alkoxy; or
(M) R₄ and R bound to adjacent carbon atoms are joined to form a 5- or 6-membered ring having 0, 1, or 2 ring heteroatoms chosen from N, O, and S; which 5- or 6-membered ring is optionally substituted with 1 or 2 substituents independently chosen from halogen, hydroxyl, oxo, C₁-C₂alkyl, and C₁-C₂alkoxy.

5. A compound or salt of Claim 1, wherein A is a group of the formula

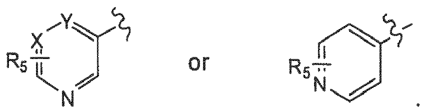

6. A compound or salt of Claim 5, wherein
(N) R₅ is 0 or 1 or more substituents independently chosen from
(i) hydroxyl, halogen, amino, cyano, nitro, -COOH, -CONH₂, -PO_{4,} C₁-C₂haloalkyl,
and C₁-C₂haloalkoxy; and
(ii) C₁-C₄alkyl, C₁-C₄alkoxy, mono- and di- C₁-C₄alkylamino, C₂-C₄alkanoyl, C₁-C₄alkylester, each of which is substituted with 0 or 1 or more substituents independently chosen from halogen, hydroxyl, amino, cyano, oxo, C₁-C₂alkoxy, mono- and di- C₁-C₂alkylamino;
(O) R₅ is 0, 1, or 2 substituents independently chosen from hydroxyl, halogen, amino, cyano, -COOH, -CONH₂, C₁-C₂haloalkyl, C₁-C₂haloalkoxy, C₁-C₄alkyl, C₁-C₄alkoxy, mono- and di- C₁-C₄alkylamino, and C₂-C₄alkanoyl;
(P) R₅ is absent or R₅ is 0, 1, or 2 substituents independently chosen from hydroxyl, halogen, cyano, -CONH₂, C₁-C₂alkyl, or C₁-C₂alkoxy; or
(Q) A is 3-pyridyl and R₅ is absent or R₅ is 0, 1, or 2 substituents independently chosen from hydroxy, fluoro, chloro, cyano, -CONH₂, methyl, and methoxy.

7. A compound or salt of Claim 6, wherein A is 3-pyridyl and R₅ is absent.

8. A compound or salt of Claim 1, wherein A is a group of the formula

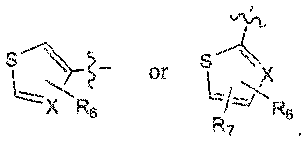

9. A compound or salt of Claim 8, wherein
(R) R₆ is 0 or I or more substituents independently chosen from hydroxyl, halogen, amino, cyano, -COOH, -CONH₂, C₁-C₂haloalkyl, C₁-C₂haloalkoxy; C₁-C₄alkyl, C₁-C₄alkoxy, mono- and di- C₁-C₄alkylamino, C₂-C₄alkanoyl, and C₁-C₄alkylester;
(S) R₆ is 0, 1, or 2 substituents independently chosen from halogen, C₁-C₂alkyl, and C₁-C₂alkoxy;
(T) R₆ is joined with R₇ to form a 6-membered aryl or heteroaryl ring, which is substituted with 0 or 1 or more substituents independently chosen from halogen, hydroxyl, C₁-C₂alkyl, C₁-C₂alkoxy; or
(U) A is a group of the formula: 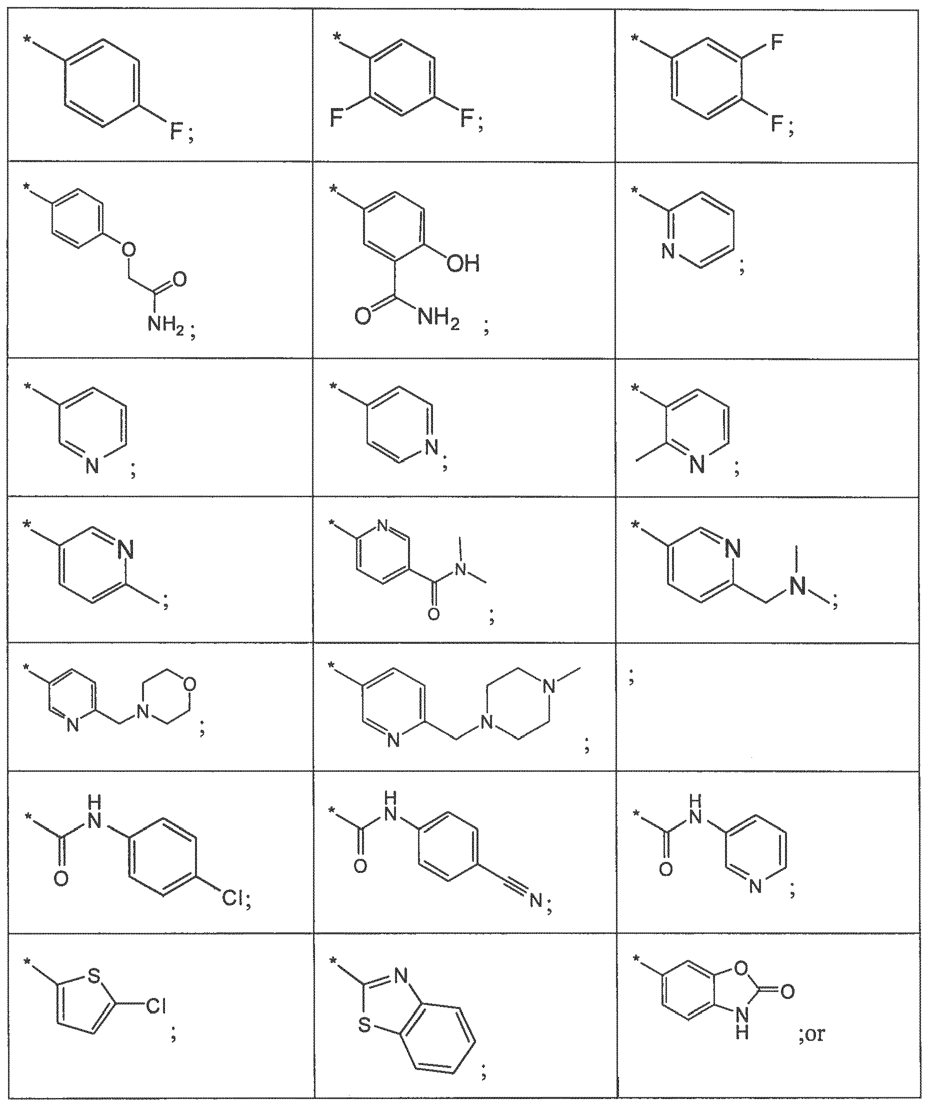 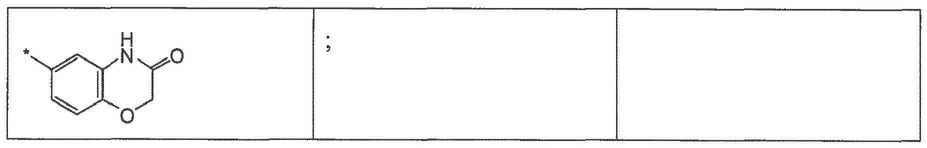 wherein the * indicates the bond of attachment.

10. A compound or salt of Claim 1 of Formula IA

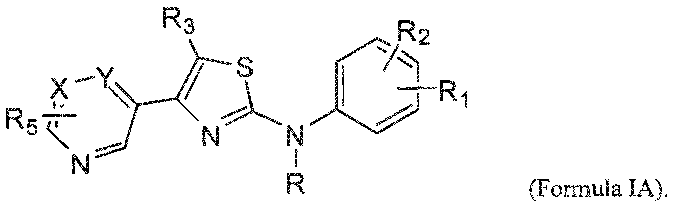

11. A compound or salt of Claim 10, wherein
(V) R₁ is in the para position and R₂ is absent or located in the meta position;
R₃ is hydrogen or halogen;
X and Y are CH; and
R₅ is 0, 1, or 2 substituents independently chosen from hydroxyl, halogen, amino, cyano, -COOH, -CONH₂, C₁-C₂haloalkyl, C₁-C₂haloalkoxy, C₁-C₄alkyl, C₁-C₄alkoxy, mono- and di- C₁-C₄alkylamino, and C₂-C₄alkanoyl;
(W) R₁ is in the para position and is C₄-C₁₀alkoxy, C₄-C₁₀alkyl, C₄-C₁₀alkylamino, or di-(C₁-C₆)(C₄-C₁₀)alkylamino;
R₂ is absent or R₂ is a meta substituent and is halogen, trifluoromethyl, or trifluoromethoxy;
R₃ is fluoro; and
R₅ is absent or R₅ is 0, 1, or 2 substituents independently chosen from hydroxyl, halogen, cyano, -CONH₂, C₁-C₂alkyl, or C₁-C₂alkoxy.

12. A compound or salt of Claim 1, of Formula

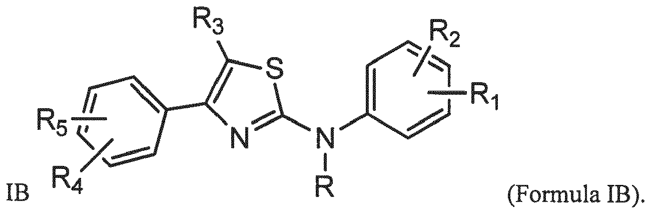

13. A compound or salt of Claim 12 wherein R₄ is a halogen atom and R₅ is absent or a halogen atom.

14. A compound or salt of Claim 1, of Formula IC 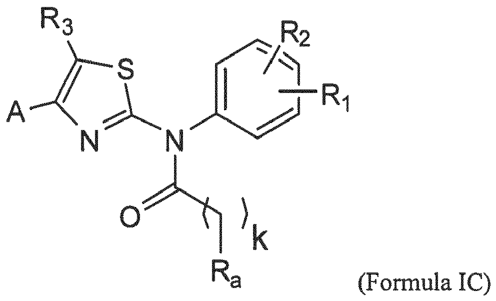 where k is 0 or 1.

15. A compound or salt of Claim 14, wherein Rₐ is amino, a C₁-C₄ alkoxy, or C₁-C₄alkylester.

16. A compound of salt of Claim 1, wherein the compound is
N-(4-(pentyloxy)-3-(trifluoromethyl)phenyl)-N-(4-(pyridin-3-yl)thiazol-2-yl)cyclopropanecarboxamide;
methyl 2-((3-fluoro-4-(pentyloxy)phenyl)(4-(pyridin-3-yl)thiazol-2-yl)amino)-2-oxoacetate; methyl 2-oxo-2-((4-(pentyloxy)-3-(trifluoromethyl)phenyl)(4-(pyridin-3-yl)thiazol-2-yl)amino)acetate;
methyl 4-(pentyloxy)-3-(trifluoromethyl)phenyl(4-(pyridin-3-yl)thiazol-2-yl)carbamate; N-(4-(pentyloxy)-3-(trifluoromethyl)phenyl)-N-(4-(pyridin-3-yl)thiazol-2-yl)cyclohexanecarboxamide;
2-oxo-2-((4-(pentytoxy)-3-(trifluoromethyl)phenyl)(4-(pyridin-3-yl)thiazol-2-yl)amino)ethyl acetate;
5-methyl-N-(4-(pentyloxy)-3-(trifluoromethyl)phenyl)-N-(4-(pyridin-3-yl)thiazol-2-yl)isoxazole-3-carboxamide;
methyl2-oxo-2-((4-(pentyloxy)-3-(trifluoromethyl)phenyl)(4-(pyndin-4-yl)thiazol-2-yl)amino)acetate;
methyl 4-(pentyloxy)-3-(trifluoromethyl)phenyl(4-(pyridin-4-yl)thiazol-2-yl)carbamate; methyl 2-((4-(2,4-difluorophenyl)thiazol-2-yl)(4-(pentyloxy)-3-(trifluoromethyl)phenyl)amino)-2-oxoacetate;
methyl 2-((4-(4-fluorophenyl)thiazol-2-yl)(4-(pentyloxy)-3-(trifluoromethyl)phenyl)amino)-2-oxoacetate;methyl 2-((4-octylphenyl)(4-(pyridin-3-yl)thiazol-2-yl)amino)-2-oxoacetate;
methyl 2-((4-(heptyloxy)phenyl)(4-(pyridin-3-yl)thiazol-2-yl)amino)-2-oxoacetate; 2-(dimethylamino)-N-(4-(pentyloxy)-3-(trifluoromethyl)phenyl)-N-(4-(pyridin-3-yl)thiazol-2-yl)acetamide;
methyl 3-((3-fluoro-4-(pentyloxy)phenyl)(4-(4-fluorophenyl)thiazol-2-yl)amino)-3-oxopropanoate;
methyl 3-((4-(4-fluorophenyl)thiazol-2-yl)(4-(pentyloxy)-3-(trifluoromethyl)phenyl)amino)-3-oxopropanoate;
ethyl 3-((4-(4-fluorophenyl)thiazol-2-yl)(4-(octyloxy)phenyl)amino)-3-oxopropanoate; methyl 2-((3-fluoro-4-(pentyloxy)phenyl)(4-(4-fluorophenyl)thiazol-2-yl)amino)-2-oxoacetate;
methyl 4-(4-fluorophenyl)thiazol-2-yl(4-(pentyloxy)-3-(trifluoromethyl)phenyl)carbamate; 1-(4-(pentyloxy)-3-(trifluoromethyl)phenyl)-1-(4-(pyridin-3-yl)thiazol-2-yl)urea;
2-((4-(methyl(pentyl)amino)-3-(trifluoromethyl)phenyl)(4-(pyridin-3-yl)thiazol-2-yl)amino)-2-oxoethyl acetate;
5-methyl-N-(4-(methyl(pentyl)amino)-3-(trifluoromethyl)phenyl)-N-(4-(pyridin-3-yl)thiazol-2-yl)isaxazole-3-carboxamide;
methyl 2-((4-(methyl(pentyl)amino)-3-(trifluoromethyl)phenyl)(4-(pyridin-3-yl)thiazol-2-y)amino)-2-oxoacetate;
2-((4-(3,4-difluorophenyl)thiazol-2-yl)(4-(pentyloxy)-3-(trifluoromethyl)phenyl)amino)-2-oxoethyl acetate;
methyl 2-((4-(methyl(pentyl)amino)-3-(trifluoromethyl)phenyl)(4-(pyridin-3-yl)thiazol-2-yl)amino)-2-oxoacetate;
methyl 4-(3,4-difluorophenyl)thiazol-2-yl(4-(pentyloxy)-3-(trifluoromethyl)phenyl)carbamate;
2-((4-(3,4-difluorophenyl)thiazol-2-yl)(4-(octyloxy)phenyl)amino)-2-oxoethyl acetate; methyl 2-((4-(3,4-difluorophenyl)thiazol-2-yl)(4-(octyloxy)phenyl)amino)-2-oxoacetate; 2-((4-(4-fluorophenyl)thiazol-2-yl)(4-(octyloxy)phenyl)amino)-2-oxoethyl acetate; 2-hydroxy-N-(4-(pentyloxy)-3-(trifluoromethyl)phenyl)-N-(4-(pyridin-3-yl)thiazol-2-yl)acetamide;
1-((4-(6-methylpyridin-3-yl)thiazol-2-yl)(4-(octyloxy)phenyl)amino)-1-oxopropan-2-yl acetate;
1-oxo-1-((4-(pentyloxy)-3-(trifluoromethyl)phenyl)(4-(pyridin-3-yl)thiazol-2-yl)amino)propan-2-yl acetate;
2-oxo-2-((4-(pentyloxy)-3-(trifluoromethyl)phenyl)(4-(pyridin-3-yl)thiazol-2-yl)amino)-1-phenylethyl acetate;
ethyl 2-((4-(octyloxy)phenyl)(4-(pyridin-3-yl)thiazol-2-yl)amino)-2-oxoacetate; 2-((4-(octyloxy)phenyl)(4-(pyridin-3-yl)thiazol-2-yl)amino)-2-oxoethyl acetate; N-(4-(octyloxy)phenyl)-N-(4-(pyridin-3-yl)thiazol-2-yl)acetamide; 1-(4-(4-fluorophenyl)thiazol-2-yl)-1-(4-(pentyloxy)-3-(trifluoromethyl)phenyl)urea; 1-(4-(2-methylpyridin-3-yl)thiazol-2-yl)-1-(4-(pentyloxy)-3-(trifluoromethyl)phenyl)urea; 1-(4-(4-fluorophenyl)thiazol-2-yl)-1-(4-(heptyloxy)phenyl)urea; 1-(4-(octyloxy)phenyl)-1-(4-(pyridin-3-yl)thiazol-2-yl)urea; 3-ethyl-1-(4-(pentyloxy)-3-(trifluoromethyl)phenyl)-1-(4-(pyridin-3-yl)thiazol-2-yl)urea; 3-tert-butyl-1-(4-(pentyloxy)-3-(trifluoromethyl)phenyl)-1-(4-(pyridin-3-yl)thiazol-2-yl)urea; N,N-dimethyl-5-(2-(1-(4-(pentyloxy)-3-(trifluoromethyl)phenyl)ureido)thiazol-4-yl)picolinamide;
1-(4-(pentyloxy)-3-(trifluoromethyl)phenyl)-3-phenyl-1-(4-(pyridin-3-yl)thiazol-2-yl)urea; 1-(4-fluoro-3-(trifluoromethyl)phenyl)-1-(4-(pyridin-3-yl)thiazol-2-yl)urea; 1-(4-methoxy-3-(trifluoromethyl)phenyl)-1-(4-(pyridin-3-yl)thiazol-2-yl)urea; 1-(4-(4-fluorophenyl)thiazol-2-yl)-1-(4-methoxy-3-(trifluoromethyl)phenyl)urea; 1-(4-(2,4-difluorophenyl)thiazol-2-yl)-1-(4-(pentyloxy)-3-(trifluoromethyl)phenyl)urea; 1-(3-fluoro-4-(pentyloxy)phenyl)-1-(4-(pyridin-3-yl)thiazol-2-yl)urea;
N-(4-(5-chlorothiophen-2-yl)thiazol-2-yl)-5-methyl-N-(4-(octyloxy)phenyl)isoxazole-3-carboxamide;
N-(4-(5-chlorothiophen-2-yl)thiazol-2-yl)-N-(4-(heptyloxy)phenyl)-5-ethylisoxazole-3-carboxamide;
N-(4-(benzo[d]thiazol-2-yl)thiazol-2-yl)-N-(4-(heptyloxy)phenyl)-5-methylisoxazole-3-carboxamide;
N-(4-(benzo[d]thiazol-2-yl)thiazol-2-yl)-5-methyl-N-(4-(pentyloxy)-3-(trifluoromethyl)phenyl)isoxazole-3-carboxamide;
N-(4-(pentyloxy)-3-(trifluoromethyl)phenyl)-N-(4-(pyridin-2-yl)thiazol-2-yl)cyclohexanecarboxamide;
ethyl 2-((4-(5-chlorothiophen-2-yl)thiazol-2-yl)(4-(octyloxy)phenyl)amino)-2-oxoacetate; methyl 2-((4-(5-chlorothiophen-2-yl)thiazol-2-yl)(4-(heptyloxy)phenyl)amino)-2-oxoacetate; methyl 2-((4-(5-chlorothiophen-2-yl)thiazol-2-yl)(4-(pentyloxy)-3-(trifluoromethyl)phenyl)amino)-2-oxoacetate;
ethyl2-oxo-2-((4-(2-oxo-2,3-dihydrobenzo[d]oxazol-6-yl)thiazol-2-yl)(4-(pentyloxy)-3-(trifluoromethyl)phenyl)amino)acetate;
N-(4-(benzo[d]thiazol-2-yl)thiazol-2-yl)-N-(4-(pentyloxy)-3-(trifluoromethyl)phenyl)nicotinamide;
1-(4-butoxy-3-(trifluoromethyl)phenyl)-1-(4-(pyridin-2-yl)thiazol-2-yl)urea; 2-hydroxy-5-(2-(1-(4-(methyl(pentyl)amino)-3-(trifluoromethyl)phenyl)ureido)thiazol-4-yl)benzamide;
1-(4-(methyl(pentyl)amino)-3-(trifluoromethyl)phenyl)-1-(4-(pyridin-2-yl)thiazol-2-yl)urea;
1-(4-(3,4-difluorophenyl)thiazol-2-yl)-1-(4-(methyl(pentyl)amino)-3-(trifluoromethyl)phenyl)urea;
1-(3-fluoro-4-(pentyloxy)phenyl)-1-(4-(6-methylpyridin-3-yl)thiazol-2-yl)urea;
1-(3-fluoro-4-(pentyloxy)phenyl)-1-(4-(4-fluorophenyl)thiazol-2-yl)urea;
1-(4-(cyclohexylmethoxy)-3-fluorophenyl)-1-(4-(pyridin-3-yl)thiazol-2-yl)urea;
1-(4-(cyclohexylmethoxy)-3-fluorophenyl)-1-(4-(3,4-difluorophenyl)thiazol-2-yl)urea;
1-(4-(4-fluorophenyl)thiazol-2-yl)-1-(4-(hexyloxy)phenyl)urea;
1-(4-(hexyloxy)phenyl)-1-(4-(pyridin-2-yl)thiazol-2-yl)urea;
1-(4-(3,4-difluorophenyl)thiazol-2-yl)-1-(4-(hexyloxy)phenyl)urea;
1-(4-(4-fluorophenyl)thiazol-2-yl)-1-(4-isobutoxy-3-(trifluoromethyl)phenyl)urea;
1-(4-(3,4-difluorophenyl)thiazol-2-yl)-1-(4-isobutoxy-3-(trifluoromethyl)phenyl)urea;
1-(4-(octyloxy)-3-(trifluoromethyl)phenyl)-1-(4-(pyridin-3-yl)thiazol-2-yl)urea;
N1-(4-(octyloxy)-3-(trifluoromethyl)phenyl)-N1-(4-(pyridin-3-yl)thiazol-2-yl)oxalamide;
1-(4-butoxy-3-(trifluoromethyl)phenyl)-1-(4-(6-methylpyridin-3-yl)thiazol-2-yl)urea;
5-(2-(1-(4-butoxy-3-(trifluoromethyl)phenyl)ureido)thiazol-4-yl)-2-hydroxybenzamide;
1-(4-butoxy-3-(trifluoromethyl)phenyl)-1-(4-(4-fluorophenyl)thiazol-2-yl)urea;
1-(4-butoxy-3-(trifluoromethyl)phenyl)-1-(4-(pyridin-3-yl)thiazol-2-yl)urea;
1-(4-butoxy-3-(trifluoromethyl)phenyl)-1-(4-(pyridin-4-yl)thiazol-2-yl)urea;
1-(4-butoxy-3-(trifluoromethyl)phenyl)-1-(4-(3,4-difluorophenyl)thiazol-2-yl)urea
1-(4-(methyl(pentyl)amino)-3-(trifluoromethyl)phenyl)-1-(4-(6-methylpyridin-3-yl)thiazol-2-yl)urea;
1-(4-(4-fluorophenyl)thiazol-2-yl)-1-(4-(methyl(pentyl)amino)-3-(trifluoromethyl)phenyl)urea;
1-(4-(hexyloxy)phenyl)-1-(4-(pyridin-3-yl)thiazol-2-yl)urea;
1-(4-isobutoxy-3-(trifluoromethyl)phenyl)-1-(4-(6-methylpyridin-3-yl)thiazol-2-yl)urea;
2-hydroxy-5-(2-(1-(4-isobutoxy-3-(trifluoromethyl)phenyl)ureido)thiazol-4-yl)benzamide;
1-(4-isobutoxy-3-(trifluoromethyl)phenyl)-1-(4-(3-oxo-3,4-dihydro-2H-benzo[b][1,4]oxazin-6-yl)thiazol-2-yl)urea;
1-(4-isobutoxy-3-(trifluoromethyl)phenyl)-1-(4-(pyridin-2-yl)thiazol-2-yl)urea;
1-(4-isobutoxy-3-(trifluoromethyl)phenyl)-1-(4-(pyridin-3-yl)thiazol-2-yl)urea;
N1-(4-(pentyloxy)-3-(trifluoromethyl)phenyl)-N1-(4-(pyridin-3-yl)thiazol-2-yl)oxalamide;
N-(4-(1,1-difluorooctyl)phenyl)-N-(4-(pyridin-3-yl)thiazol-2-yl)acetamide;
1-(5-fluoro-4-(pyridin-3-yl)thiazol-2-yl)-1-(4-hexyl-3-(trifluoromethyl)phenyl)urea;
1-(5-fluoro-4-(pyridin-3-yl)thiazol-2-yl)-1-(4-(pentyloxy)-3-(trifluoromethyl)phenyl)urea;
2-((4-(octyloxy)-3-(trifluoromethyl)phenyl)(4-(pyridin-3-yl)thiazol-2-yl)amino)-2-oxoethyl acetate;
2-hydroxy-N-(4-(octyloxy)-3-(trifluoromethyl)phenyl)-N-(4-(pyridin-3-yl)thiazol-2-yl)acetamide;
2-(dimethylamino)-N-(4-(octyloxy)-3-(trifluoromethyl)phenyl)-N-(4-(pyridin-3-yl)thiazol-2-yl)acetamide;
4-((4-(octyloxy)-3-(trifluoromethyl)phenyl)(4-(pyridin-3-yl)thiazol-2-yl)amino)-4-oxobutanoic acid;
sodium 4-((4-(octyloxy)-3-(trifluoromethyl)phenyl)(4-(pyridin-3-yl)thiazol-2-yl)amino)-4-oxobutanoate;
4-((5-fluoro-4-(pyridin-3-yl)thiazol-2-yl)(4-(octyloxy)-3-(trifluoromethyl)phenyl)amino)-4-oxobutanoic acid;
tert-butyl 2-((5-fluoro-4-(pyridin-3-yl)thiazol-2-yl)(4-(octyloxy)-3-(trifluoromethyl)phenyl)amino)-2-oxoethylcarbamate;
2-(3-(4-(octyloxy)-3-(trifluoromethyl)phenyl)-3-(4-(pyridin-3-yl)thiazol-2-yl)ureido)acetic acid;
2-(3-(5-fluoro-4-(pyridin-3-yl)thiazol-2-yl)-3-(4-(octyloxy)-3-(trifluoromethyl)phenyl)ureido)acetic acid;
2-(dimethylamino)-N-(5-fluoro-4-(pyridin-3-yl)thiazol-2-yl)-N-(4-(octyloxy)-3-(trifluoromethyl)phenyl)acetamide;
2-((5-fluoro-4-(pyridin-3-yl)thiazol-2-yl)(4-(octyloxy)-3-(trifluoromethyl)phenyl)amino)-2-oxoethyl acetate;
N-(5-fluoro-4-(pyridin-3-yl)thiazol-2-yl)-2-hydroxy-N-(4-(octyloxy)-3-(trifluoromethyl)phenyl)acetamide;
2-(dimethylammo)-N-(5-fluoro-4-(pyridin-3-yl)thiazol-2-yl)-N-(4-octyt-3-(trifluoromethyl)phenyl)acetamide;
2,5,8,11-tetraoxatridecan-13-yl 5-fluoro-4-(pyridin-3-yl)thiazol-2-yl(4-(octyloxy)-3-(trifluoromethyl)phenyl)carbamate;
N-(5-fluoro-4-(pyridin-3-yl)thiazol-2-yl)-2-(2-(2-methoxyethoxy)ethoxy)-N-(4-(octyloxy)-3-(trifluoromethyl)phenyl)acetamide;
2-(dimethylamino)-N-(5-fluoro-4-(pyridin-3-yl)thiazol-2-yl)-N-(4-(4-(thiophen-2yl)butoxy)-3-(trifluoromethyl)phenyl)acetamide;
4-((5-fluoro-4-(pyridin-3-yl)thiazol-2-yl)(4-(4-(thiophen-2-yl)butoxy)-3-(trifluoromethyl)phenyl)amino)-4-oxobutanoic acid;
1-(5-fluoro-4-(pyridin-3-yl)thiazol-2-yl)-1-(4-(octyloxy)-3-(trifluoromethyl)phenyl)urea;
1-(5-fluoro-4-(pyridin-3-yl)thiazol-2-yl)-1-(4-octyl-3-(trifluoromethyl)phenyl)urea;
2-(dimethylamino)ethyl 5-fluoro-4-(pyridin-3-yl)thiazol-2-yl(4-octyl-3-(trifluoromethyl)phenyl)carbamate; 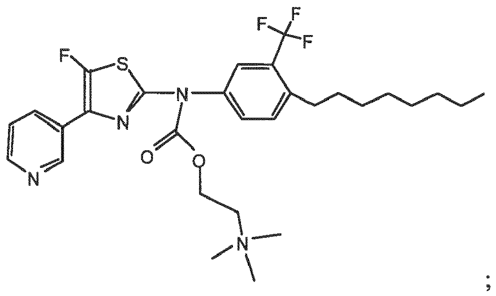 2-amino-N-(5-fluoro-4-(pyridin-3-yl)thiazol-2-yl)-3-methyl-N-(4-octyl-3-(trifluoromethyl)phenyl)butanamide; 1802-(phosphonooxy)ethyl 5-fluoro-4-(pyridin-3-yl)thiazol-2-yl(4-octyl-3-(trifluoromethyl)phenyl)carbamate;
2-((5-fluoro-4-(pyridin-3-yl)thiazol-2-yl)(4-octyl-3-(trifluoromethyl)phenyl)amino)-2-oxoethyl dihydrogen phosphate;
4-((5-fluoro-4-(pyridin-3-yl)thiazol-2-yl)(4-octyl-3-(trifluoromethyl)phenyl)amino)-4-oxobutyl dihydrogen phosphate;
((5-fluoro-4-(pyridin-3-yl)thiazol-2-yl)(4-octyl-3-(trifluoromethyl)phenyl)carbamoyloxy)methyl 2-amino-3-methylbutanoate;
phosphonooxymethyl 5-fluoro-4-(pyridin-3-yl)thiazol-2-yl(4-octyl-3-(trifluoromethyl)phenyl)carbamate;
((5-fluoro-4-(pyridin-3-yl)thiazol-2-yl)(4-(octyloxy)-3-(trifluoromethyl)phenyl)amino)methyl pivalate; or
N1-(5-fluoro-4-(pyridin-3-yl)thiazol-2-yl)-N1-(4-(4-(thiophen-2-yl)butoxy)-3-(trifluoromethyl)phenyl)ethane-1,2-diamine.

17. A pharmaceutical composition comprising a compound or salt of Claim 1 and at least one pharmaceutically acceptable carrier and optionally containing at least one additional antiviral agent that is not a compound or salt of Claim 1.

18. The pharmaceutical composition of Claim 17, wherein the composition is formulated as an injectable fluid, an aerosol, a cream, a gel, a tablet, a pill, a capsule, a syrup, ophthalmic solution, or a transdermal patch.

19. The compound of salt of claim 1 for use in treating hepatitis C infection.
